# EUROPEAN PATENT SPECIFICATION

(11) **EP 1 309 727 B1**
(45) Date of publication and mention of the grant of the patent: **06.01.2010**
(21) Application number: 01942677.4
(22) Date of filing: 23.01.2001
(51) Int. Cl.: C12Q 1/68, A01K 67/033

(54) **Method for functional mapping of the gene network of Alzheimer's disease**
Verfahren zur funktionellen Kartierung des Gesamtnetzwerkes der Alzheimerischen Krankheit
Méthode permettant de cartographier un réseau de gènes de la maladie d'Alzheimer

(30) Priority: 24.01.2000 US 490243
(43) Date of publication of application: 14.05.2003
(73) Proprietor: Neurosciences Research Foundation Inc., San Diego, CA 92121 (US)
(72) Inventor: GREENSPAN, Ralph, J., Coronado, CA 92118 (US); EDELMAN, Gerald, M., La Jolla, CA 92037 (US)
(74) Representative: UEXKÜLL & STOLBERG
(86) International application number: PCT/US2001/002332
(87) International publication number: WO 2001/053538

(56) References cited:
- WO-A-98/17782
- WO-A-99/55906
- WO-A1-00/55620
- PRICE D L ET AL: "GENETIC NEURODEGENERATIVE DISEASES: THE HUMAN ILLNESS AND TRANSGENIC MODELS" SCIENCE, AMERICAN ASSOCIATION FOR THE ADVANCEMENT OF SCIENCE,, US, vol. 282, November 1998 (1998-11), pages 1079-1083, XP002929296 ISSN: 0036-8075
- HOLCOMB L A ET AL: "BEHAVIORAL CHANGES IN TRANSGENIC MICE EXPRESSING BOTH AMYLOID PRECURSOR PROTEIN AND PRESENILIN-1 MUTATIONS: LACK OF ASSOCIATION WITH AMYLOID DEPOSITS" BEHAVIOR GENETICS, NEW YORK, NY, US, vol. 29, no. 3, May 1999 (1999-05), pages 177-185, XP000978983 ISSN: 0001-8244
- GUO YIQUAN ET AL: "Drosophila presenilin is required for neuronal differentiation and affects notch subcellular localization and signaling." JOURNAL OF NEUROSCIENCE, vol. 19, no. 19, 1 October 1999 (1999-10-01), pages 8435-8442, XP002196478 ISSN: 0270-6474
- DATABASE EMBL [Online] Database entry S76957, 29 July 1995 (1995-07-29) "olfactory receptor (clone HbA1)" Database accession no. S76957 XP002208318
- BRYANT ZEV ET AL: "Characterization of differentially expressed genes in purified Drosophila follicle cells: Toward a general strategy for cell type-specific developmental analysis." PROCEEDINGS OF THE NATIONAL ACADEMY OF SCIENCES OF THE UNITED STATES, vol. 96, no. 10, 11 May 1999 (1999-05-11), pages 5559-5564, XP002214578 May 11, 1999 ISSN: 0027-8424
- HO L. ET AL: 'ALTERED EXPRESSION OF A-TYPE BUT NOT B-TYPE SYNAPSIN ISOFORM IN THE BRAIN OF PATIENTS AT HIGH RISK FOR ALZHEIMER'S DISEASE ASSESSED BY DNA MICROARRAY TECHNIQUE' NEUROSCIENCE LETTERS vol. 298, no. 3, 09 February 2001, LIMERICK, IE, pages 191 - 194, XP009056555
- LORING J.F. ET AL: "Molecular Pathology: Gene expression microarrays used to analyze Alzheimer's disease brain" INTERNATIONAL GENOME SEQUENCING AND ANALYSIS CONFERENCE, vol. 12, September 2000 (2000-09), page 26-27, XP002215872
- GINSBERS S.D. ET AL: 'Expression profile of individual Alzheimer's disease neurofibrillary tangle-bearing CA1 neurons using gene microarrays' SOCIETY FOR NEUROSCIENCE ABSTRACTS vol. 25, no. 1-2, 1999, page 833, XP001115020
- JEE SEUNG W. ET AL: 'Analysis of differentially expressed genes in early- and late-stage APPsw-transgenic and normal mice using cDNA microarray' INTERNATIONAL JOURNAL OF MOLECULAR MEDECINE vol. 19, no. 3, March 2007, GREECE, pages 461 - 468, XP009110797
- SEUNG W. JEE ET AL: "Oligonucleotide-based Analysis of Differentially Expressed Genes in Hippocampus of Transgenci Mice Expressing NSE-controlled APPsw" NEUROCHEM. RES., vol. 31, 8 August 2006 (2006-08-08), pages 1035-1044,
- FRANK S. ET AL: 'TREM2 is Upregulated in Amyloid Plaque-Associated Microglia in Aged APP23 Transgenic Mice' GLIA vol. 56, 2008, pages 1438 - 1447

### SEQUENCE LISTING

<110> , Neurosciences Research Foundation, Inc.
   Greenspan, Ralph J.
   Edelman, Gerald M.
<120> Method For Functional Mapping of An
   Alzheimer's Disease Gene Network and For Identifying
   Therapeutic Agents for the Treatment of Alzheimer's Disease
<130> FP-NI 4562
<150> US 09/490,243
   <151> 2000-01-24
<160> 80
<170> FastSEQ for Windows Version 4.0
<210> 1
   <211> 509
   <212> DNA
   <213> Drosophila melanogaster
<400> 1
   gatagattcg tggccatctg taacccattg ttatactcag ttgctatgtc ccagaggctc 60
   tgcatccagc tagtggtggg tccctatgtc attggactca tgaataccat gactcacaca 120
   acaaatgcat tttgtctccc tttttgtggc cctaatgtca tcaatccttt cttctgtgat 180
   atgtccccct tcctttccct tgtatgtgct gataccaggc tcaataagtt ggcagttttc 240
   atcgtggctg gagctgtggg agtcttcagt ggcccgacta tcctgatttc ctacatttac 300
   atcctcatgg ccatcctgag gatgtccgct gatgggaggt gcagaacctt ttctacttgc 360
   tcttctcacc cgacagctgc tttcatctcg tatggtaccc tcttctttat ttatgtacat 420
   cccagtgcaa ccttctccct ggatctcaat aaagtagtgt ctgtgtttta cacagcagtg 480
   attcccatgc tcaacccctt catctgcag 509
<210> 2
   <211> 264
   <212> DNA
   <213> Drosophila melanogaster
<400> 2
   gcaataaata cagacaatta tatatatttt tctataattt gtttcatttt atcattttat 60
   tgattgtgaa cgaaaaagga aaaaaataaa cttagaaaaa gataacaaat aaattgctaa 120
   atttaagcgc aaaagttcaa ttaataaaaa ttagaatttt aatactaaca taatttggac 180
   tatttatata tacatacgca tatatataca actctatata tgaataatta gtaacaaatc 240
   aaatcaattt ccataacaac cgct 264
<210> 3
   <211> 367
   <212> DNA
   <213> Drosophila melanogaster
<400> 3
   tcttttttga attttattca attttaaagt acaattgcac gagtgatttt tgttgcactc 60
   gtcactaaga atatatatat atttttttgt ttttttttct ggcggttgtt atggaaattg 120
   atttgatttg ttactaatta ttcatatata gagttgtata tatatgcgta tgtatatata 180
   aatagtccaa attatgttaa tattaaaatt ctaattttta ttaattgaac ttttgcgctt 240
   aaatttagca atttatttgt tatctttttc taagtttatt tttttccttt ttcgttcaca 300
   atcaataaaa tgataaaatg aaacaaatta tagaaaaata tatataattg tctgtattta 360
   ttgcgga 367
<210> 4
   <211>483
   <212> DNA
   <213> Drosophila melanogaster
<220>
   <221> misc_feature
   <222> (1)...(483)
   <223> n = A,T,C or G
<400> 4
   ttggtttgat aatgntcnta ctccaaaaac aaaacaataa tttatactgg tcntgnttgn 60
   ttgtcataac ntcacagtag tatactcgnt cnttttggta ataatcccga aaaaactgna 120
   ccgaatccag naanatatcc ccgtcaanaa aaaaaacata taaaatatga aatggtacat 180
   aanaaatatg tccantccaa ccaaccaacc aaacaaccaa ccaacaaaca acaannacca 240
   accaacccaa aatcccaaat aaccgccaac aatccaaaat ggtaactaaa accattggtg 300
   aaaacaggat acaagccact tatcctaaca aacgccaggc tacactgaga aaataagcat 360
   cgngagttgg tatggatagc agaaattacc catattcgtg gactaaaggt ggtgtactga 420
   ttgactgatt gattgacgtg ttgggtggtg aatacatata tttcgactgc atgccaagga 480
   ata 483
<210> 5
   <211> 395
   <212> DNA
   <213> Drosophila melanogaster
<400> 5
   tgtttgtatg tctactccaa aaacaaaaca ataatttata ctgtctgttg ttgtcataac 60
   tcacatatat actcgtcttt tgtatatccg aaaaactgac cgatccaaaa atatccccgc 120
   aaaaaaaaac atataaaata tgaatgtaca taaaaatatg tccatccaac caaccaacca 180
   aacaaccaac caacaaacaa caaaaccaac caacccaaaa tcccaaaaac cgccaacaat 240
   ccaaaatgta actaaaacca ttgtgaaaac agatacaagc cacttatcct aacaaacgcc 300
   aggctacact gagaaaataa gcatcggagt tggtatggat agcagaaatt acccatattc 360
   gtggactaaa ggtggtgtac tgattgactg attga 395
<210> 6
   <211> 188
   <212> DNA
   <213> Drosophila melanogaster
<400> 6
   agtattttct ttagtttctt tgaggtgtgt tagcacactc attgttgctt tagcctagcg 60
   ctggtttatt aaatgttagc taagtttaaa ttatgtattt acagatgctg tgtgctagct 120
   cgaaagtgat aatttsgtgt tattttttgt gtatgggatt ttgataaatg ccttatgagt 180
   ttagaacg 188
<210> 7
   <211> 186
   <212> DNA
   <213> Drosophila melanogaster
<400> 7
   agtattttct ttagtttctt tgaggtgtgt tagcacactc attgttgctt tagcctagcg 60
   ctggtttatt aaatgttagc taagtttaaa ttatgtattt acagatgctg tgtgctagct 120
   cgaaagtgat aatttgtgtt atttttgtgg tatgggattt tgataaatgc cttatgagtt 180
   tagaac 186
<210> 8
   <211> 297
   <212> DNA
   <213> Drosophila melanogaster
<400> 8
   acatttccat ggtttatttt aatgtgaagt taaactgcaa atttctagtc taagcgtagt 60
   agttaagatt agccttcttc ttcgcctgca cttccatgat ggcgtccatg aagtcttcgt 120
   gtgtaaccga gttggcggag cgacgcagtg cgatcatacc agcttccaca cagacggctt 180
   tgcactgggc gccgttgaag tcatccgtgg atcgggacaa ttcctcgaaa ttcacatcat 240
   tgctaacgtt cattttacgc gagtgaatct gcataatacg ggcacgggct tcctcgt    297
<210> 9
   <211> 710
   <212> DNA
   <213> Drosophila melanogaster
<220>
   <221> misc_feature
   <222> (1)...(710)
   <223> n = A,T,C or G
<400> 9
   gatatatatt tttgtttatt ttaaaaaggt tgggtattga tttcagtacg tctcccattc 60
   tagtaaatgg ttacttttaa nagccgttcc gatgttattt tataggcgat cttcnttgtg 120
   caactcagat cgaaactgaa aaattttaca tttccatggt ttattttaat gtgaagttaa 180
   actgcaaatt tctagtctaa gcgtagtagt taagattagc cttcttcttc gcctgcactt 240
   ccatgatggc gtccatgaat ctcgtgcgta accgaattgg cggancgacg cagtgcgatc 300
   ataccacttc cacacaaacg gtttgcactg ggcgccgttg aatcatccgt ggatcgggac 360
   atcctcgaaa tcacatcatg ctaactttca tttacgcaat gaattgcata atacggccgg 420
   cttccccttg ggatttgaaa ncatctacat ccnangacca accccccaac cgatccaaan 480
   tccccaatgg tcccaatcca aggnaattcn aactcccnct gnggcccact gcntaaggcc 540
   atccccattn atcttaatcc ggcgcnttnn ctctnaggaa ccgnttccat atcctgncnn 600
   cctccntggt tacaaagccc antccccatn ccnaaggaat gaccttcgct accgggtggt 660
   ccntactntc nccaccnttc ttctctnctt cgtccacang gctnggcatg           710
<210> 10
   <211> 479
   <212> DNA
   <213> Drosophila melanogaster
<400> 10
   gatatatatt tttgtttatt ttaaaaaggt tgggtattga tttcagtacg tctcccattc 60
   agtaaatggt tacttttaag agccgttccg atgttatttt aaaggcgatc ttcaaggtcg 120
   aactcagatc gaaactgaaa aattttacat ttccatggtt tattttaatg tgaagttaaa 180
   ctgcaaattt ctagtctaag cgtagtagtt aagattagcc ttcttcttcg cctgcacttc 240
   catgatggcg tccatgaagt cttcgtgcgt aaccgaattg gcggagcgac gcagtgcgat 300
   cataccagct tccacacaga cggctttgca ctgggcgccg ttgaagtcat ccgtggatcg 360
   ggacaattcc tcgaaattca catcattgct aacgttcatt ttacgcgagt gaatctgcat 420
   aatacgggca cgggcttcct cgttgggatg tgggaactcg atcttacgat ccagacgac  479
<210> 11
   <211> 355
   <212> DNA
   <213> Drosophila melanogaster
<400> 11
   tggccccagg acgagcgttg cctcgcccga ggacgatata ccctgcccca taataatcct 60
   aaacccatac cgaccggcag gtggtcttcc agaggagacg ataacgacgt agcgtgttcg 120
   aaagggacag tggagtcagt ggtcggcaaa ggtggtccca ggacgagcgt ttgcctcgcc 180
   cgaggacgat acaccctaac ccataacatc ataatcccag ccgggccgac tcgtcgtccg 240
   tgtcaaggag caagcaggac cacggaggca aggcgttgca ggagaaatgc cgcaggagca 300
   ccgagattgc cgaagaagtt atccataagg ctgtagaata aaatactata ataag      355
<210> 12
   <211> 171
   <212> DNA
   <213> Drosophila melanogaster
<400> 12
   gtatgttaac aaaaattact aaccctataa acattaacgt ccatcgggac taaataaagc 60
   aaatgtaaca cgtctagaca ttgacataat ccctgttcaa tatcacgcaa ttttaaacca 120
   tccaacggca gcataaattt cttctccttc tcatcctcgt ccttacacac a          171
<210> 13
   <211> 170
   <212> DNA
   <213> Drosophila melanogaster
<400> 13
   tatgttaaca aaaattacta accctataaa cattaacgtc catcgggact aaataaagca 60
   aatgtaacac gtctagacat tgacataatc cctgttcaat atcacgcaat tttaaaccat 120
   ccaacggcag cataaatttc ttctccttct catcctcgtc cttacacaca            170
<210> 14
   <211> 162
   <212> DNA
   <213> Drosophila melanogaster
<400> 14
   gtatgttaac aaaaattact aaccctataa acattacgtc catcgggact aaataaagca 60
   aatgtaacac gtctagacat tgacataaat ccctgttcaa tatcacgcaa ttttaaacca 120
   tccaacggca gcataaattt cttctccttc tcatcctcgt cc                    162
<210> 15
   <211> 249
   <212> DNA
   <213> Drosophila melanogaster
<400> 15
   ddtcaatagg wraaaatata gaataataaa gatatgaaty aaagattggg taaagagata 60
   ggataaamat agtaaaggaa gaaagtgtgc atggaattag aaattaggaa ttaggttttt 120
   ttdtttttca gataaaagga maaagaagga aaaatttaaa gaaaggatat ggaaaaatga 180
   gagaagaaat tatagagaaa ataatgcatg attgagaatg aagtaagaat tgagaggaat 240
   waaattaag 249
<210> 16
   <211> 709
   <212> DNA
   <213> Drosophila melanogaster
<220>
   <221> misc_feature
   <222> (1)...(709)
   <223> n = A,T,C or G
<400> 16
   canataatgt ctctatcgct gtaataattc cancgtaaca cgaaggcaat gtgatcagta 60
   natgagaaat tttatccatc tcttctattt ttgcaccagc tgccaacaat tcnttttttt 120
   tttcgtctaa aatangaaaa tggcttaata gtgacatctc acttgatagc ttcagagaaa 180
   gcaaacgttt tcgcagcgcc agttgcgacg ccaaactttt tcgttcataa acggcgtcca 240
   aattctcaag aactgacgcg ccgtaatgtc cttgttgcga aattaaaaac aatccttagg 300
   tacaantcnc gaagggcaat tctgcanata tccatcacac tggcggcgct cgaacatgca 360
   ctananggcc aattccccta tagtgatcta ttacaatcct ggcgtcttta cactctgann 420
   ggaaaccggc ntaccaatta tcnctgacca tcccttcnca cngnttnaac aaagcccnga 480
   cccccanttg ccccgaagga aggaccctgt acgccntacc gnggtgngtn cccntactac 540
   tcnccnancn tctttctttn ctnttccctc cggtccgtaa ccatgggcct tgtcatatct 600
   cngccancna ntatnggagc cttggcccca aagntcccaa tgatctcnaa ngactcncga 660
   nccccccgcc ntaataggat cangctgnna nacataatcn catcacngg 709
<210> 17
   <211> 468
   <212> DNA
   <213> Drosophila melanogaster
<400> 17
   cagataatgt ctctatcgct gtaataattc catcgtaaca cgaaggcaat gtgatcagta 60
   gatgagaaat tttatccatc tcttctattt ttgcaccagc tgccaacaat tcacttagta 120
   agttcgtcaa aaatatgaaa atggcttaat agtgacatct cactcgatag cttcagagaa 180
   agcaaacgtt ttcgcagcgc cagttgcgac gccaaacttt ttcgttcata aacggcgtcc 240
   aaattctcaa gaatctgacg cgccgtaatg tcgcttgttg cgaaatttaa aaacgagtcg 300
   cttaggtacg aattcacgaa gggcgaattc tgcagatatc catcacactg gcggccgctc 360
   gagcatgcat ctagagggcc caattcgccc tatagtgagt cgtattacaa ttcactggcc 420
   gtcgttttac aacgtcgtga ctgggaaaac cctggcgtta cccaactt 468
<210> 18
   <211> 416
   <212> DNA
   <213> Drosophila melanogaster
<400> 18
   cattgacttg gcaaaatgaa acaaaacaaa ttgaaatcta tttgtaattt acatttaagc 60
   ctaaaaacat atgattatat caaacactta gttttagtcg ataattgttt ataatttttc 120
   agacacacac acgcaacaca cacagacaca ttcaacttaa agtgcgtaac ataaagtaaa 180
   ataaataaat gaaaacacat taacacgaac aaaacaataa tcaagaactg gagcggattg 240
   ggtttcgttt tccagcgatt acctggagat caccatggca accagtcaca ctcatttaca 300
   cttggaatgc atgggagttc ttctatcaac taacaaatcc tatttcatat acaacacgtt 360
   aactatgttt gcttggttag ttcgctttcc tgtcgcttgt tataagtaca caatat     416
<210> 19
   <211> 286
   <212> DNA
   <213> Drosophila melanogaster
<400> 19
   tcaaagcagg tgcaacgttg tacatacata tatagaaaga acaaaatgag agagatcaat 60
   ctgtaacttg aatgtggtta agtaaagagg tgcatatata tttttttaca cgcgtatata 120
   gtttgcgttt ttcgctttcc acacaagata cgtacttcgt agcccccctt cccctttcca 180
   aatactgtat cacaaagatc ataactcaaa atgctattgc tttgacttac atcttatttc 240
   ggtggtgtca actgcgccac catacgaaaa tacataaatt atagcg                286
<210> 20
   <211> 706
   <212> DNA
   <213> Drosophila melanogaster
<220>
   <221> misc_feature
   <222> (1)...(706)
   <223> n = A,T,C or G
<400> 20
   atgatacaac aagactttaa tgcatgattt gcgcagctta cactacacta caaaaatgga 60
   atacatactt ttctgcttat tggaatagtc tacacacttt tgctacatag gtacaattaa 120
   gtttgtggct tgccctttgc gaattacaat atggaaacgg atacagaaca gaaaatagtt 180
   taacaataat attgctggaa taaacacatc caaggtaata ctcagacagc actcgtcatc 240
   gccctcatcc angatattgg cctgctggcg cacatcgatg ccctgctgca caactccgcc 300
   ttcttggctt cggcttgaag ncttnccccc ctcctgttcn ggatctcctc antccgtaaa 360
   accctccccn caactctcca ctccaaatga tttnggcaat tcnatcaatc cggganaatc 420
   catgcccatg gctttngtat tcccctccct tggcactncn aacccccggn taaacgcatt 480
   cctgtgttca ttcaatccaa ggnaatccgc attctcnctg nggcctcact ctctaaggcc 540
   atcccnaata tctntaatcc ggcgctttaa nctatggaac ngntacatac ctgacatcct 600
   tccatggtaa caaagcccat ccccaatncc cangangacc ctcgctaccg ggttggtcct 660
   actatnncac ccctnttctc ttcttcgtcc anatggctng tctttt                706
<210> 21
   <211> 459
   <212> DNA
   <213> Drosophila melanogaster
<220>
   <221> misc_feature
   <222> (1)...(459)
   <223> n = A,T,C or G
<400> 21
   atgatacaac aagactttaa tgcatgattt gcgcagctta cactacacta caaaaatgga 60
   atacatactt ttctgcttat tggaatagtc tacacacttt tgctacatag gtacaattaa 120
   gtttgtggct tgccctttgc gaattacaat atggaaacga tacagaacag aaaatagttt 180
   aacaataata ttgctggaat aaacacatcc aaggtaatac tcagacanca cgtcgtcatc 240
   gccctcatcc aggatattgg cctgctggcg cacatcgatg ccctgctgca gcaactccgc 300
   cttcttggcg tcggccttga tgcgtgcctc gcgcttcttg tcctggatct tcttcagtcg 360
   gtagaactcc tcacgctcga gctcgtccag ctccganatg atgtaggcca aagtcctatc 420
   gattcggggg atgatcacat gctcaatggc gttttggta                        459
<210> 22
   <211> 483
   <212> DNA
   <213> Drosophila melanogaster
<400> 22
   cgggcataaa gtaggtggga aggtaaggaa ggtactaagc gcactccaaa tctgtttggt 60
   aaacattgta gacgaagcat gtggaattaa agccaaacac gataattgtg ccgagactct 120
   tggccagaga ttgtcaaggt cgtgcatctt acgcgagtaa atcaaggaaa atgtgagcag 180
   gttaaagaaa atttctacct actaaaaaca atattaatgc atctccaaat attagtttct 240
   tcctacagga tggtagatgg ttttggaaat gtatcttttt atgtaacctg ctctttggtg 300
   tcagatccga attcacgaag ggcgaattct gcagatatcc atcacactgg cggccgctcg 360
   agcatgcatc tagagggccc aattcgccct atagtgagtc gtattacaat tcactggccg 420
   tcgttttaca acgtcgtgac tgggaaaacc ctggcgttac ccaacttaat cgccttgcag 480
   caa                                                               483
<210> 23
   <211> 514
   <212> DNA
   <213> Drosophila melanogaster
<220>
   <221> misc_feature
   <222> (1)...(514)
   <223> n = A,T,C or G
<400> 23
   cggctctaat ttcattttgt gcatattttg gtcctggttc tggtgcctat ccctcctttt 60
   tggntcggcc cgtgcaggag cttaattaat tcccccaaaa atatttataa ctttgggscc 120
   aatacggctg ctgttgctgc tgctgactac tgaracatat ttaatttata tttcttggag 180
   tgtgtgcggc ttgtcaatgg ctgggaatct aagaaattta tgcatgactg caacagggtc 240
   aagttgcaaa gcccttagcc tttaatgcca tccagctgcc gggaaagccg ggaaagctga 300
   naaaacaaaa ctgactcctt actgaagctg aaactgaaag aacttttagt cctatccagg 360
   gttgcggatg gatccaactc cccagataag cagatttatg acctaaacac cgaaactcca 420
   atactggaaa nacaatcngt tttcngtttc gtactggatc cgaatcncaa aggcaaatct 480
   gcnattcctc accgcgggcg cycaacatct ctaa                             514
<210> 24
   <211> 430
   <212> DNA
   <213> Drosophila melanogaster
<220>
   <221> misc_feature
   <222> (1)...(430)
   <223> n = A,T,C or G
<400> 24
   cggctctaat ttcattttgt gcatattttg gtcctggttc tggtgcctat ccctcctttt 60
   tggctcggcc cgtgcaggag cttaattaat tcccccaaaa atatttataa ctttgggccc 120
   aatacggctg ctgttgctgc tgctgactac tgaaacatat tttaatttat atttcttgga 180
   gtgtgtgcgg cttgtcaatg gctgggaatc taagaaattt atgcatgact gcaacagggt 240
   caagttgcaa agcccttagc ctttaatgcc atccagctgc cgggaaagcc gggaaagctg 300
   agaaaacaaa actgactcgt actgaagctg aaactgaaag aacttttagt cctattccrg 360
   gggttncgga tggatccaac yccccagata agcagattta tgacctaaac accgaaactc 420
   aaataactgg 430
<210> 25
   <211> 213
   <212> DNA
   <213> Drosophila melanogaster
<400> 25
   aacattttag attgaaacac attccaaaag tctaagactc tagcttcaca acggtcgtct 60
   tctcggacac gtacagbbcg tcaaggaact tacggatatc cttgttcttg acgstcgtgg 120
   actgctggat gagggcggca gatccggaga cagactcaat atcgttccgt amscgtaagg 180
   tyggccctct ggavagtgag gtcacccacc gcg 213
<210> 26
   <211> 365
   <212> DNA
   <213> Drosophila melanogaster
<400> 26
   aacattttag attgaaacac attccaaaag tctaagactc tagcttcaca acggtcgtct 60
   tctcggacac gtacagaccg tcaaggaact tacggatatc cttgttcttg acggtcgtgg 120
   actgctggat gagggcggca gatccggaga cagactcaat atcgtttccc tccacgataa 180
   gttcgtcctt ctgggcagtg gagttgacca cggtgacgcc aggagccatc tccacacgac 240
   ggatgtactt ctcacccaag aagttacgga tctcaatgac cgtgttgttc tcggaggtga 300
   cacagttgat ggggaaatgg gcgtacacag cacgcatctt gtactggatc cgaattcaca 360
   aaggg 365
<210> 27
   <211> 212
   <212> DNA
   <213> Drosophila melanogaster
<400> 27
   acattttaga ttgaaacaca ttccaaaagt ctaagactct agcttcacaa cggtcgtctt 60
   ctcggacacg tacagbbcgt caaggaactt acggatatcc ttgttcttga cgstcgtgga 120
   ctgctggatg agggcggcag atccggagac agactcaata tcgttccgta mscgtaaggt 180
   yggccctctg gavagtgagg tcacccaccg cg                               212
<210> 28
   <211> 691
   <212> DNA
   <213> Drosophila melanogaster
<220>
   <221> misc_feature
   <222> (1)...(691)
   <223> n = A,T,C or G
<400> 28
   atgatacaac aagactttaa tgcatgattt gcgcagctta cactacacta caaaaatgga 60
   atacatactt ttctgcttat tggaatagtc tacacacttt tgctacatag gtacaattaa 120
   gtttgtggct tgccctttgc gaattacaat atggaaacgg atacagaaca gaaaatagtt 180
   taacaataat attgctggaa taaacacatc caaggtaata ctcagacagc actcgtcatc 240
   gccctcatcc angatattgg cctgctggcg cacatcgatg ccctgctgca caactccgcc 300
   ttcttggctt cggcttgaag ncttnccccc ctcctgttcn ggatctcctc antccgtaaa 360
   accctccccn caactctcca ctccaaatga tttnggcaat tcnatcaatc cggganaatc 420
   catgcccatg gctttngtat tcccctccct tggcactncn aacccccggn taaacgcatt 480
   cctgtgttca ttcaatccaa ggnaatccgc attctcnctg nggcctcact ctctaaggcc 540
   atcccnaata tctntaatcc ggcgctttaa nctatggaac ngntacatac ctgacatcct 600
   tccatggtaa caaagcccat ccccaatncc cangangacc ctcgctaccg ggttggtcct 660
   actatnncac ccctnttctc ttcttcgtcc a                                691
<210> 29
   <211> 677
   <212> DNA
   <213> Drosophila melanogaster
<220>
   <221> misc_feature
   <222> (1)...(677)
   <223> n = A,T,C or G
<400> 29
   cgggcataaa gtaggtggga aggtaaggaa ggtactaagc gcactccaaa tctgtttggt 60
   aaacattgta nacnaagcat gtggaattaa agccaaacac natttttntg ccnatactct 120
   tggccagaga ttgtcaaggt cgtgcatctt acgcgagtaa atcaaggaaa atgtgagcan 180
   gttaaagaaa atttctacct actaaaaaca atattaatgc atctccaaat attagtttct 240
   tcctacagga tggtagatgg ttttggaaat gtatcttttt atgtacctgc tctttggtgt 300
   canatccnaa tcncgaaggg caattctgca aatatccaca cctggcgggc cgctcgaaca 360
   tcntctaaan ggccaatccn ccnattatga atcctatana atcnctggcc gtcttttaca 420
   ctctganggg aaaccnggcn ttnccactaa ccctgcacct cccttccnct gnttatacaa 480
   aagccncatc cctccacatt gcccctaagn atgacccctt cgcctanccg gggtntgttc 540
   cntactcttc nnctaccccc tcttcctctt ccnttcggtc cnactaaggg cctggcattt 600
   tgcccccaat aaggngnctt gcccnaagtc ccaatgtctc nangactccg aaccccnccc 660
   ctaaaggacn cctgaaa 677
<210>30
   <211>141
   <212> DNA
   <213> Drosophila melanogaster
<400> 30
   atgatataat ggattggtaa tcaattggca tcgaaattaa tttacgatat aaacaccact 60
   taacgccgcc tcaacctaat tactgtctgc atatgcaata gaaaacgtat ataaattaat 120
   taaataaaaa aaaaggaaag t 141
<210> 31
   <211> 322
   <212> DNA
   <213> Drosophila melanogaster
<220>
   <221> misc_feature
   <222> (1)...(322)
   <223> n = A,T,C or G
<210> 32
   <211> 308
   <212> DNA
   <213> Drosophila melanogaster
<220>
   <221> misc_feature
   <222> (1)...(308)
   <223> n = A,T,C or G
<400> 32
   acgcatacaa tatatgatta tacatacata tatatattta caatgataaa gaatgtaagg 60
   cccaagccaa gcaaacacat atgtaacgtg tatttgaacc acgtacttat tatttacatg 120
   tttacatata cgaacatcca aagcaaaggt atatacacgt ataggactca acatttacaa 180
   attcaatatt cttatatgtg gaaagcanag cgttacgatt atctcccanc taactggaag 240
   cgattgaatg tctatacatn atttgtaatg ccaaataaaa taaaatatat cacgttatat 300
   taaacagt 308
<210> 33
   <211> 201
   <212> DNA
   <213> Drosophila melanogaster
<400> 33
   acgcatacaa tatatgatta tacatacata tatatattta caatgataaa gaatgtaagg 60
   cccaagccaa gcaaacacat atgtaacgtg tatttgaacc acgtacttat atatttacat 120
   gtttacatat acgaacatcc aaagcaaagg tatatacacg tataggactc aacatttaca 180
   aattcaatat tcttatatgt g 201
<210> 34
   <211> 187
   <212> DNA
   <213> Drosophila melanogaster
<400> 34
   acgcatacaa tatatgatta tacatacata tatatattta caatgataaa gaatgtaagg 60
   cccaagccaa gcaaacacat atgtaacgcg tatttgaacc acgtacttat atatttacat 120
   gtttacatat acgaacatcc aaagcaaagg tatatacacg tataggactc aacatttaca 180
   aattcat 187
<210> 35
   <211> 687
   <212> DNA
   <213> Drosophila melanogaster
<220>
   <221> misc_feature
   <222> (1)..(687)
   <223> n = A,T,C or G
<400> 35
   agaattacca cgcgaacaca attctgtttt attgttttta atacatattt aatctttgcg 60
   anaagagcta gtgtaggtag tctggaattt ttcatatatt taacgatatc cattggtaat 120
   gattacatag ttggattaga actaatactt gtagcagtta atggaatgtt caccaccgct 180
   ctggatcatc gttgctggtc agctggcaag gcatcatcac gcacttttcc atgcggacgc 240
   natccttgca cttgtggctc aatcggtgtt cattaaggtt cgggttcgtt ggcgaacggc 300
   attatcgcca cacgttgcgg tgcatggtgt ccaagcggaa cactcccaat tancnacact 360
   cgtcctgcgg tccggttgcn gactcttacc acatccttcc tctccaatcc ccgtccctga 420
   ttgattacnn tcatccaccc ctggtaacac nattccaact tccagttgct tggaaatgct 480
   gcnccctact ccgaatacga cnctcccttc ccatgaaccn ccccagagct tgcacgtgga 540
   ccntcatcat ccaagnaatc tgcattctcc cgcggcncac tcttaagcca tccccaatat 600
   cttaatccgc ccttaatcta tgaaacgntt ccatacctgn cancctccct ggtaaaaanc 660
   ccatctccct tnccnangan gaccctc 687
<210> 36
   <211> 311
   <212> DNA
   <213> Drosophila melanogaster
<220>
   <221> misc_feature
   <222> (1)... (311)
   <223> n = A,T,C or G
<400> 36
   tcccatcaat tcgttactca tcaattgaaa tttcagattt ggtaatgcta aagggctatc 60
   atgattgcag ttctatgaag tggatcaaag cgatttcggg tcaaagattg cgggtcgctg 120
   ctagaaagat tgatctctag tgcttctcca gtgcttgctt agttcggcga gggcataacc 180
   ttgatgcgct ccaaggcttg tttctccang gtctcgcggt gcttgggatc ggcgatctgg 240
   ataagttcgt acatcctctg gcgcacattc ttgccgaaca gcgaagcgat tccatgctcc 300
   gtgacgactt a                                                      311
<210> 37
   <211> 670
   <212> DNA
   <213> Drosophila melanogaster
<220>
   <221> misc_feature
   <222> (1)...(670)
   <223> n = A,T,C or G
<400> 37
   cccatcaatt cgttactcat caattgaaat ttcagatttg gtaatgctaa agggctatca 60
   tgattgcagt tctatgaagt ggatcaaagc gatttcgggt caaanattgc gggtcgctgc 120
   tagtaaaata gtgatctcta gtgcttcttc agtgcttgct tagttcggcg agggcataac 180
   cttgatgcgc tcgaagcttg tttctccagg gtctcgcggt gcttgggatc ggcgatctgg 240
   ataagttcgt acatcctctg gngcacattc ttgccgaaca cgaagcgatt ccntgctccg 300
   tgacnactta ntggacttng gcacgcgaan ttgacaaccc agcgcctgcc ttcacgttng 360
   gaacaatctt gctctcccct tgttggtggt caatgcatgg cnataattgc acacccatcc 420
   atcnaaacct ccncgtcccc naatnaattc acctntcccc naaccgggat taaanccgga 480
   acatcatcta cncctgtcnt ccattccaat ccaagggaat ctnnattcac cngcgggcnc 540
   caacatctcn aaggccatcc caatatnttt anatccggct cttaactcta tggaacnnct 600
   tncataacct gantccttcc ctgtttcaag ccncatcccc ncttcccaag ataccctcgc 660
   taacgggtng                                                        670
<210> 38
   <211> 192
   <212> DNA
   <213> Drosophila melanogaster
<400> 38
   accatttaat tattaaatat gatttattta tattaatatg tagtcaaaaa ctccgtgtta 60
   gctttaattt acctacccca ctttggatct aaataaatat gttaaatgtt gattcaagcg 120
   tgataattta tttggaacag cattgcgaaa attgrgtagt ycataatgtt ttttcttcct 180
   ggkcactgag ca 192
<210> 39
   <211> 362
   <212> DNA
   <213> Drosophila melanogaster
<220>
   <221> misc_feature
   <222> (1)...(362)
   <223> n = A,T,C or G
<400> 39
   gctgaactgg acctgaatat aaacntatac acatctattg caacaangat acacaccttg 60
   ctgttaacca cctgcaacat ccaancttct tacatccctg gtgttagttc gacanactct 120
   acatttcccc acctctgccg antgctgana gttaantcat gggaacagga natnccnctt 180
   ccccaaaggg aatattttnt gttnaaataa atactgcctc ttgcngttca acgtananan 240
   anaaataccn aattccgaaa ggggccnaan ttnccgggcn canannggcc tgcctcntag 300
   ggaatcncca nccccttntt atangccctc ttccgcctat aaacttgtgc cngaancccc 360
   ng 362
<210> 40
   <211> 322
   <212> DNA
   <213> Drosophila melanogaster
<220>
   <221> misc_feature
   <222> (1)...(322)
   <223> n = A,T,C or G
<400> 40
   atttcncgac aggcttcggc acgccagtat ataacccaaa acacacaaac gtcaggggct 60
   ggaacgcgtc actgccgtgc tcctccagcc ggcacagtca ttccccgccc ccacaccaag 120
   caaaaccggc cgcttgtgca gatgacatag gcgcgaccag ccaactgacc cggctgacca 180
   nacttgcacc gtgcgccatc aactggaatc ttggccacaa gcacagcaat agtttggccc 240
   gctatcccca cacanaaacc cacantgggg gtcnatggaa gaacacaagt ggttgcgtgg 300
   aactgctaaa aatataaaac tg 322
<210> 41
   <211> 323
   <212> DNA
   <213> Drosophila melanogaster
<220>
   <221> misc_feature
   <222> (1)...(323)
   <223> n = A,T,C or G
<400> 41
   atttcgcgac aggcttcggc acgccagtat ataacccana acacacaaac ntcaggggct 60
   ggaacgcgtc actgccgtgc tcctccagcc ggcacagtca ttccccgccc ccacaccaag 120
   caaaaccggc cgcttgtgca gatgacatag gcgcgaccag ccaactgacc cggctgacca 180
   gacttgcacc gtgcgccatc aactggaatc ttggccacaa gcacagcaat agtttggccc 240
   gctatcccca cacagaaacc cagantgggg gtctatggaa gacnacaagt ggttgcgtgg 300
   aactgctaaa aatataaaac tgt                                         323
<210> 42
   <211> 176
   <212> DNA
   <213> Drosophila melanogaster
<400> 42
   caagtgcggc ggcgacaaga aatccgcctg cggctgctcc aagtgagctt tcccccaaaa 60
   aagatctgga gtagaggcgc tgcatcttgt ctccgaactg atttctgtat aactcccaat 120
   actaaaacga catgttttct catttacaca ccctgcaata aatgtccaat taaagt     176
<210> 43
   <211> 323
   <212> DNA
   <213> Drosophila melanogaster
<220>
   <221> misc_feature
   <222> (1)...(323)
   <223> n = A,T,C or G
<400> 43
   atttcgcgac aggcttcggc acgccagtat ataacccaaa acacacaaac gtcaggggct 60
   ggaacgcgtc actgccgtgc tcctccagcc ggcacagtca ttccccgccc ccacaccaag 120
   caaaaccggc cgcttgtgca gatgacatag gcgcgaccag ccaactgacc cggctgacca 180
   gacttgcacc gtgcgccatc aactggaatc ttggccacaa gcacagcaat agtttggccc 240
   gctatcccca cacagaaacc cacantgggg gcctatggaa gaccacaagt ggttgcgtgg 300
   aactgctaaa aatataaaac tgc                                         323
<210> 44
   <211> 176
   <212> DNA
   <213> Drosophila melanogaster
<400> 44
   caagtgcggc ggcgacaaga aatccgcctg cggctgctcc aagtgagctt tcccccaaaa 60
   aagatctgga gtagaggcgc tgcatcttgt ctccgaactg atttctgtat aactcccaat 120
   actaaaacga catgttttct catttacaca ccctgcaata aatgtccaat taaagt     176
<210> 45
   <211> 323
   <212> DNA
   <213> Drosophila melanogaster
<220>
   <221> misc_feature
   <222> (1)...(323)
   <223> n = A,T,C or G
<400> 45
   atttcgcgac aggcttcggc acgccantat atancccaaa acacacaaac gtcaggggct 60
   ggaacgcgtc actgccgtnc tcctccancc ggcacngtcn ttccccgccc ccacaccaag 120
   canaaccggc cgttgtgcag atgacataag cgcgaccanc caactgaccc ggctgaccag 180
   acttgcaccg tgcgccatca actggaatct tggccacaag cacagcanta gtttggcccg 240
   ctatccccac acatanaacc cagattgggg gvvtatngaa naacacaagt ggttgcgtgg 300
   aactgctaaa natatnaaac tgc                                         323
<210> 46
   <211> 362
   <212> DNA
   <213> Drosophila melanogaster
<220>
   <221> misc_feature
   <222> (1)...(362)
   <223> n = A,T,C or G
<400> 46
   gctgaactgg acctgaatat aaacntatac acatctattg caacaangat acacaccttg 60
   ctgttaacca cctgcaacat ccaancttct tacatccctg gtgttagttc gacanactct 120
   acatttcccc acctctgccg antgctgana gttaantcat gggaacagga natnccnctt 180
   ccccaaaggg aatattttnt gttnaaataa atactgcctc ttgcngttca acgtananan 240
   anaaataccn aattccgaaa ggggccnaan ttnccgggcn canannggcc tgcctcntag 300
   ggaatcncca nccccttntt atangccctc ttccgcctat aaacttgtgc cngaancccc 360
   ng                                                                362
<210>47
   <211> 416
   <212> DNA
   <213> Drosophila melanogaster
<400> 47
   agtttacatg tactttattc gttttgtata tcccagacag atagagttat ttattgaaca 60
   cttcaactgg ctaggtcgta ttagggtctg cttgtaactt ttgtgtcagt aaccactcta 120
   aaatagtata atgctagtaa ttctacccat caacccattg tatacatact tatattcaaa 180
   accctttcac cacatttcta agcctagatt atggataatg cctctaatat gtaacgagtg 240
   cttaggtcac cttagccagc cgctggtcga tgcatttctg gctgcgaagg tcgaaccaat 300
   ttcccggact gcagtaatgc aaaaccgctt ttcccttcaa gcaaacataa tacttgttat 360
   gctgcttgac gtctccaaat cgtgtatcct ctttcacttt ggtgcaatcg ggtacc     416
<210> 48
   <211> 413
   <212> DNA
   <213> Drosophila melanogaster
<400> 48
   caaatagttt acatgtactt tattcgtttt gtatatccca gacagataga gttatttatt 60
   gaacacttca actggctagg tcgtattaga gtctgcttgt aacttttgtg tcagtaacca 120
   ctctaaaata gtataatgct agtaattcta cccatcaacc cattgtatac atacttatat 180
   tcaaaaccct ttcaccacat ttctaagcct agattatgga taatgcctct aatatgtaac 240
   gagtgcttag gtcaccttag ccagccgctg gtcaatgcat ttctggctgc gaaggtcgaa 300
   ccaatttccc ggactgcagt aatgcaaaac cgcttttccc ttcaagcaaa cataatactt 360
   gttatgctgc ttgacgtctc caaatcgtgt atcctctttc actttggtgc aat        413
<210> 49
   <211> 885
   <212> DNA
   <213> Drosophila melanogaster
<220>
   <221> misc_feature
   <222> (1)...(885)
   <223> n = A,T,C or G
<400> 49
   rtnstartmn ctmrtnsttt ctamcmmntd skasamdsdy strmrdtaca stanyrmrma 60
   chndsnnnng nagatacgcc aagctattta ggtgacacta tagaatactc aagctatgca 120
   tcaagcttgg taccgagctc ggatccacta gtaacggccg ccagtgtgct ggaattcgcc 180
   cttcgtgaat tcggatctga ctgcaagtgc ggcggcgaca agaaatccgc ctgcggctgc 240
   tccaagtgag ctttccccca aaaaagatct ggagtagagg cgctgcatct tgtctccgaa 300
   ctgatttctg tataactccc aatactaaaa cgacatgttt tctcatttac acaccctgca 360
   ataaatgtcc aattaaagta aaaaaaaaca aaaaaaaaaa accgaattcc gaagggcgaa 420
   ttctgcagat atccatcaca ctgggggccg ctcgagcatg catctagaag gcccaattcg 480
   ccctatagtg attcgtatta caattcactg gccgtcgttt tacaacgtcg tgactgggaa 540
   aacctgggtt tacccaactt aatcgccttg cacacatccc ctttcgccag ctggcntnta 600
   caaaaaggcc cncgattgcc ttcccacant gccacctgaa tgggaatgaa ccccccgtac 660
   cggccttaac cgnggttggt ggttacccac ntacgcaacn tgcaccccta cccnncttcc 720
   ttttcctctt cccnttccgg ttccctcacc tantggggcc taggtcaatt tcttnngcca 780
   ccaaatntag tangtctttg cccccaaaag ttccctaatt gatcttctaa atganntcnn 840
   gaaaccncac cgtntttant aaggatgcat cgcnngtaaa catcc                885
<210> 50
   <211> 496
   <212> DNA
   <213> Drosophila melanogaster
<400> 50
   cttgatccag caatctattt ttcacaaacg ccaatgtcaa attttcttca gataatgtct 60
   ctatcgctgt aataattcca tcgtaacacg aaggcaatgt gatcagtaga tgagaaattt 120
   tatccatctc ttctattttt gcaccagctg ccaacaattc acttataagt tcgtcaaaaa 180
   tatgaaaatg gcttaatagt gacatctcac tcgatagctt cagagaaagc aaacgttttc 240
   gcagcgccag ttgcgacgcc aaactttttc gttcataaac ggcgtccaaa ttctcaagaa 300
   tctgacgcgc cgtaatgtcg cttgttgcga aatttaaaaa cgagtcgctt aggtacgaat 360
   tcacgaagcc gaattctgca gatatccatc acactggcgg ccgctcgagc atgcatctag 420
   agggcccaat tcgccctata gtgagtcgta ttacaattca ctggccgtcg ttttacaacg 480
   tcgtgactgg gaaaac                                                496
<210> 51
   <211> 936
   <212> DNA
   <213> Drosophila melanogaster
<220>
   <221> misc_feature
   <222> (1)...(936)
   <223> n = A,T,C or G
<400> 51
   acatcaatgc tagtgcttcc ttttaccgaa aacctattga atacgctaaa aaattggaat 60
   agtcgcaagc ggaagtcggc caaaaaaatc cttaagaatt ttggaaccag ttcttctact 120
   tgtcgtatcg aaccaggcgc gtgtcgtcgc cgacctcctc cagatccttt ggatcgcggc 180
   ggaagcgata agtgcccaca tcctggttgg ccgattccgg caacgtcacc ttgatgccct 240
   tgtactcggt tcgaccttcc ctgacctccg gcacccgcag ctccatctcg gccttgtact 300
   cgtcatcgtt accaatgtcc acgtcctgga ccgttctttt gcacggtggg atcctcctcg 360
   tcctggttcc agccatcaaa tctcgatggg gacaatgggg ttgccgtcga cgcctacgac 420
   ggnactangt gcgccantag ggcaggatct ccacgggtaa tctccagaaa atcggaattc 480
   tctggctggg ttggcagact caaactgcan tcccgcantc cacnaatgtt tgggtcanct 540
   ccntttgaaa tgggaggtat gggtccatca aggnagcgaa attcacnaaa nggggnaatt 600
   ctgcannata tccatcacac tggngggccg ctccaagcaa tgcatctaaa agggccccaa 660
   ttcctcccta atangngagt ccgtattaca aattcaacng ggccgtcgtt ttanaanngt 720
   cgggaatggg gaaaaacccn gggngntaan caaacttaat ccnccttgga agcanaatcc 780
   cccttttcgc aagangggng tatnannaaa nagggccgca acgantgncc cttcccaana 840
   antttccnan cctgaatngn gaatggacnc nccctgtnnn ggggcaatna acccggnggg 900
   gttgntggta nccncaangt ntacggctaa anttgc                           936
<210> 52
   <211> 629
   <212> DNA
   <213> Drosophila melanogaster
<220>
   <221> misc_feature
   <222> (1)...(629)
   <223> n = A,T,C or G
<400> 52
   gtttgcaaac cttcctattt aagtaaagtg tttgactctg gctcccaaag cttnccttgg 60
   gaaacgggaa aaattctcta cantgtatat gtgcgcatgc aaactcattt ggtaaattac 120
   acatnaataa atatgtataa caacaactan acatatgtnn atggaaaata aaaattttca 180
   gtaacgactn aactcgantg tcggtagcat naaggganna agtcgtcnan tgttattatc 240
   taatttgcag cctgtattgt ccagatacaa tatgtnatng atgcantgta tatctnttgt 300
   gtacatanat atatgtttaa ggcgactcct atttntctgc ntgtgcatat cgatcaaatg 360
   cctactttcn tgattgtttn gtgtgtttcc nctaaggaaa anatacatgt gttatatcny 420
   naaaagaatt gtatcgtatt aggtttgctt cctcaaacat ccaccaaaaa tcgntntcnt 480
   ntananccna aaaatacgaa aatnnttgtg ccttaaaaan aaacaatcga ggnaatccca 540
   antccnaatg cggngtcact cngntaccat atgctcnaan cttccctggt tcaaagccca 600
   tncccacttn cccatganga ccttcgctg                                   629
<210> 53
   <211> 977
   <212> DNA
   <213> Drosophila melanogaster
<220>
   <221> misc_feature
   <222> (1)...(977)
   <223> n = A,T,C or G
<400> 53
   cgtttgttgc cggtattgtt ggttggtagg ttgtttgtta gtagagagag agagaaccgg 60
   tacgctataa aactacgctc ccattgccgg attgttattg gagaattgcg cccgccaccc 120
   aagcagccac ccacgtatca cccgctcaca agagcggaaa atggatacag tccgggttcc 180
   tggcggtaga accgtaattt ctgtgatttg ctttttttgt gttaagtaag tatttaataa 240
   gtagattact gangtttgct gctccgcggg cgattccctt aggcggccac ttcgctangc 300
   ctcggnccca ttctgaacct catcctttgt gctgggcctc atcaagcanc gaattcacna 360
   agggcgaatt ctgcagatat ccatcacact ggcggccgct cgagcatgca tccgagaggg 420
   cccaattccg cccctaatag ntgantccct attacaattc actgggccgg tcgtttttaa 480
   naaccggtcn ntgactgggg aaaaccctgg gcggttnccc aaacttaatt cnccttgcaa 540
   gcacantcnc ccctttcgcc aagctgggng taattancga aaagnaggcc cgcacccgat 600
   nggcccttcc caacnngttg cgcaggccng aaannggccg anatggancg cgccccggtn 660
   agccggngca attaatccgc nggnggggtg ttggtgnggt taanccgcaa accgtgaccg 720
   gcntatacct tgccaagggc ccctanctga ccnggntcnt tttcggcttt cnttcncctt 780
   ccttttnctn ggcnaaantt cgnncgggtt ttcnccggtc aaagctcnta aatnnggggg 840
   gntccctttt agggnttccn natttnaggg gctttnacgg gnaanctcca anccccaaaa 900
   aancttgctt nnnggtgaan gggtnnacgt tnntggggca ncncccctna taaagggntt 960
   tnccnctttg nagatgt                                                977
<210> 54
   <211> 875
   <212> DNA
   <213> Drosophila melanogaster
<220>
   <221> misc_feature
   <222> (1)...(875)
   <223> n = A,T,C or G
<400> 54
   gcgatcttac aaaataaata acagcaaata gaaagataaa cttacatata agcgcaatat 60
   tcaaatgttt agtggcgtct acgaaatgtt tttcaattac tgctggtgta agacacatag 120
   ataataaatg tgatgtgttt tgtgtgtttt tttangtttg gcctaccaga agtgtgctct 180
   aaatatatac caatgtgaat cgaaatcgta gctccttgcg ttctcctata tacatgtgca 240
   ccgtgagatc catagtccca tcgttttcgg tttaagttac ccycgggcyy yggcagattc 300
   gnaatcatat gcacgtataa agatagactg cgtgcacagc tccggccctc ctcctgggaa 360
   aacgcatagc cataccgaat tatccgatcc caangcatac atgggtagaa ngatctcggg 420
   tccgttcatc aacttcggga natgtcgcnn cgntccggtc tccgtttccg cgaacagcct 480
   tccggtcagt gtcctannnc acgggtatta aggtaccaag tttgcaagat cacatcgatc 540
   agcagcgtgg gtaaatgngg gcaccagcag tcaaggcang cgaattccac cnaangggcg 600
   aaattccggc aagaataatc catcacactg gggggccggc tcgaagcatg caatcctaga 660
   aggggcccaa aattccgccc natattgagg tccatattan aaaagttcaa tgggccgtcc 720
   gntttannaa acgttcntga ntgggaaaaa ncccnggcgt ttacccaact taaatcnccc 780
   ttncaagnaa atnccccttt tcagcnaanc tgggcgtaat nnncnaaana ngncccgcac 840
   cggntgcccc tttcccaaca atttngccca agnct                            875
<210> 55
   <211> 465
   <212> DNA
   <213> Drosophila melanogaster
<220>
   <221> misc_feature
   <222> (1)...(465)
   <223> n = A,T,C or G
<400> 55
   ggggtcgtac tcggtgagga aatccaagcg cttatcatgc ttcactttgc agacaatcag 60
   tacatcgatt gatgaggaaa aagaagaccc cttgaatggg tcgataatca ttactgtcca 120
   actcgattag agctccctcg ttgaggaagg tcttgccttc cagattgcca ttgaagccct 180
   ggaccatttc cttgaccgcc cgcgtggcat ggctattctc cagatcctcc gtcgccgtan 240
   tgctctccgc ctccaaactc tctgccttca ggtgactgga agtcttgcca tccgtcatgg 300
   tggccanaat attgcgctgc tcaatcagaa tgtgcgacag ttgatacatt tccgactcga 360
   gatgtgatat ctccttggnc gtctgtataa actccatata gttctttttg catgtttgct 420
   tgagcgttgc tgccgtcgtt tcgttgtagg cctcgatttc ctttt                 465
<210> 56
   <211> 238
   <212> DNA
   <213> Drosophila melanogaster
<220>
   <221> misc_feature
   <222> (1)...(238)
   <223> n = A,T,C or G
<400> 56
   tgctgcctgc tccttttggg actcctgggc ttcctagctg ctcccggcgt cgcctcgcca 60
   tctcgccaca ctggaccagg aaacggatcg ggatctggag ctgggtccgg aaatccgttc 120
   aggtctccaa gctcacagca acgaccactg tactacgacg ctccgattgg gaaaccatcn 180
   aagactatgt acgcctgacg tanagaatga aacaanaaag atttgaaacn cctanact   238
<210> 57
   <211> 237
   <212> DNA
   <213> Drosophila melanogaster
<220>
   <221> misc_feature
   <222> (1)...(237)
   <223> n = A,T,C or G
<400> 57
   gctgcctgct ccttttggga ctcctgggct tcctanctgc tcccggcgtc gcctcgccat 60
   ctcgccacac tggaccagga aacggatcgg gatctggagc tgggtccgga aatccgttca 120
   ngtctccaag ctcacagcaa cnaccactgt actacgacgc tccgattggg aaaccatcga 180
   agactatgta cgcctgacgt aaagaatgaa acaataaaga tttgaaacgc ctaaact    237
<210> 58
   <211> 238
   <212> DNA
   <213> Drosophila melanogaster
<400> 58
   tgctgcctgc tccttttggg actcctgggc ttcctagctg ctcccggcgt cgcctcgcca 60
   tctcgccaca ctggaccagg aaacggatcg ggatctggag ctgggtccgg aaatccgttc 120
   aggtctccaa gctcacagca atgaccactg tactacgacg ctccgattgg gaaaccatcg 180
   aagactatgt acgcctgacg taaagaatga aacaataaag atttgaaacg cctaaact 238
<210> 59
   <211> 253
   <212> DNA
   <213> Drosophila melanogaster
<400> 59
   attacgtccc tgccctttgt acacaccgcc cgtcgctact accgattgaa ttatttagtg 60
   aggtctccgg acgtgatcac tgtgacgcct tgcgtgttac ggttgtttcg caaaagttga 120
   ccgaacttga ttatttagag gaagtaaaag tcgtaacaag gtttccgtag gtgaacctgc 180
   ggaaggatca ttattgtata atatccttac cgttaataaa catttgtaat tatacaaata 240
   aaaacaattt acc 253
<210> 60
   <211> 236
   <212> DNA
   <213> Drosophila melanogaster
<220>
   <221> misc_feature
   <222> (1)...(236)
   <223> n = A,T,C or G
<400> 60
   aacaggcaaa agcgatatca gtaataaact aaacgcacca attgtttaaa taaccaaagc 60
   gttaagaaaa aaatcaaaga caaagccacg gcaaaaggcg cagacaacaa gttgtttgct 120
   tttagttcgc gttctcctta ttttattttc cttccgttcg attttccacg cacgcgcgtc 180
   gcagaaacgt caaattgaaa acatcancag ttgaaagcca actgttgcat tctacc 236
<210> 61
   <211> 247
   <212> DNA
   <213> Drosophila melanogaster
<220>
   <221> misc_feature
   <222> (1)...(247)
   <223> n = A,T,C or G
<400> 61
   ttcaggcatc ttccttctaa ttctggctgt gggtttggca caaatgccgc tgcaggtggc 60
   cgcccagggc caaaatggac attcgcaggg acagccgcca agaccgccaa atggcaatgg 120
   aaacggcaac canncagagt ggacaaggac aaagcgggca gaacaactag aactgggata 180
   tttctggagg gggacaacac acctcctcgc cactttccca gttacttaaa taaacacttt 240
   ccccagc 247
<210> 62
   <211> 767
   <212> DNA
   <213> Drosophila melanogaster
<220>
   <221> misc_feature
   <222> (1)...(767)
   <223> n = A,T,C or G
<400> 62
   ctaattgcgc tccatccatt tgttcctgtc cggtgattcc cacatcttta atggtggagt 60
   tatagaaatt attttgaata atcaaatcat ctccaattat cttcactatt tcactcaaag 120
   acatggtttt tagcgtgctg gtcgtgttgc ttccaattgc gctgacggct ttcgaccatg 180
   atccgaattc acnaagggcg aattctgcag atatccatca cactggcggc cgctcganca 240
   tgcatctaaa agggccccat tcgccctata ntgagtccta ttacaattca ctggccgtcg 300
   ttttacaacg tccttgaact gggaaaaccc tggccgttac cccaacttna tcgcctttgc 360
   agcacatccc ccctttcccg ccagctnggn gttaatacca anaaggcccc ctawtawtga 420
   cactatagaa tactcaagct atgcatcaag ctwrratacc gagcawcgga tccamataag 480
   ataancagag accagcacaa gtwgtagcat rggabayata tacagcccat atacggagam 540
   ayatatcagg atatwtwtat atatatatat ataaacagaa acatacatat wtatacagta 600
   tatawgcama aaaaaataca ttatataaaa aaatatatac ragtatatam acacacacva 660
   gtatatatat atacgtacga rcacgtacgc atwarcacac acacrvcacg gacacacaat 720
   wtacrcgacg cacgcacatt tahacacaat tahtatacac mtaccaa               767
<210> 63
   <211> 353
   <212> DNA
   <213> Drosophila melanogaster
<220>
   <221> misc_feature
   <222> (1)...(353)
   <223> n = A,T,C or G
<400> 63
   tawtgacact atagaatact caagctatgc atcaagctwr rataccgagc awcggatcca 60
   mataagataa ncagagacca gcacaagtwg tagcatrgga bayatataca gcccatatac 120
   ggagamayat atcaggatat wtwtatatat atatatataa acagaaacat acatatwtat 180
   acagtatata wgcamaaaaa aatacattat ataaaaaaat atatacragt atatamacac 240
   acacvagtat atatatatac gtacgarcac gtacgcatwa rcacacacac rvcacggaca 300
   cacaatwtac rcgacgcacg cacatttaha cacaattaht atacacmtac caa        353
<210> 64
   <211> 609
   <212> DNA
   <213> Drosophila melanogaster
<400> 64
   aatttttagc aatttcttat ttggtttttc ggtactttct ctagctgctt ttacttgatc 60
   gcacatatat atatatatat atattctata catatacata ttcatatgaa tatatctttt 120
   atcatcttta agaggagatt ttcagtgtct gtgtgggtgt gtgtgtttgt gtatgcttgt 180
   atgtgtccgg ttgtcctata gccatttgaa ccactaagaa tttgtagccg gggaagttgc 240
   tatcaaatag agttgctcaa caacggctct ggctcgggtt gaaggaattt ttggaggtcg 300
   aggggagcca acgacacaac gcaagctgcc ccaaaaaaac gggctaagaa atcaggttgg 360
   gctaatgaaa tacaaagctt gcaagggcaa gaagaagaag aagactgagc actttctttt 420
   cggctgcatc gcttacaacc agttcatagt gcgcctctct ccgcgcttct catcgatggt 480
   aggtaagccc ttgtttcaaa tgatgtgaat gggtctaatt aggagtttgt ctgtctgtgt 540
   ctgtattgtg tctgcacaag ccagagaaag agaggctggg gagaatggga gaaagtgggt 600
   gatgggagg                                                         609
<210> 65
   <211> 554
   <212> DNA
   <213> Drosophila melanogaster
<400> 65
   taaacaaaag aaaaacaaaa ttccttttga aaatgcaaca ttaacaaata gaaagaaaca 60
   aaacagaaca aacacgtaaa gaaagaggcc actacaaaac tgaaaagaaa atgtgaaaaa 120
   tacaaaattt cgtttagcca ttaagattgt taagaatcag agtgttagat gtagatgagc 180
   aagtgaattt tgtagggctt tgctaccagt tttacctgct taatgaataa gggtaaaaca 240
   ttcatatgat tggattggaa gaatatatcg ggaatgctaa aaattattgg agtataagtt 300
   aaatacaact gcgatttatc tgtttaagtt ttaaatgcta tattaacgat gtataacttt 360
   ggttcaatgt tttagtcata ggtttttaca tttaactcaa tgtggggaga gagcttttaa 420
   atagatcata cgaacctaca tattacattt atcggttatt ataattgttt tggccctctc 480
   atccaatata tacatatttt atggtcctag gttgtctttt ttaagttttc cattttgtta 540
   aagaaagttc gatt                                                   554
<210> 66
   <211> 647
   <212> DNA
   <213> Drosophila melanogaster
<220>
   <221> mise_feature
   <222> (1)...(647)
   <223> n = A,T,C or G
<400> 66
   tggactgata tgcaaaaaag catttcacca cggcacctgc gcatataatg gtggatagcc 60
   tgtggaacgt ccttatctta tcgtgtaagg tggacacgac acgaacacta atcagagaat 120
   agagcagttc taactcacaa tattgataaa caaagtaagg gccagccgag agatacacgc 180
   gcatttattg gcagcaaaca gaagccaaaa ctacggacat gtccgaatcg ggaatcaaaa 240
   agttgagcca ggagcggact cgcgaatggt tggctagtca ggaggacgag gaactggagt 300
   ccattgcaga gtcctcggtt gtggacagct tggactacga ttataccgag gaagaggagg 360
   atgccgacca aaataccagt gaagaaatca gcactatgac actaggcact caaatcgcta 420
   ccaaaaagca ttcgatcatc agcgacacca taagggacct tatgaactcg atcaacagca 480
   ttcagacttt gggcaacgtt aatataagca actccacgaa cgtccatatc ggcaatgtta 540
   ccaatattaa tggaaatata caaatcatag ccgatggcct tactcaaaac cgaagagatc 600
   ggcggcatgt ttcaccaccg agagataacg cttccaaaac tccgacn               647
<210> 67
   <211> 600
   <212> DNA
   <213> Drosophila melanogaster
<400> 67
   gttttcaaac gctcagcgga gaaaatgtaa cggacgaacg cggctggcaa aactcacaga 60
   cggtacaaga gaaccagaat aaaaaaggac tccacaagaa acggcaactc gacaaaatct 120
   atacaaaagt gtctggctcg actgtgtgtg tgcttctgag tgaatgcttg tgtatgtgtg 180
   tataaattag tttggttgtg tgagttgtta gagtcaaaga actaaaataa gactttcaga 240
   tctagcaaat atgtcccata gttccccgag acgcgtatcc actgctgtag ccacttaaca 300
   aacaatgccc aaagttaagg cgcacggaat ctctaataat cgaaaccaat aaaatgagcc 360
   ccgttgcctg cagcaccaac actaacatcg gtcacatcga gcaggttgca ggcaatcaaa 420
   ggacaaatat agctgggata agatcaatcc aaattggaac aaccacaatc acaacgatat 480
   tgaaccagcg atgagatgga gcgtccgttg ggatgacgaa ctcagaaact cagtaaggga 540
   gctgcaactg atactgaaac tgaaacagaa accacagcgg cactcggaat ttagaggcga 600
<210> 68
   <211> 598
   <212> DNA
   <213> Drosophila melanogaster
<400> 68
   ccgccgagcg cctgctgcag catcccttcg tccagtgcga gatgtccttg cgggtggcca 60
   aggagctgct gcagaagtac cagagtccca acccgcagtt ctactactat ctcgatggcg 120
   atgaggagtc tgtggcagga gtgccacaac gcattgccag caaaatgacg tcacgcacca 180
   atggcgtgcc agcgcaaaat cacacactaa aaacaggcat gacgacgaac tccacgtgga 240
   atgagcgatc ttctagtccc gaaacgttac ccagtgacat gagcctctta caatatattg 300
   atgaggagct gaagctaaga gcgaccttgc cactgaacaa cgacaccaaa gatccactcg 360
   gcgccgagtg cagctgctcc tcccacaatg gaggagccgc cggaggagga ggaggaggag 420
   gagttggagt aggagcaggc ggagcagccg cgagcggcag cagcagcagc agcggaggcg 480
   caacagtcgg caccactcat catcagcacc aacagcacca ccaggatcac caccatccga 540
   atcatctgca tcagcatcag gcccatcaat tgccgcaaca gcagcagcag cagtcaca   598
<210> 69
   <211> 420
   <212> DNA
   <213> Drosophila melanogaster
<400> 69
   cagctggacg cgccgagcat catggacgcc ttcctggaca ccgagcgaca gagaatcgag 60
   cgcgagcagc aattggcggc ggcggagcag gatgccgatc gccgggcgga gcagaaccgg 120
   ctggaactgt accagatttt ggccgcctcc gagcctgatc cgcaacctta ccagaggaag 180
   ccggcggcac agccgaatgc tatggaccaa ctggaggcca ttgtggagca gcagcagcag 240
   cgcgagctga aggagcagca ggagcaggcc aaggcaccgg tctacgtgcc tcccgaggag 300
   gtgaacgagt cgagcgagct gtacttcccg gacaactttg ctcctttcaa gagagcaagg 360
   ggtcgctcca ggggaggatt ggccgaggag gtggaggact aacagccgaa gcgctccttc 420
<210> 70
   <211> 547
   <212> DNA
   <213> Drosophila melanogaster
<400> 70
   aagcgtgcca gaaatggcaa cgacagttcg ggttcggact cgaattccag cagtccgcgc 60
   cagcaaggca gccctccagt gatctgtgag gatgcggctg cttgcgcagc tctctccggt 120
   tacactgtgg atcagctctc ggatctggcc agtcactgcc cagtgctgag taacaacaat 180
   gctgtgggac ctaccggagt tagtggtggt ggcgatgcgg ataccaacaa tgtgaacacc 240
   actccccgtc agtgccctct tcgcttggtg ggcggtcagg aagtgatggg ccagtgccca 300
   gtgccgcaca atcaggcaat ggttcctgcc aaatgtccag tagcgcatgc agactctggg 360
   gattccttca gcgccaagag tggaagtgga ggggaatcgg ccaccactgc tcactgtcca 420
   ctacagatgc ccgtgggaca ggacttcatg ggcgaatgtc cgtacgttaa caacgatgtg 480
   aaggtatcct ttgcccaagc tggaaagtgt ccagtgactg gcggtgtggc aggagcatca 540
   gcttcta                                                           547
<210> 71
   <211> 605
   <212> DNA
   <213> Drosophila melanogaster
<400> 71
   atgaatcctc tggacaaaat acacgctcta gatgagatcg aaaaggagat aatcctgtgc 60
   atgcaaagtg caggacaagc cttgcaggag ttgggcaagg aaaagtcttc ccagaaaaat 120
   gcggagaccc agtcgcagca gtttctcaag agtctgtcca gcgtggaatc gaagctgtcc 180
   gagcagatca actacttgac ccaggtgtcc acgggtcagc cacacgacgg ttccggctat 240
   gcatccgcca aagtgctcca aatggcttgg catcgcattc agcacgctag gtccagagtg 300
   cgtgaacttg aggaaactaa ggccaaacac tcacatgcag ctcgtcagca gttgaagcgt 360
   cacaggaaca tgccgccgcc cagcaacagc agcagcaaca acaacagcag cagcagcaac 420
   aacaacagat gcaacaggcg gcacaacagc agcaacaaca aaccggagga ggaaatgccg 480
   gcagcggaga tcatccctgg gcggagactc ctcaatgtca accaactaat cttgcgctat 540
   ctttaagggt aagggtttta aattttttag agtgcattcc gaaaaggcac attttgtcca 600
   ccaat                                                             605
<210> 72
   <211> 630
   <212> DNA
   <213> Drosophila melanogaster
<400> 72
   tagatccgac agcacagtca tgaaatcaga ccgagaagcc ggtcgtgccg attcgcgatc 60
   ctggcgggtc cattgctcgt cctcgtgcaa tcggacattg tattcctcct gattctcatt 120
   tccatcgggt cgcgaccaga tgagcttcaa tccattgcca ataagcacaa tatcgtggcc 180
   acgctcatag ttgccatatg actccactat tagactgtac gacaggcggc caccgtacga 240
   gaatagctgg ttgcccagca cacttcccct aagactccag tacttgggca gataggaggt 300
   gtgcgtgtag gtatacatat tcctagatat gtcgggaatt aagttctcgg tgtcctggac 360
   agctccgctt tcgtctgtaa ttaatggtgc gttaagaata aagtccaccg gtattagctg 420
   gcggtacaga gctgccgaac gacactggct ggccaatcca gagcagtagc actctttgca 480
   gccatcctga ttttgagcag acagtccata ggttccaggg cggcattggt cgcattgatc 540
   accaatcacg tttctcttgc acaggcattc gttgccgcgg caatcataga tgccctctat 600
   ttggcaatag gccgtgcatt ccaaagtttg                                  630
<210> 73
   <211> 638
   <212> DNA
   <213> Drosophila melanogaster
<400> 73
   taaagacccg cattgctgaa gtgatgcgcg atgatattgg ttatggaaag aatcggactg 60
   tcgaggtgcg aacagaggat gaagtaaccg ccgatatggt ggcacattcg catgccgccg 120
   tccatgctgc acatgtggcg cacgcagccc atgtcgccca tgccgctgct atggagttgc 180
   agcacagaag caaggaacca ccgccgccag agatcagtgt gtcacgtaag acgcccaacc 240
   aatacgaggt ggtagacgcc agtggtcggc gctcagctgg cagtggttcc gtttcggttt 300
   ccgtttcggg cgccaatagc caccattcgc cgtatcatcc accggcggcg gcctatgccc 360
   ccagcaccta tgccttcccg tacagcgccc tgaatgtgcc cggtgccgcc ggtggattgc 420
   caccgcacca gccgttgcag ctagcccacc aggcggtggc accacctggt gcctttgcca 480
   aggccaaggc agcgcatgcc ctgagtgaac tgggtgcagt cggtggtggg gtgtcattgg 540
   tggtgggcgg cggctctgga ggaattgcag gcggaccagg tggtgtctca gtcggtgtcg 600
   gtgtaccggg cggcggcgga ccaggaagcg gtggctgc 638
<210> 74
   <211> 629
   <212> DNA
   <213> Drosophila melanogaster
<220>
   <221> mist_feature
   <222> (1)...(629)
   <223> n = A,T,C or G
<400> 74
   atcaatgctc tatgctacta tatcttgcct tttactataa ctcgtcgcag ctccgacgaa 60
   caggaatgtc aggcctgcca atcagtgtcc tcggtcatca tgatggtgct ccagtactcc 120
   aacaatccag cgcatcattg ccagctcctg gagtgcctga tgactcttaa gcacaatgtc 180
   gtcaaggaca tcctctgcgt tgtggcatac ggaaccgctg tttcccgcac ctcggctgcc 240
   aagctgctct tctactactg gccagccttt aacgccaatc tgttcgatcg caaagtccta 300
   ctctccaaac taaccaatga cctagtgccc ttcacctgcc aacgggagca ctgtccgaac 360
   tccgggaatg cggaggcagc aaaggtgtgc tacgaccaca gcattagcat cgcatacgcg 420
   cccgattgtc caccgcccct ttacctgtgc atcgagtgcg ccaacgagat tcatcgggag 480
   cacggaagcc tggagttcgg cgacattctg catcccatgc agcaggtatc gatggtgtgc 540
   gaaaacaaga actgtcgctc caacgagaag tccgncttct tcatctgctt ttccacggag 600
   tgtgccagct tcaatggcca ccatccgat 629
<210> 75
   <211> 588
   <212> DNA
   <213> Drosophila melanogaster
<400> 75
   agagagacaa cgacacgaca cgacataagt gggggtgggg gatagcgaac gagcccatcc 60
   agcaacaaac ttcgcgaacg gcggcgacga cgcgcaaagc tcgactgaat tccaattcga 120
   attcgggcac gctcagaagt accgttggag tgcagcgacg ccggcgatgg gtaaacaaaa 180
   ataggaatgg ctaaagacgt gcggagccct tgcgctcctc cagcccccgt ttccgaccct 240
   ccccccgctg ccgctcccgc tccaaagaca cactcctaca aagagctcaa ctgtttacac 300
   acacacacac acacacaggc acggacacgg aagtgtgtat gggtgagacg taattaaagc 360
   ttgaaaccga gtttacaaca acaacgagcc cgccagtcgc cacccaccac cccacgccgc 420
   acaccccctg cgaagagccg aagtcgaagc aacagctaga agaagaggct taagagagag 480
   agagagagag agagagagag agagcgggaa agagggaaaa ttggatactt cgcgcagaga 540
   gaaaccccca acaacgagcg cagtttataa ataaaccttg ttcttttc 588
<210> 76
   <211> 579
   <212> DNA
   <213> Drosophila melanogaster
<400> 76
   tttggctaac catttctttt tatataaaag taagtaaact aagaactaat cctaggcctg 60
   caggaagtct ccgagattgc cacatatttt gtcgatttcc gcacatcccg attgctccag 120
   cgctgaaatg gcattggcga gggccacggt ttctttcagg gaatgggcct tcaaccatat 180
   cctgccgttg actcccacag cgatctcgta gggcagttcc cgggtaagag cggcgagaac 240
   agggcagttt tcccgcagca gcatccttcc cagattcagg ctgcacttga agaagaatcc 300
   atcgatagac atgcaatcca cagctacgtg tggatcccga ttactctaac cttgtgcgaa 360
   ggtcaatttt ccccaaaaaa tataggaaac gtaccaggga aaacaacaaa aaagggaaag 420
   cgcaccccca cactgaaaac cggcgagcac ctggaaacgc atacatataa aaggagagta 480
   aatatacaaa ttggtagcac tttcgccgcc gtcttttaca cattcaagcc atgtcttgga 540
   ccgcttcagt tttcttgagg acttacacca ctagcatga 579
<210> 77
   <211> 656
   <212> DNA
   <213> Drosophila melanogaster
<400> 77
   attatgttca gaaccttccg cccggagtca tcgaagtggg tggtctccac atcaagaacc 60
   agaccagccc tttgcccacg tatatacaag aattcacgga gaagttcttc gacggcattg 120
   tgtacatcaa tatgccctat attgagtata tgaatgacca gggattgaag gctatgtata 180
   cgatgattca cggaaatccc aatgttgcct tcatttggaa tgtggagcaa ctagagcagt 240
   tgccggccaa gaaaccaaat ctgttgacgc ttcatgtgaa tcaatcacta cagcaagaca 300
   tcttggctat gcagtacgtc aaggggttcc tgaatcatgg agatagtttc agtcttcagg 360
   aggcaattca ctatggagtg cccgtcgtcg tgcttcccct taaactagag gaatttaata 420
   atgcccaacg tgtaatggaa cgcaacttgg gtgtgatgct tcaggtcaag gaatttaacc 480
   aaagctccct gtcggatgcc cttacgcgaa tcctggatga ggagcgtttc ataagtgctc 540
   tccaccaggc ccagttgaag ttccggaccc gtccgcaatc cgccctggaa ttggctgtat 600
   ggcatgcgga acaacttatc gccgaaccac gactatttaa acattttgca caaact 656
<210> 78
   <211> 549
   <212> DNA
   <213> Drosophila melanogaster
<220>
   <221> misc_feature
   <222> (1)...(549)
   <223> n = A,T,C or G
<400> 78
   caacttcgat cggggcatat aaaaccagtg cttccaatcc gaaggcaaag cataaaagat 60
   cagaacatca gtagccgaag attggctgag tagcacggac agcgggcaag tcctttgaaa 120
   cgttggtagt ttgcaaccgg gtttgccaac ttcctttgga gttcagtggt gctcaactat 180
   cgacacaact atcctcggct ttcgcaaaac tcagtaaacc gacacattga cattcgaaaa 240
   ttgggattga aaactcaaga tgccgactac accacaggat cttgnccctt gcccactctc 300
   ttgctcaaag acctccgacc gatagcagtg aggccaagga gcaggaggcc ggcgaatcgg 360
   acaacctgcc caacctgtgc actttgtcgc tggacgaact gaaacagctg gacagggatc 420
   ccgagttctt cgaggacttc atcgaggaga tgtccgtggt gcagtacctg aacgaggagc 480
   tcgattcaat gatggaccag gtggagatta tatcaagaga gaacgagtgc aagggcattc 540
   atctggtag 549
<210> 79
   <211> 486
   <212> DNA
   <213> Drosophila melanogaster
<400> 79
   ccgtcggaca gctccgactc ggacatctca ctgggcaccc actcgccggt gccgagcagc 60
   ctgcagctgc agcatagtcc gggcagcacc tccaacggcg ccaacgaccg cgaggagagc 120
   ttgagcgtgg acgacgacaa gccgcgggat ctgagcggat cgctgccact gcccctctcg 180
   ctgcccctgc cgctggcctc gcccacccac acgccgcccc aactgccgcc gggctacggg 240
   ggcggggcgg gcgcaggacc cggaggacct ctgaccggtc cgggctgtct gccacccttc 300
   aagctggacg cggtcaccag tctgttcagc gccggctgtt acctgcagag cttcagcaac 360
   ctgaaggaga tgtcgcagca gtttcccatc cagccgattg tcctgcgtcc gcatacgcag 420
   ctgccgcagt cgctggcact gaacggcgca tccggcggac cgacactgca tcacccggcc 480
   tacgcg                                                            486
<210> 80
   <211> 590
   <212> DNA
   <213> Drosophila melanogaster
<400> 80
   aaaggggaaa ggcaggctta taagatgagt aaaacgagct tagcgacgca gaccaatggc 60
   acggcggaaa atggaaactg ttttgatatc ctcaaagtgt ctaatttgtt cgaaaccagc 120
   cttctggacg aggatgacgt acaattagac gcatacttgg cagcctacga ggaaataatg 180
   aagttcttcc agctcatggg cagtgtcttc agttttgtca gcagcgatgt gcgcagtaaa 240
   atagatatct tatacgccct gagagccaag gacgcggagg agcaggaaca ctttaatacc 300
   ttcagaacca tgctggatta cgagaaggag gcccagttgc ttactcagaa gggttacgtg 360
   tctggcagtc gaacgctgct acgtcttcat cgcggtcttg actttgtcta cgagtttctc 420
   aatcggatcc aggcgatacc cgacgaccaa aagactgtgg acgtgtgcaa ggaggcctac 480
   gatgacaccc tgggcaagca tcactcattc ctcatccgca aaggagcgcg cctggctatg 540
   tacgcgatgc ccactagggg agatcttctc aagaaagtgt gctccgatgt           590

## Description

### BACKGROUND OF THE INVENTION

### FIELD OF THE INVENTION

The invention relates generally to the fields of genetics and molecular biology and more specifically to a method of using *Drosophila* to map the network of genetic interactions relating to Alzheimer's Disease.

### BACKGROUND INFORMATION

Alzheimer's disease, the most common neurodegenerative disease in the world, is a progressive disease that attacks the brain and results in impaired memory, thinking and behavior. Approximately 4 million Americans have Alzheimer's disease and, unless a cure or prevention is found, it is estimated that by the middle of this century 14 million Americans will suffer from the disease. The average lifetime cost per patient is approximately $174,000, with Americans spending at least $100 billion a year on Alzheimer's disease. The only two drugs currently approved by the FDA for treatment of Alzheimer's disease work to temporarily relieve some symptoms but do not extend life expectancy, which is an average of eight years from the onset of symptoms. Thus, there is at present no medical treatment available to cure or stop the progression of Alzheimer's disease.

The human amyloid protein precursor (APP) is centrally implicated in Alzheimer's disease, in part as the source of the amyloid-rich plaques characteristic of this disease. In individuals suffering from Alzheimer's disease, beta-amyloid peptides accumulate and aggregate to form plaques in the brain parenchyma and around blood vessels. In addition, certain APP alleles are known to predispose individuals to Alzheimer's, further implicating the amyloid protein precursor in this disease. However, the normal function of APP is currently unknown; moreover, very little is known about the network of genes that interact with APP or other genes involved in Alzheimer's disease. Understanding the network interactions of genes that directly or indirectly interact with APP is a critical step towards identifying new drug targets, assessing their likelihood of success, and augmenting the efficacy of existing drugs for the treatment of Alzheimer's disease.

Malleable genetic organisms such as *Drosophila melanogaster* provide convenient experimental systems to study functional gene interactions. That *Drosophila* can be used to elucidate a human genetic network is supported by the fundamental conservation of cellular mechanisms between humans and *Drosophila.* Conserved cellular mechanisms include homologous signal transduction pathways, such as the receptor tyrosine kinase activation of Ras and mitogen-activated protein (MAP) kinase (Engstrom et al., Curr. Topics Dev. Biol. 35:229-261 (1997)) and the cAMP activation of protein kinase A (PKA) and cAMP-responsive element-binding protein (CREB) (Dubnau and Tully, Ann. Rev. Neurosci. 21:407-444 (1998)). The fundamental conservation of cellular mechanisms between humans and *Drosophila* also is supported by mutants such as the fly very long chain fatty acids (VLCFA) acyl CoA synthetase mutant, which shares molecular and phenotypic similarity to the neurodegeneration produced in human adrenoleukodystrophy (Min and Benzer, Science 284:1985-1988 (1999)). Furthermore, the use of *Drosophila* for elucidating the gene network involved in human Alzheimer's disease is supported by the identification of a *Drosophila* homolog of human APP. The *Drosophila* homolog, β *amyloid protein precursor-like* gene *(Appl),* is homologous in sequence and function to human APP (Rosen et al., Proc. Natl. Acad. Sci. 86:2478-2482 (1989); Luo et al., Neuron 9: 595-605 (1992)). Price et al., Science, vol. 282, p. 1079-1083 (1998) discloses the mating of mice having mutation in the Appl and the presenilin gene, respectively. In view of the above, a lower genetic system such as *Drosophila,* which carries a gene homologous to a human disease gene, can provide a valuable model system for the study of the functional networks underlying human diseases such as Alzheimer's.

Thus, there is a need for identification of new genes involved in Alzheimer's disease and for a means of mapping the network interactions involved in this disease. The ability to elucidate the genetic network involved in Alzheimer's disease would provide a means of identifying new drug targets and of increasing our understanding of the genetic interactions underlying undesirable side effects. The present invention satisfies this need, relying on the powerful genetics of an experimental genetic system such as *Drosophila* to provide a method for determining the functional network of genetic interactions underlying Alzheimer's disease. Related advantages are provided as well.

### SUMMARY OF THE INVENTION

The present invention provides a method as defined in claim 1 of mapping a network of functional gene interactions relating to Alzheimer's disease. In one embodiment, a method of the invention further includes the step of identifying the gene that is a member of an Alzheimer's disease genetic network. In another embodiment, the steps of the method are iteratively repeated in order to identify a network of functional gene interactions relating to Alzheimer's disease.

The methods of the invention can be conveniently practiced by assaying for an altered phenotype such as altered viability, morphology or behavior in test progeny produced by mating two parent strains of, for example, *Drosophila melanogaster.* The mutation can be, for example, an amorph, hypomorph, antimorph, hypermorph or neomorph, and the series of genetic variations contains at least twenty or at least one hundred genetic variations. In one embodiment, one or all of the genetic variations map to the X-chromosome. In another embodiment, one or all of the genetic variations map to the autosomes or to one particular autosome.

The present disclosure also describes a method of identifying a therapeutic agent for treating Alzheimer's disease. The method includes the steps of (a) producing test progeny by performing matings between a first parent strain carrying a mutation in an Alzheimer's disease gene and a second parent strain containing a genetic variation where, in the absence of an agent, the parent strains produce test progeny having an altered phenotype relative to at least one sibling control; (b) administering an agent to the first or second parent strain or the test progeny; and (c) assaying the test progeny for the altered phenotype, where a modification of the altered phenotype producing a phenotype with more similarity to a wild type phenotype than the altered phenotype has to the wild type phenotype indicates that the agent is a therapeutic agent. An Alzheimer's disease gene useful for identifying a therapeutic agent in a method of the invention can be, for example, *Appl* or *Psn.* An altered phenotype to be assayed can be, for example, increased or decreased viability in a species such as *Drosophila melanogaster.*

The disclosure additionally describes an isolated nucleic acid molecule which is differentially expressed in *Appl^{d}* versus *Appl⁺ Drosophila melanogaster and contains* a nucleic acid sequence having substantially the sequence of one of SEQ ID NOS: 1 to 63. Such a differentially expressed nucleic acid molecule can have, for example, the sequence of one of SEQ ID NOS: 1 to 63. Also described herein is an isolated nucleotide sequence that contains at least 10 contiguous nucleotides of the nucleic acid sequence of one of SEQ ID NOS: 1 to 63.

Further described herein is an isolated nucleic acid molecule which is differentially expressed in *Appl^{d}* versus *Appl⁺ Drosophila melanogaster* and contains a nucleic acid sequence having substantially the sequence of one of SEQ ID NOS: 64 to 80. Such an isolated nucleic acid molecule can have, for example, the sequence of one of SEQ ID NOS: 64 to 80. The disclosure additionally describes an isolated nucleotide sequence containing at least 10 contiguous nucleotides of the nucleic acid sequence of one of SEQ ID NOS: 64 to 80.

### BRIEF DESCRIPTION OF THE DRAWINGS

Figure 1 shows the network of genes that interact with *Appl*, the *Drosophila* homolog of human APP. In the schematic, "+" denotes increased viability and, "-" denotes decreased viability in comparison to the indicated mutation alone or Appl⁻ alone.
Figure 2 shows MALDI-TOF analysis of tryptic digests of proteins A1.1, A1.2, A1.3, A1.5, A1.7 and A1.9, which are differentially expressed in Appl^{d} versus *Appl⁺ Drosophila.*
Figure 3 shows MALDI-TOF analysis of tryptic digests of proteins A1.12, A1.13, A1.14, A1.15, A1.16 and A1.17, which are differentially expressed in *Appl^{d}* versus *Appl⁺ Drosophila.*
Figure 4 shows MALDI-TOF analysis of tryptic digests of proteins A1.18, A1.21, A1.22, A1.23, A1.24, and A1.26, which are differentially expressed in *Appl^{d}* versus *Appl⁺ Drosophila.*
Figure 5 shows MALDI-TOF analysis of tryptic digests of proteins A1.27, A1.28, W1.1, A1.2, W1.3 and W1.4, which are differentially expressed in *Appl^{d}* versus *Appl⁺ Drosophila.*
Figure 6 shows MALDI-TOF analysis of tryptic digests of proteins W1.5, W1.6, W1.7, W1.9, W1.10 and W1.11, which are differentially expressed in *Appl^{d}* versus *Appl⁺ Drosophila.*
Figure 7 shows MALDI-TOF analysis of tryptic digests of proteins W1.12, W1.14, W1.15, W1.17, W1.20, W1.21 and W1.22, which are differentially expressed in *Appl^{d}* versus *Appl⁺ Drosophila.*
Figure 8 shows MALDI-TOF analysis of tryptic digests of proteins W1.23 and W1.24, which are differentially expressed in *Appl^{d}* versus *Appl⁺ Drosophila.*

### DETAILED DESCRIPTION OF THE INVENTION

The outpouring of new data on human genetics and genome content accentuates the need for a new approach to the problem of understanding gene networks, particularly as these networks relate to diseases such as Alzheimer's disease. Whereas sequence data can provide clues that indicate the physiological role of encoded proteins, malleable experimental systems such as the fruit fly, *Drosophila melanogaster,* provide a means of systematically analyzing the functional interrelationships of groups of gene products.

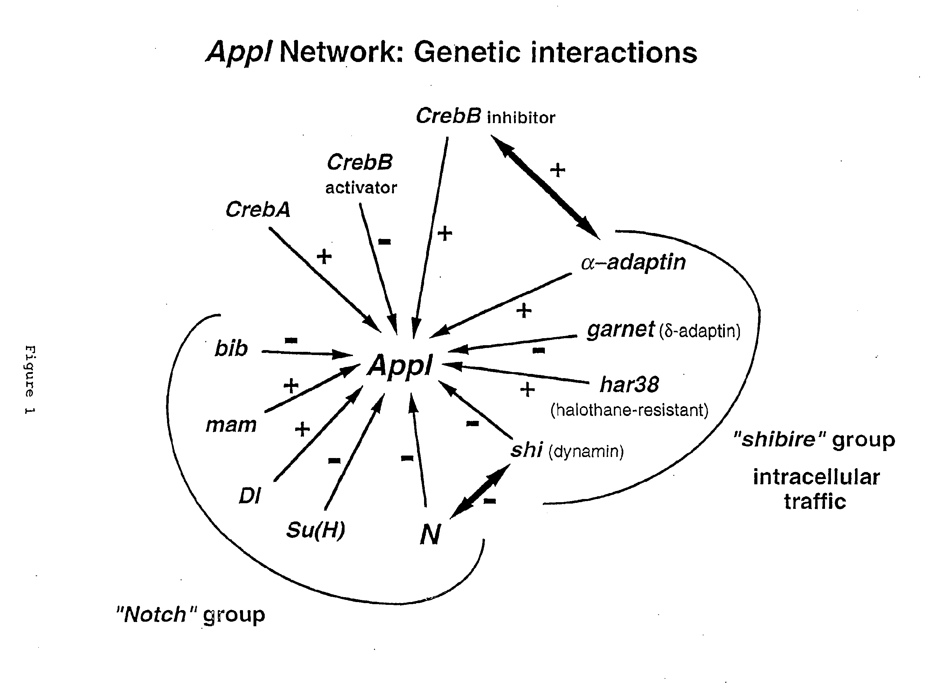

The present invention is directed to a rapid new means of mapping interactions in a gene network. This method, which relies on the powerful genetics of *Drosophila* and the extensive homology between human and *Drosophila* genes, is useful for detecting both direct and indirect gene interactions. As disclosed herein, flies bearing a chromosome that lacks the *Drosophila* homolog of human amyloid precursor protein, *Appl (w Appl^{d}),* were crossed with a series of flies bearing 34 individual deficiencies of the X, chromosome, *"Df (1) s. "* Subsequently, the number and genotype of adults emerging from each cross were scored, and the viability of flies bearing both the *Appl^{d}* mutation and the deficiency calculated relative to sibling controls. As shown in Table 1, in 34 combinations of X chromosomal deficiencies with the *Appl^{d}* mutant, most test progeny had approximately normal viability, five had severely reduced viability (less than 35%), seven had moderately reduced viability, and three had increased viability relative to the controls. Thus, as disclosed herein, the chromosomal segments including 3C2;3E4, 17A1;18A2, 12D2;13A5 and 18E-20 were identified as containing one or more genes that is a member of Alzheimer's disease genetic network. As further disclosed herein, several mutant alleles of genes lying within these chromosomal segments increased or decreased viability in combination with *Appl^{d},* thus identifying these genes as members of the genetic network involved in Alzheimer's disease (see Example I). Three prominent gene groupings were identified on the basis of altered viability when combined with *Appl^{d}:* genes related to the dynamin-encoding *shibire;* genes relating to *Notch,* the *Drosophila* homolog of a human gene implicated in a form of hereditary degenerative dementia; and Creb-related genes, which encode transcription factors implicated in neuronal plasticity and long-term memory formation (see Figure 1).

**Table 1**

| **Viability of X Deficiencies with *Appl*^{d}** | | | |
|---|---|---|---|
| *Df(1)* | Breakpoints | N | % viability¹ (1 dose *Appl*⁺) |
| *AD11* | 1B;2A | 241 | 92.8 |
| *A94* | 1E3-4;2B9-10 | 109 | 94.6 |
| *64c18* | 2E1-2;3C2 | 144 | 87.0 |
| *JC19* | 2F6;3C5 | 232 | 26.1** |
| *N8* | 3C2-3;3E3-4 | 461 | 32.1** |
| *cho2* | 3E;4A | 236 | 112.6 |
| *JC70* | 4C15-16;5A1-2 | 556 | 103.6 |
| *C149* | 5A8-9;5C5-6 | 312 | 110.8 |
| *N73* | 5C2;5D5-6 | 662 | 104.9 |
| *JF5* | 5E6;5E8 | 1060 | 127.4** |
| *5D* | 5D1-2;5E | 827 | 95.9 |
| *Sx1-bt* | 6E2; 7A6 | 1454 | 81.5** |
| *Ct4b1* | 7B2-4;7C3-4 | 214 | 18.8** |
| *C128* | 7D1; 7D5-6 | 551 | 72.7** |
| *KA14* | 7F1-2;8C61 | 40 | 68.6 |
| *1z-90b24* | 8B5-6; 8D8-9 | 62 | 34.7** |
| *9a4-5* | 8C7-8;8E1-2 | 145 | 68.6* |
| *v-L15* | 9B1-2;10A1-2 | 542 | 75.9** |
| *HA85* | 10C1-2;11A1-2 | 89 | 71.1 |
| *N105* | 10F7;11D1 | 58 | 45. 0** |
| *JA26* | 11A1;11D-E | 136 | 86.3 |
| *N12* | 11D1-2;11F1-2 | 512 | 99.2 |
| *KA9* | 12E2-3;12F5-13A | 196 | 104.1 |
| *RK2* | 12D2-E1;13A2-5 | 241 | 122.4 |
| *RK4* | 12F5-6;13A9-B1 | 408 | 91.5 |
| *Sd72b* | 13F1;14B1 | 205 | 70.8* |
| *4b18* | 14B8;14C1 | 826 | 110.7 |
| *N19* | 17A1;16A2 | 697 | 62.8** |
| *JA27* | 18A5.18D | 530 | 91.3 |
| *HF396* | 18E1-2;20 | 47 | 11.9** |
| *mal17* | 19A2-3;19E1 | 106 | 96.2 |
| *2*/*19B* | 19F3-4;19F6 | 708 | 125.4** |
| *JC4* | 20A1;20EF | 604 | 92.3 |
| *A209* | 20A;20F | 1030 | 87.9 |

| | | | |
|---|---|---|---|
| *w Appl^{d}*/*Y* males were mated to *Df(1)*/*FM7* virgin females and the female progeny counted. *"Df(1)"* is the name of the deficiency. "Breakpoints" indicates the chromosomal segment deleted. "N" is the total number of progeny counted. ¹% viability = [(# *Df(1)*/*w Appl^{d})* / (*#FM7*/*w Appl^{d}*)]x 100 * indicates significant departure (P<0.05) from 100% by χ² test ** indicates significant departure (P<0.01) from 100% *by* χ² test | | | |

While APP has been implicated in the pathology of Alzheimer's, its normal biological role has heretofore been unclear. *In vitro* studies have implicated the protein in neuron-substrate adhesion (Koo et al., Proc. Natl. Acad. Sci. 90:4748-4752 (1993) and Coulson et al., Brain Res. 770:72-80 (1997)); neurite extension (Mattson, J. Neurobiol. 25:439-450 (1994) and Perez et al., J. Neurosci. 17:9407-9414 (1997)); response to copper toxicity or oxidative stress (White et al., J. Neurosci. 19:9170-9179 (1999) and in synaptic plasticity (Roch et al., Proc. Natl. Acad. Sci., USA 91:7450-7454 (1994); Ishida et al., NeuroReport 8:2133-2137 (1997)). Studies of the *Appl^{d}* mutant in *Drosophila* have supported its involvement in synaptic plasticity and differentiation (Torroja et al., Curr. Biol. 9:489-492 (1999)). Furthermore, overexpression of *Appl⁺* in *Drosophila* in conjunction with expression of human tau, produces developmental defects including a disruption of axonal transport (Torroja et al., J. Neurosci. 19:7793-7803 (1999)).

The functional gene interactions disclosed herein indicate that the *APPL* protein in flies, and similarly the human APP protein, is involved in vesicle endo- and exo-cytosis. Such a role is supported, for example, by the disclosed interactions of *Appl* with *shibire, α-adaptin* and *garnet (δ-adaptin).* Such a role also is supported by interactions with *halothane-resistant* mutants, given that anesthesia-resistant mutants in C. *elegans* affect the vesicle fusion machinery (van Swinderen et al., Proc. Natl. Acad. Sci., USA 96:2479-2484 (1999)). In addition, a role for APP in vesicle endo- and exocytosis is supported by several genes that are differentially expressed in *Appl^{d}* versus wild type flies, for example, *exo84, Frequenin, 14-3-3*ζ, *PPA2a2, myosin-IB* and *actin57B* (see below). Furthermore, the interactions of *Appl* with *Notch* and its gene group can be related to the requirement for precise and reciprocal regulation of the quantities of *Notch* and *Delta* proteins on the membranes of neighboring cells during development (Heitzler and Simpson, Cell 64:1083-1092 (1991)), given that endocytosis can be the mechanism of their down-regulation.

Where normal APP protein is involved in the actual process of vesicle cycling, APP mutants can render the actual machinery abnormal, not merely the final disposition and clearing of this particular protein and its derivatives. In view of the viability of null mutants for *Appl* in flies and APP knockouts in mice, the APP mutants do not result in a major defect in vesicle cycling. Thus, the disclosed methods for mapping functional gene interactions can elucidate the normal biology of critical genes as well as their role in pathogenesis.

The present invention provides a method as defined in claim 1 of mapping a network of functional gene interactions relating to Alzheimer's disease.

In one embodiement, a method of the invention can further include the step of identifying the gene that is a member of an Alzheimer's disease genetic network. In another embodiment, the steps of the invention are iteratively repeated in order to identify a network of functional gene interactions relating to Alzheimer's disease.

The methods of the invention can be conveniently practiced by assaying for an altered phenotype such as altered viability, morphology or behavior in test progeny produced by mating parent strains of, for example, *Drosophilidae* such as *Drosophila melanogaster.* The mutation can be, for example, an amorph, hypomorph, antimorph, hypermorph or neomorph, and the series of genetic variations can contain, for example, at least twenty or at least one hundred genetic variations. In one embodiment, one or all of the genetic variations map to the X-chromosome. In another embodiment, one or all of the genetic variations map to the autosomes or to one particular autosome.

As used herein, the term "Alzheimer's disease gene" means a homolog of a human gene that has genetic variants associated with an increased risk of Alzheimer's disease or that encodes a gene product associated with Alzheimer's disease. While *Appl* is provided herein as Alzheimer's disease genes useful in the invention.

Alzheimer' s disease genes identified herein as interacting (directly or indirectly) with *Appl are Notch (N), Suppressor of Hairless (Su(H)), Delta (D1), mastermind (mam), big brain (bib), halothane resistant (har38) , cAMP-responsive element-binding protein A (CrebA), cAMP-responsive element-binding protein B (CrebB, activator) , cAMP-responsive element-binding protein B (CrebB, inhibitor), α-adaptin, garnet (δ-adaptin),* and *shibire* (shi)(dynamin). In addition, an Alzheimer's disease gene can be a gene that is differentially expressed at the mRNA or protein level in *Appl^{d}* flies as compared to *Appl⁺* flies as described further below (see Tables 4-6).

Generally, an Alzheimer's disease gene will encode a polypeptide having at least about 25%, 30%, 40%, 50%, 75% or greater amino acid identity with its human homolog and will share one or more functional characteristics with its human homolog. Methods for cloning homologs of human genes using routine methods such as PCR or library screening are well known in the art as described, for example, in Ausubel et al., Current Protocols in Molecular Biology, John Wiley and Sons, Baltimore, MD (1998).

The term "mutation," as used herein in reference to an Alzheimer's disease gene, means a stably inherited change in the primary nucleic acid sequence of the Alzheimer's disease gene. Preferably, the mutation is restricted to the Alzheimer's disease gene and does not affect additional genes. As used herein, the term mutation encompasses genetic lesions that result in a complete or partial loss of function, a function that is antagonistic to the activity of the wild type protein, increased function or gain of a novel function. The designation *Appl^{d}* represents a chromosome lacking the *Appl* gene and is an exemplary mutation in an Alzheimer's disease gene. The designation *Appl⁻* is used herein to refer to any null or hypomorphic mutation of *Appl* (see below).

The term "amorph," as used herein, means a mutation that completely eliminates the function of a gene product and is synonymous with "null mutation." An amorph or null mutation can be produced, for example, by partial or complete deletion of a gene, by a molecular lesion that blocks transcription or translation, or by a nonsense or missense mutation or other lesion within the coding sequence.

The term "hypomorph," as used herein, means a mutation that results in.a partial loss of function of an Alzheimer's disease gene product. A hypomorph can be, for example, a mutation that reduces the expression, stability or activity of the encoded gene product.

The term "antimorph," as used herein, means a mutation that is antagonistic to the activity of the encoded wild type gene product and is synonymous with "dominant negative mutation."

As used herein, the term "hypermorph" means a mutation that results in increased function of the encoded Alzheimer's disease gene product. A hypermorph can be, for example, a mutation that enhances the expression, stability or activity of the encoded gene product.

The term "neomorph," as used herein, means a mutation that results in a novel function of the encoded gene product and is synonymous with "gain-of-function mutation." Such a novel function can occur as a consequence, for example, of ectopic expression of the encoded gene product.

The methods of the invention as exemplified herein use the genetic system *Drosophila.* Examples of genetic systems disclosed herein include, for example, mice *(Mus musculus),* zebrafish *(Danio rerio),* nematodes *(Caenorhabditis elegans),* and yeast *(Saccharomyces cerevisiae* and *Schizosaccharomyces pombe).* Homologs of human disease genes have been identified in each of these species. For example, the murine *frizzled* gene is the homolog of human FZD9 deleted in Williams-Beuren syndrome (Wang et al., Genomics 57:235-248 (1999). In zebrafish, homologs of human genes implicated in, for example, Huntington's disease or congenital sideroblastic anemia have been isolated and characterized (Karlovich et al., Gene 217: 117-125 (1998); and Brownlie et al., Nat. Genet. 20(3):244-50 (1998)). In *C*. *elegans,* Alzheimer's disease genes include *vab-3,* a homolog of *PAX-6,* which is associated with aniridia in humans; and *sma-4,* a homolog of the pancreatic carcinoma gene *DPC4* (Ahringer, Curr. Opin. Genet. Dev. 7:410-415 (1997) and the *Presenilin* homologs *spe-4* and *sel-12* (Hutton and Hardy, Human Molecular Genetics 10:1639-1646 (1997); and Levitan and Greenwald, Nature 377:351-354 (1995))). In yeast (S. *cerevisiae* and *S*. *pombe*) and mice, homologs of the human RAD30 gene implicated in xeroderma pigmentosum have been identified (McDonald et al., Genomics 60:20-30 (1999). Methods of performing matings and analyzing progeny in mice, zebrafish, nematodes and yeast are well known in the art (see Jackson, for example, Mouse Genetics and Transgenics: A Practical Approach, Oxford University Press, Oxford, U.K. (2000); Detrich et al., The Zebrafish: Genetics and Genomics, Academic Press, San Diego (1998); Hope, C. Elegans: A Practical Approach, Oxford University Press, Oxford, U.K. (1999); and Adams et al., Methods in Yeast Genetics, 1997: A Cold Spring Harbor Laboratory Course Manual, Cold Spring Harbor Laboratory Press, Cold Spring Harbor, New York (1997)).

The methods of the invention use *Appl* in order to map a network of functional gene interactions relating to Alzheimer's disease. However, a network of functional interactions relating to another human disease is disclosed. As set forth in Table 2, *Drosophila* homologs of a variety of human disease genes are available, including genes involved in sclerosis such as tuberous sclerosis, amyotrophic lateral sclerosis; diseases of the eye such as aniridia, cataract, glaucoma, nystagmus, atypical colobomata, slitlike iris, stromal defects, optic nerve hypoplasia; and numerous other diseases such as Huntington's disease, retinitis pigmentosum, Waardenburg syndrome (Type 1), basal cell nevus syndrome, adenomatous polyposis of the colon, holoprosencephaly (Type 3), myotonic dystrophy, atrial septal defect with atrioventicular conduction defects, hyperprolinemia (Type 1), branchiooterenal dysplasia, renal-coloboma syndrome, selective tooth agenesis, X-linked hydrocephalus, Masa syndrome, spastic paraplegia 1, oroticaceduria 1, Saethre-Chotzen syndrome, Greig cephalopolysyndactyly, Pallister-Hall syndrome, postaxial polydactyly type A, parathyroid adenomatosis 1, SCID, cerebral autosomal dominant arteriopathy, multiple endocrine neoplasia (Type IIA), Hirschsprung disease, mesothelomia, WAGR syndrome, juvenile polyposis syndrome, neurofibromatosis, neurofibromatosis Type II, Cowden disease, Lhermitte-Duclos disease, Bannayan-Zonana syndrome, xeroderma pigmentosum A, xeroderma pigmentosum B, xeroderma pigmentosum D, Angelman syndrome and Vohwinkel syndrome. Table 2 further describes *Drosophila* homologs of genes involved in cancers such as alveolar rhabdomyosarcoma, sporadic basal cell carcinoma, colorectal cancer, hepatoblastoma, chronic myelogenous leukemia, T-cell leukemia, gastric adenocarcinoma, ovarian and pancreatic carcinoma, malignant melanoma, B-cell lymphoma, retinoblastoma, pre-B cell acute lymphoblastic leukemia, acute lymphoblastic leukemia, non-Hodgkin lymphoma, myeloid leukemia, Burkitt's lymphoma, T-cell lymphoblastic leukemia, bladder carcinoma, renal pelvic carcinoma, mammary carcinosarcoma, ovarian cancer, medullary thyroid carcinoma, neuroblastoma, glioblastoma, esophageal carcinoma, Wilm's tumor, lung cancer and sporadic prostate cancer. One skilled in the art understands that additional homologs of human disease genes are known in the art for *Drosophila* as well as for other species such as mice, zebrafish, nematodes and yeast, or can be identified by routine methods.

**Table 2**

| **Fly Gene** | **Human Gene** | **Disease / Reference** |
|---|---|---|
| *huntingtin* (AF 177386) | *HUNTINGTIN* (NM_002111) | Huntington's disease (Cell 72:971-983 (1993)) |
| | | |
| Appl (J04516) | *AMYLOID PROTEIN PRECURSOR* (NM_000484) | Alzheimer' s disease (Goate et al. Nature 349:704-706 (1991)) |
| | | |
| *Psn* (U77934) | *PRESENILIN-1* (NM_000021) | Alzheimer's disease, familial type 3 (Sherrington et al., Nature 375:754-760 (1995)) |
| | | |
| Sod (Z19591) | *SUPEROXIDE DISMUTASE* (NM_000454) | Amyotrophic lateral sclerosis (Rosen et al., Nature362:59-62 (1993)) |
| | | |
| *Eaat1* | AF001784 (U03505) | Amyotrophic lateral sclerosis |
| *Eaat2* | AF166000 (U03505) | Amyotrophic lateral sclerosis |
| *gigas* (AF172995) | *TSC2* (X75621) | Tuberous sclerosis (Kumar et al., Hum. Molec. Genet. 4:1471-1472 (1995)) |
| | | |
| *NinaE* (K02315) | *RHODOPSIN* (NM_000539) | Retinitis pigmentosum (Dryja et al., New Eng. J. Med. 323:1302-1307 (1990)) |
| | | |
| *forkhead* (J03177) | *FKHR* (AF032885) | Rhabdomyosarcoma, alveolar (Whang-Peng et al., Genes Chromosomes Cancer 5:299-310 (1992)) |
| | | |
| *paired* (M14548) | *PAX3* (U12263) | Waardenburg syndrome, type 1 (Milunsky et al., Am. J. Hum. Genet. 51(suppl.):A222 (1992)) |
| | | |
| *patched* (X17558) | *PTCH2* (AF087651) | Basal cell nevus syndrome (Wicking et al., Am. J. Hum. Genet. 60:21-26 (1997)) |
| | | |
| *dAPC1* (U77947) *dAPC2* (AF113913) | *APC* (NM_000038) | Adenomatous polyposis of the colon (Groden et al., Cell 66:589-600 (1991)) |
| | | |
| *hedgehog* (L02793) | *SONIC HEDGEHOG* (NM_000193) | Holoprosencephaly, type 3 (Roessler et al., Nature Genet. 14:357-360 (1996)) |
| | | |
| *Sine oculis* (L31626) | *SIX5* (X84813) | Myotonic dystrophy, ophthalmic aspects (Winchester et al., Hum. Molec. Genet. 8:481-492 (1999)) |
| | | |
| *tinman* (AF004336) | *CSX* (NM_004387) | Atrial septal defect with atrioventricular conduction defects (Schott et al., Science 281:108-111 (1998)) |
| | | |
| *sluggish-A* (L07330) | *PRODH* (NM_005974) | Hyperprolinemia, type 1 (Campbell et al., Hum. Genet. 101:69-74 (1997)) |
| | | |
| *eyeless* (X79493) | *PAX6* (NM_000280) | Aniridia, cataract, glaucoma, nystagmus, atypical colobomata, slitlike iris, stromal defects, optic nerve hypoplasia (Jordan et al., Nature Genet. 1:328-332 (1992)) |
| | | |
| *clift*/*eyes* absent (L08502) | *EYA2* (NM_005244) | Branchiootorenal dysphasia (Abdelhak et al., Nature Genet. 15:157-164 (1997)) |
| | | |
| *Pox-m* (X58917) | *PAX2* (AH006910) | Renal-coloboma syndrome (Sanyanusin et al., Nature Genet. 9:358-364 (1995)) |
| | | |
| *muscle segment homeobox* (X85331) | *MSX1* (M76731) | Tooth agenesis, selective (Vastardis et al., Nature Genet. 13:417-421 (1996)) |
| | | |
| *Fasciclin-2* (M77165) | *L1-CAM* (Z29373) | Hydrocephalus, X-linked, Masa syndrome, Spastic paraplegia 1 (Jouet et al., Nature Genet. 4:331 (1993)) |
| | | |
| *rudimentary-1* ike (L00968) | *OPRT and OMP* (NM_000373) | Oroticaciduria 1 (Suchi et al., Am. J. Hum. Genet. 60:525-539 (1997)) |
| | | |
| *smoothened* (U87613) | *SMOH* (NM_005631) | Basal cell carcinoma, sporadic (Xie et al., Nature 391:90-92 (1998)) |
| | | |
| *twist* (X12506) | *TWIST* (Y10871) | Saethre-Chotzen syndrome (Krebs et al., Hum. Mol. Genet. 6:1079-1086 (1997)) |
| | | |
| *armadillo* (X54468) | *CATENIN-β-1* (NM_001904) | Colorectal cancer, hepatoblastoma, pilomatricoma (Morin et al., Science 275:1787-1790 (1997)) |
| | | |
| *Abl* (M19690) | *ABL1* (Abelson) (NM_005157) | Chronic myelogenous leukemia (Chissoe et al., Genomics 27:67-82 (1995)) |
| | | |
| *Akt1* (X83510) | *AKT2* (M95936) | T-Cell leukemia, gastric adenocarcinoma ovarian and pancreatic carcinoma (Cheng et al., Proc. Natl. Acad. Sci. 89:9267-9271 (1992)) |
| | | |
| *aurora* (X83465) | *AIM1* (U83115) | Malignant melanoma (Trent et al., Science 247:568-571 (1990)) |
| | | |
| *cubitus interruptus* (X54360) | *GLI3* (NM_000168) | Greig cephalopolysyndactyly, Pallister-Hall syndrome, and postaxial polydactyly type A (Kang et al., Nature Genet. 15:266-268 (1997)) |
| | | |
| *cyclin D* (U41808) | *CCND1* (NM-001758) | Parathyroid adenomatosis 1, breast and sqaumous cell cancer (Rosenberg et al., Oncogene 6:449-453 (1991)) |
| | | |
| *dorsal* (M23702) | *NFKB1* (M58603) | B-cell lymphoma, inflammation (Barnes and Karin, New Eng. J. Med. 336:1066-1071 (1997)) |
| | | |
| *E2F* (U10184) | *E2F* transcription factor 1 (NM_006286) | Retinoblastoma (Pan et al., Molec. Cell 2:283-292 (1998)) |
| | | |
| *extradenticle* (U33747) | *PBX1* (M31522) | Pre-B cell acute lymphoblastic leukemia (Kamps et al., Cell 60:547-555 (1990)) |
| | | |
| *hopscotch* (L26975) | *JAK3* (NM_000215) | SCID, autosomal recessive, T-negative/B-positive type (Macchi et al., Nature 377:65-68 (1995)) |
| | | |
| *Myb* (M11281) | *MYB* (NM_005375) | acute lymphoblastic leukemia, non-Hodgkin lymphoma, myeloid leukemia, malignant melanoma, metastases (Linnenbach et al., Proc. Natl. Acad. Sci. 85:74-78 (1988)) |
| | | |
| *Myc* (K03060) | *MYC* (X00364) | Burkitt's lymphoma (Bhatia et al., Nature Genet. 5:56-61 (1993)) |
| | | |
| *Notch* (M16150/M1166 4) | *NOTCH1* (M73980) | T-cell lymphoblastic leukemia (Ellisen et al., Cell 66:649-661 (1991)) |
| | | |
| | *NOTCH3* (U97669) | Cerebral autosomal dominant arteriopathy with subcortical infarcts and leukoencephalopathy (Joutel et al., Nature 383:707-710 (1996)) |
| | | |
| *Ras64B* (K01961) | *HRAS* (JOO277) | Bladder, lung, renal pelvic carcinoma, mammary carcinosarcoma, melanoma, ovarian and colorectal cancer (Taparowsky et al., Nature 300:762-765 (1982); Nakano et al., Proc. Natl. Acad. Sci. 81:71-75 (1984)) |
| | | |
| *Ras85D* (K01960) | *KRAS* (K01912) | Bladder, lung, renal pelvic carcinoma, mammary carcinosarcoma, melanoma, ovarian and colorectal cancer (Taparowsky et al., Nature 300:762-765 (1982); Nakano et al., Proc. Natl. Acad. Sci. 81:71-75 (1984)) |
| | | |
| *Ret* (D16401) | *RET* (X12949) | Multiple endocrine neoplasia, Type Iia, Hirschsprung disease, medullary thyroid carcinoma (Mulligan et al., Nature 363:458-460 (1993)) |
| | | |
| *Src42A* (D42125) *Src64B* (K01043) | *SRC* (NM_005417) | Colon cancer (Irby et al., Nature Genet. 21:187-190 (1999)) |
| | | |
| *trithorax* (M31617) | *ALL1* (NM_005935) | Myeloid/lymphoid leukemia (Sorensen et al., J. Clin. Invest. 93:429-437 (1994)) |
| | | |
| *Egfr* (K03054) | *ERBB2* (NM_004448) | Neuroblastoma, glioblastoma, mammary carcinoma (Di Fiore et al., Science 237:178-182 (1987)) |
| | | |
| *frazzled* (U71001) | *DCC* (NM_005215) | Colorectal, esophageal carcinoma (Cho et al., Genomics 19:525-531 (1994)) |
| | | |
| *klumpfuss* (Y11066) | *WT1* (X51630) | Wilms tumor, mesothelioma, WAGR syndrome (Pelletier et al., Cell 67:437-447 (1991)) |
| | | |
| *Medea* (AF019753) | *MADH4* (NM_005359) | Pancreatic carcinoma, juvenile polyposis syndrome (Schutte et al., Cancer Res. 56:2527-2530 (1996)) |
| | | |
| *Nfl* (L26500) | *NF1* (NM_000267) | Neurofibromatosis (Upadhyaya et al., Hum. Mutat. 4:83-101 (1994)) |
| | | |
| *Merlin* (U49724) | *NF2* (NM_000268) | Neurofibromatiosis, Type II (Rouleau et al., Nature 363:515-521 (1993)) |
| | | |
| *PP2A-29B* (M86442) | *PPP2R1B* (AF083439) | Lung cancer (Wang et al., Science 282:284-287 (1998)) |
| | | |
| *Pten* (AF161257) | *PTEN* (NM_000314) | Cowden disease, Lhermitte-Duclos disease, Bannayan-Zonana syndrome, endometrial carcinoma, juvenile polyposis syndrome, sporadic prostate cancer (Liaw et al., Nature Genet. 16:64-67 (1997)) |
| | | |
| *Rbf* (X96975) | *RB1* (NM_000321) | Retinoblastoma, soft tissue carcinomas (Harbour et al., Science 241:353-357 (1998)) |
| | | |
| *spellchecker* (U17893) | *MSH2* (NM_000251) | Colon cancer, familial nonpolyposis, Type 1 (Leach et al., Cell 75:1215-1225 (1993)) |
| | | |
| *mus210* (Z28622) | *XPA* (NM_000380) | Xeroderma pigmentosum A (Tanaka et al., Nature 348:73-76 (1990)) |
| | | |
| *haywire* (L02965) | *ERCC3* (NM_000122) | Xeroderma pigmentosum B (Weeda et al., Cell 62:777-791 (1990)) |
| | | |
| *Xpd* (AF132140) | *ERCC2* (X52221) | Xeroderma pigmentosum D (Frederick, Hum. Molec. Genet. 3:1783-1788 (1994)) |
| | | |
| *hyperplastic discs* (L14644) | *UBE3A* (NM_000462) | Angelman syndrome (Kishino et al., Nature Genet. 15:70-73 (1997)) |
| | | |
| 1(3) *malignant blood neoplasm-1* (Z47722) | *LOR* (NM_000427) | Vohwinkel syndrome (Maestrini et al., Nature Genet. 13:70-77 (1996)) |

As used herein, the term "genetic variation" means a stably inherited change in the nucleic acid sequence of genomic DNA. A genetic variation can be a naturally occurring or man-made variation such as a chromosomal deficiency, inversion, duplication or translocation, or a substitution, insertion or deletion of one or more nucleotides. Thus, a genetic variation can be, for example, a substitution, insertion or deletion of 1 to 1000 nucleotides, 1 to 100 nucleotides, 1 to 50 nucleotides or 1 to 10 nucleotides. One skilled in the art understands that a genetic variation also can be a molecular variation such as abnormal methylation or other modification that does not produce a difference in the primary nucleic acid sequence of genomic DNA, provided that such a molecular variation is stably inherited. One skilled in the art understands that an individual heterozygous or hemizygous for a genetic variation may or may not exhibit an altered phenotype relative to its wild type siblings; however, a genetic variation useful in the methods of the invention generally affects the function or expression of an encoded gene product.

A genetic variation can be readily obtained from a variety of public sources or can be routinely prepared using, for example, standard mutagenesis procedures. For example, a series of parent *Drosophila* strains, each containing one of a series of genetic variations, can be obtained from The Bloomington *Drosophila* Stock Center at Indiana University (Bloomington, IN), a public repository containing about 7000 fly stocks including a variety of deficiency stocks and stocks carrying mutant alleles of particular genes. A complete list of stocks is available on the Internet at http://www.flystocks.bio.indiana.edu.

Mutagenesis, such as radiation, chemical or insertional mutagenesis, also can be used to routinely prepare a series of genetic variations in *Drosophila.* One skilled in the art understands that the appropriate mutagen will depend, in part, on the desired genetic variation. For example, a chemical mutagen such as ethylmethane sulfonate (EMS) is most suitable for obtaining a point mutation or a small, intragenic deletion, while radiation with X-rays or gamma-rays is most suitable for producing chromosomal rearrangements. Insertional mutagenesis with a transposable element such as a P-element also is useful for producing a series of genetic variations and facilitates rapid molecular analysis of the variations. A detailed account of various *Drosophila* mutagens, their properties and uses is available, for example, in Ashburner, Drosophila: A Laboratory Handbook, Cold Spring Harbor Press, Cold Spring Harbor, New York (1989).

EMS, an alkylating agent, is particularly useful for chemical mutagenesis in *Drosophila.* The dose of EMS required to induce a mutation depends on whether the goal is to induce a new mutation or a new allele of an existing mutation. Generally, 3 to 5 day old males are fed a solution of about 2.5 mM to 7.5 mM EMS in 1% sucrose overnight and mated to females for 4 to 5 days, followed by recovery and further test-crossing of the F1 progeny in order to reveal the presence of new mutations (Ashburner, supra, 1989). Appropriate test crosses of the F1 progeny aimed at identification of a new nutation or allele, and recovery of the mutation bearing chromosome are routine in the art as described, for example, in Greenspan, Fly Pushing: The Theory and Practice of Drosophila Genetics, Cold Spring Harbor Laboratory Press, Plainview, New York (1997).

Radiation also can be used to prepare one or more genetic variations, although generally the frequency of introducing a genetic variation by radiation mutagenesis is considerably lower than for chemical mutagenesis. X-ray machines and cobalt or cesium sources for gamma rays are particularly useful sources of radiation, which, in *Drosophila,* are typically used to irradiate mature males. Methods of using radiation to produce genetic variations in *Drosophila* are well known in the art as described, for example, in Kelley et al., Genetics 109:365-377 (1985); Sequeira et al., Genetics 123:511-524 (1989); and Sliter et al., Genetics 123:327-336 (1989);

In addition to chemical and radiation mutagenesis, transposable genetic elements allow for insertional mutagenesis in *Drosophila.* P-elements, especially "enhancer-trap" P-elements, which express β-galactosidase in a tissue-specific manner depending on the site of insertion, are particularly useful for producing one or more genetic variations in *Drosophila* (O'Kane et al., Proc. Natl. Acad. Sci. 84:9123-9127 (1987); Bellen et al., Genes Dev. 3:1288-1300 (1989); and Bier et al., Genes Dev. 3:1273-1287 (1989)). In contrast to classical chemical or radiation mutagenesis, insertional mutagenesis with enhancer-trap P-elements provides a means for identifying new genes based on expression pattern rather than mutant phenotype. New insertion lines can be routinely generated and screened based on the position-dependent tissue expression of the lacZ reporter gene. An insertion line showing an expression pattern of interest can be further analyzed to ascertain whether the insertion has disrupted a gene of interest. Methods for insertional mutagenesis utilizing enhancer trap P-elements, including mating schemes appropriate for the identification of newly induced genetic variations, are well known in the art as described, for example, in Bier et al., *supra*, 1989, and Greenspan, supra, 1997.

Chromosomal deficiencies are genetic variations that can be particularly useful in a method of the invention. As used herein, the term "chromosomal deficiency" is synonymous with "deficiency." or "deletion" and means a rearrangement in which a contiguous portion of a chromosome is excised and the regions flanking the excised portion are joined together, thus excluding the excised portion of the chromosome. A chromosomal deficiency can be a very short excision of only a few nucleotides, or can be large enough to excise, for example, several hundred genes or about 15% of one arm of *a Drosophila* chromosome.

A series of chromosomal deficiencies can be useful for genetically scanning a significant portion of the genome to map functional gene interactions involved in Alzheimer's disease. As disclosed herein, males carrying *Appl^{d}* were crossed with a series of females heterozygous for 34 individual deficiencies of the X chromosome *(Df(1)s).* This series of deficiencies together covers roughly 70% of the X chromosome and nearly 15% of the entire *Drosophila* genome, thus serving as a representative example of the *Drosophila* genome. As disclosed herein, several deficiencies, including *Df(1)N8, Df (1) N19, Df (1) JC19, Df(1)JF5, Df (1) Sxe-bt, Df(1)ct461, Df(1)c128, Df(1)LZ-90624, Df(1)9a4-5, Df(1)V-L15, Df(1)N105, Df(1)sd72b, Df(1)HF396 and Df(1)2*/*19B,* were identified as containing one or more genes that are members of an Alzheimer's disease gene network based on an altered phenotype of increased or decreased viability when combined with *Appl^{d}.* One skilled in the art understands that deficiencies which produce an altered phenotype can be further subdivided, if desired, and matings with *Appl^{d}* flies repeated in order to identify the genes within the chromosomal deficiency that are members of the Alzheimer's disease gene network.

The methods of the invention rely on screening a series of test progeny for an altered phenotype. As used herein, the term "phenotype" refers to the physical appearance or observable properties of an individual that are produced, in part, by the genotype of the individual. A variety of behavioral, morphological and other physical phenotypes are useful in the methods of the invention including *Drosophila* phenotypes such as eye color, wing shape, bristle appearance, size, phototaxis and viability. Additional phenotypes useful for practicing the invention include the size, viability, eye color, coat color, or exploratory behavior of mice; the size, viability, skin color, or optomotor response of zebrafish; the size, viability, phototaxis or chemotaxis of nematodes; and the colony color, colony size or growth requirements of yeast.

Viability represents a phenotype that is particularly useful for establishing a functional interaction between genes: as disclosed in Example I, flies carrying a combination of *Appl^{d}* and the chromosomal deficiency *Df(1)N8, Df(1)JC19, 9Df(1)ct4bl, Df (1)lz-90b24* or *Df(1)HF396* had significantly decreased viability as compared to sibling controls, while flies carrying *Appl^{d}* and the chromosomal deficiency *Df(1)JF5, Df(1)2*/*19B* or *Df(1)RK2* had significantly increased viability as compared to sibling controls. As further disclosed in Example III, *Appl^{d} Drosophila* have a defect in fast phototaxis; such a behavioral phenotype also can be useful in the methods of the invention for establishing a functional interaction as is disclosed herein for *Appl* and *Notch, Delta, α-adaptin, dCrebA* and *dCrebB.* Two mutants *(Notch* and *Delta)* showed significant phototaxis interactions with *Appl^{d}*/*+* flies, and three mutants (*α-adaptin, dCrebA* and *dCrebB*) showed significant phototaxis interactions with *Appl^{d}* flies.

The term "altered phenotype," as used herein in reference to the phenotype of test progeny as compared to a sibling control, means a significant change in the physical appearance or observable properties of the test progeny as compared to a sibling control. Thus, the term altered phenotype is used broadly to encompass both a phenotype that is dramatically changed as compared to the phenotype of a sibling control as well as a phenotype that is slightly but significantly changed as compared to a sibling control.

It is recognized that there can be natural variation in the phenotypes of test progeny. However, an altered phenotype readily can be identified by sampling a population of test progeny and determining that the normal distribution of phenotypes is changed, on average, as compared to the normal distribution of phenotypes in a population of sibling controls. Where a phenotype can be quantified, the alteration will be statistically significant and generally will be an increase or decrease of at least about 5%, 10%, 20%, 30%, 50% or 100% as compared to sibling controls. For example, as disclosed herein in Example I, viability scores less than 80% or more than 110% of sibling controls carrying one copy of *Appl^{d}* were statistically significant and, thus, are examples of an "altered phenotype."

As used herein, the term "test progeny" refers to progeny carrying both a mutation in an Alzheimer's disease gene and a genetic variation. Test progeny, which are produced by mating a parent strain carrying a mutation in an Alzheimer's disease gene with a parent strain carrying a genetic variation, may or may not have an altered phenotype.

Test progeny can be doubly heterozygous for a mutation in an Alzheimer's disease gene and for a genetic variation. The term "doubly heterozygous," as used herein in reference to test progeny, means diploid test progeny with both a single allele of the mutation in an Alzheimer's disease gene and a single allele of a genetic variation.

As used herein, the term "sibling control" means control progeny that are genetically similar to the test progeny and carry either the mutation in an Alzheimer's disease gene or the genetic variation, but not both. The term sibling control encompasses actual siblings produced in the mating giving rise to the test progeny as well as control progeny produced in a parallel mating.

Control siblings can be conveniently identified in *Drosophila* using balancer chromosomes. As used herein, the term "balancer chromosome" means a multiply inverted *Drosophila* chromosome usually carrying a dominant marker mutation. One skilled in the art understands that a useful balancer chromosome carries multiple chromosomal inversions and suppresses recombination along the full length of the chromosome. A balancer chromosome also can carry a dominant marker mutation resulting in a phenotype such as a particular eye color or wing phenotype that can be readily identified in flies carrying the balancer. In addition, a balancer chromosome may contain one or more recessive marker mutations for easy identification of progeny carrying two copies of the balancer during segregation analysis. Balancer chromosomes are available for the X, second, and third *Drosophila* chromosomes: for example, *FM7a, FM7b* and *FM7c* are convenient X chromosome balancers; *SM6* and *In (2LR) O, Cy dp^{1v1} pr cn² are* convenient balancers for the second chromosome; and *TM3, TM6, TM6B* and *TM8* are convenient balancers for the third chromosome. Balancer chromosomes can be obtained from The Bloomington *Drosophila* Stock Center at Indiana University. A complete list of available balancer chromosomes is available at http://www.flystocks.bio.indiana.edu. Methods of constructing stocks utilizing balancer chromosomes are well known in the art as described, for example, in Greenspan, *supra,* 1997.

Further disclosed is a method of identifying a therapeutic agent for treating Alzheimer's disease. The present disclosure also describes a method of identifying a therapeutic agent for treating Alzheimer's disease. The method includes the steps of (a) producing test progeny by performing matings between a first parent strain carrying a mutation in an Alzheimer's disease gene and a second parent strain containing a genetic variation where, in the absence of an agent, the parent strains produce test progeny having an altered phenotype relative to at least one sibling control; (b) administering an agent to the first or second parent strains or the test progeny; and (c) assaying the test progeny for the altered phenotype, where a modification of the altered phenotype producing a phenotype with more similarity to a wild type phenotype than the altered phenotype has to the wild type phenotype indicates that the agent is a therapeutic agent.. An Alzheimer's disease gene useful for identifying a therapeutic agent in a method of the invention can be, for example, *Appl* or *Psn*, and an altered phenotype to be assayed can be, for example, increased or decreased viability. The screening assays disclosed herein are particularly useful in that they facilitate the analysis of randomly or rationally designed agents such as drugs, peptides, peptidomimetics, and the like to identify those agents that are therapeutic agents for treatment of Alzheimer's disease

As used herein, the term "agent" means a biological or chemical compound such as a simple or complex organic molecule and is a molecule that, when administered, potentially produces a modification of an altered phenotype such as a complete or partial reversion of the phenotype. Such an agent can be, for example, a macromolecule, such as a small organic or inorganic molecule; a peptide including a variant or modified peptide or peptide mimetic; a protein or fragment thereof; an antibody or fragment thereof; a nucleic acid molecule such as a deoxyribonucleic or ribonucleic acid molecule; a carbohydrate; an oligosaccharide; a lipid, a glycolipid or lipoprotein, or any combination thereof. It is understood that an agent can be a naturally occurring or non-naturally occurring molecule such as a synthetic derivative, analog, or mimetic of a naturally occurring molecule.

If desired, an agent can be combined with, or dissolved in, a compound that facilitates uptake or delivery of the agent to a parent strain. Useful compounds include organic solvents such as dimethyl sulfoxide (DMSO) or ethanol; aqueous solvents such as water or physiologically buffered saline; and other solvents or vehicles such as glycols, glycerol, oils or organic esters. Such compounds, which can act, for example, to stabilize or to increase the absorption of the agent, include carbohydrates, such as glucose, sucrose or dextrans; antioxidants, such as ascorbic acid or glutathione; chelating agents; low molecular weight proteins; and other stabilizers or excipients. One skilled in the art would know that the choice of a carrier compound depends on the species of the parent strains and the route of administration.

In the method for identifying a therapeutic agent for treating Alzheimer's disease, test progeny are assayed for a modification of the altered phenotype such as a complete or partial reversion of the altered phenotype. As used herein, the term "therapeutic agent" means an agent that produces a modification of the altered phenotype which has more similarity to the wild type phenotype than the altered phenotype has to the wild type phenotype. Such a therapeutic agent is useful for ameliorating Alzheimer's disease in mammals such as humans. A therapeutic agent can reduce one or more symptoms of Alzheimer's disease, delay onset of one or more symptoms, or prevent or cure Alzheimer's disease.

The therapeutic agent may produce a complete or partial reversion of the altered phenotype. As used herein, the term "complete or partial reversion" means a decrease in or abolishment of an altered phenotype previously established to be associated with test progeny carrying a mutation in an Alzheimer's disease gene and a genetic variation. Thus, complete or partial reversion of an altered phenotype such as the decrease in viability of about 67.9% produced by the combination of *Appl^{d}* and *Df(1)N8,* can be, for example, an increase in viability of about 5%, 10%, 15%, 30%, 60%, or more.

A variety of modes of administration are contemplated depending, in part, on the species of the parent strains. In *Drosophila,* an agent can be administered, for example, in a 1% solution of sucrose and fed to a parent strain for an appropriate amount of time, for example, overnight. For administration to mice (*M. musculus*) or nematodes (*C. elegans*) the agent can be combined with solid food. For administration to a parent strain living in water such as zebrafish (*D. rerio*), an agent can be administered, for example, by adding it directly to the water. For administration to yeast (*5. cerevisiae* or S. *pombe),* an agent can be combined with solid support media such as agarose. Thus, one skilled in the art understands that the screening methods can be practiced using a variety of modes of administration including ingestion, injection, immersion or aerosol delivery using, for example, an atomizer or vaporization.

In the methods for identifying a therapeutic agent, an agent can be administered to one or both parent strains, to the test progeny, or to a combination thereof, before, during or after the mating. The agent may be administered to the first and second parent strains as well as to the test progeny. One skilled in the art understands that, where an agent is administered to only one parent strain, the agent is preferentially administered to females, which can carry either the mutation in the Alzheimer's disease gene or the genetic variation. Furthermore, it is understood that, where an agent is administered to test progeny, the agent can be administered at any stage of development including *in utero*; the timing of administration depends, in part, on the phenotype to be assayed.

One skilled in the art understands that an agent can be administered one time or repeatedly using a single dose or a range of doses. Appropriate concentrations of an agent to be administered in a method can be determined by those skilled in the art, and will depend on the chemical and biological properties of the agent, the mode of administration and the species of the parent strains. Exemplary concentration ranges to test in *Drosophila* are from about 1 mg/ml to 200 mg/ml of agent in, for example, sucrose solution, administered for a desired period of time.

In the methods for identifying a therapeutic agent for treating Alzheimer's disease, an agent can be administered individually, or a population of agents, which can be a small population or large diverse population, can be administered en masse. A population of agents, denoted a "library," can contain, for example, more than 10; 20; 100; 10³, 10⁴, 10⁶, 10⁸, 10¹⁰, 10¹² or 10¹⁵ distinct agents. For example, test progeny produced from the mating of a parent *Appl^{d}* strain and a strain carrying the deficiency *Df(1)RK2* are about 22.4% more viable than their control siblings (see Example I). A population of agents can be administered and the *Appl^{d}* +/+ *Df(1)RK2* test progeny assayed for a complete or partial reversion of the increased viability observed in the absence of the population of agents; an active population can be subdivided and the assay repeated in order to isolate a therapeutic agent from the population.

Methods are well known in the art for producing a variety of libraries to be used in a screening method , including libraries containing chemical or biological molecules such as simple or complex organic molecules, metal-containing compounds, peptides, proteins, peptidomimetics, glycoproteins, lipoproteins, antibodies, carbohydrates, nucleic acids, and the like. As indicated above, such libraries can contain a few or a large number of different agents, varying from about two to about 10¹⁵ agents or more. Furthermore, the chemical structure of the agents within a library can be related to each other or diverse. If desired, the agents constituting the library can be linked to a common or unique tag, which can facilitate recovery or identification of a therapeutic agent.

Libraries containing diverse populations of various types of peptide, peptoid and peptidomimetic agents can be routinely prepared by well known methods or obtained form commercial sources (see, for example, Ecker and Crooke, Biotechnology 13:351-360 (1995), and Blondelle et al., Trends Anal. Chem. 14:83-92 (1995), and the references cited therein, each of which is incorporated herein by reference; see, also, Goodman and Ro, Peptidomimetics for Drug Design, in "Burger's Medicinal Chemistry and Drug Discovery" Vol. 1 (ed. M.E. Wolff; John Wiley & Sons 1995), pages 803-861, and Gordon et al., J. Med. Chem. 37:1385-1401 (1994)). Where an agent is a peptide, protein or fragment thereof, the agent can be produced in *vitro* or can be expressed from a recombinant or synthetic nucleic acid molecule. Methods of synthetic peptide and nucleic acid chemistry are well known in the art.

A library of peptide agents also can be produced, for example, by constructing a cDNA expression library from mRNA collected from a cell, tissue, organ or organism of interest. Methods for producing such peptide libraries are well known in the art (see, for example, Ausebel et al. (Ed.), *supra,* 1989). Libraries of peptide agents also encompass those generated by phage display technology, which includes the expression of peptide molecules on the surface of phage as well as other methodologies by which a protein ligand is or can be associated with the nucleic acid molecule which encodes it. Methods for production of phage display libraries, including vectors and methods of diversifying the population of peptides which are expressed, are well known in the art (see, for example, Smith and Scott, Methods Enzymol. 217:228-257 (1993); Scott and Smith, Science 249:386-390 (1990); and Huse, WO 91/07141 and WO 91/07149).

A library of agents also can be a library of nucleic acid molecules, which can be DNA, RNA or analogs thereof. For example, a cDNA library can be constructed from mRNA collected from a cell, tissue, organ or organism of interest, or genomic DNA can be treated to produce appropriately sized fragments using restriction endonucleases or methods that randomly fragment genomic DNA. A library containing RNA molecules can be constructed, for example, by collecting RNA from cells or by synthesizing the RNA molecules chemically. Diverse libraries of nucleic acid molecules can be made using solid phase synthesis, which facilitates the production of randomized regions in the molecules. If desired, the randomization can be biased to produce a library of nucleic acid molecules containing particular percentages of one or more nucleotides at a position in the molecule (U.S. Patent No. 5,270,163, issued December 14, 1993).

Nucleic acid agents also can be nucleic acid analogs that are less susceptible to degradation by nucleases. RNA molecules containing 2'-0-methylpurine substitutions on the ribose residues and short phosphorothioate caps at the 3'- and 5'-ends, for example, exhibit enhanced resistance to nucleases (Green et al., Chem. Biol. 2:683-695 (1995)). Similarly, RNA containing 2'-amino- 2'-deoxypyrimidines or 2'-fluro-2'-deoxypyrimidines is less susceptible to nuclease activity (Pagratis et al., Nature Biotechnol. 15:68-73 (1997)). Furthermore, L-RNA, which is a stereoisomer of naturally occurring D-RNA, is resistant to nuclease activity (Nolte et al., Nature Biotechnol. 14:1116-1119 (1996); and Klobmann et al., Nature Biotechnol. 14:1112-1115 (1996)). Such RNA molecules and routine methods of producing them are well known in the art (see, for example, Eaton and Piekern, Ann. Rev. Biochem. 64:837-863 (1995)). DNA molecules containing phosphorothioate linked oligodeoxynucleotides are nuclease resistant (Reed et al., Cancer Res. 50:6565-6570 (1990)). Phosphorothioate-3' hydroxypropylamine modification of the phosphodiester bond also reduces the susceptibility of a DNA molecule to nuclease degradation (see Tam et al., Nucl. Acids Res. 22:977-986 (1994)). If desired, the diversity of a DNA library can be enhanced by replacing thymidine with 5-(1-pentynyl)-2'-deoxoridine (Latham et al., Nucl. Acids Res. 22:2817-2822 (1994)).

A library of agents also can be a library of chemical compounds, which can be generated, for example, by combinatorial chemical methods. Such a library can contain, for example, chemical oligomers such as peptoids, tertiary amines and ethylene glycols; decorated monomers such as benzodiazapines, sugar analogs, β-mercaptoketones and aminimides; and modified biological monomers such as sugar derivatives and random chemistry monomers. Such a library also can contain, for example, biological oligomers such as peptides, oligonucleotides, oligosaccharides, polysomes, random chemistry oligomers, phage proteins and bacterial membrane proteins. Applications of combinatorial technologies to drug discovery and library screening strategies are well known in the art as described, for example, in Gordon et al., *supra,* 1994; Ecker and Crooke, *supra,* 1995), and Jung, Combinatorial Peptide and Nonpeptide Libraries: A Handbook, VCH Publishers, Weinheim (FRG) and New York, New York (USA)(1996). Combinatorial chemical libraries for screening also can also be obtained commercially, for example, from Trega Biosciences Inc. (San Diego, California); ProtoGene Inc. (Palo Alto, CA); Array Biopharma Inc. (Boulder, CO); or Maxygen Inc. (Redwood City, CA).

As disclosed herein, a complementary molecular analysis was performed to assay for differential expression of mRNA and protein levels in *Appl^{d}* flies compared to *Appl⁺* flies. Using differential display analysis, DNA microarrays or 2D gel electrophoresis coupled with mass spectrophotometry, a variety of mRNAs or proteins were isolated that are differentially expressed in *Appl^{d}* versus *Appl⁺ D*. *melanogaster.* Each of these differentially expressed mRNAs or proteins can correspond to a gene that is a member of an Alzheimer's disease genetic network and, thus, can correspond to an Alzheimer's disease gene useful in the above methods for identifying a therapeutic agent for treating Alzheimer's disease.

Thus, the disclosure describes an isolated nucleic acid molecule which is differentially expressed in *Appl^{d}* versus *Appl⁺ D. melanogaster* and contains a nucleic acid sequence having substantially the sequence of one of SEQ ID NOS: 1 to 63. Such a differentially expressed nucleic acid molecule can have, for example, the sequence of one of SEQ ID NOS: 1 to 63. Also provided herein is an isolated nucleotide sequence that contains at least 10 contiguous nucleotides of the nucleic acid sequence of one of SEQ ID NOS: 1 to 63.

The disclosure also describes an isolated nucleic acid molecule which is differentially expressed in *Appl^{d}* versus *Appl⁺ D. melanogaster* and contains a nucleic acid sequence having substantially the sequence of one of SEQ ID NOS: 64 to 80. Such an isolated nucleotide sequence can have, for example, the sequence of one of SEQ ID NOS: 64 to 80. The disclosure additionally describes an isolated nucleotide sequence containing at least 10 contiguous nucleotides of the nucleic acid sequence of one of SEQ ID NOS: 64 to 80.

As disclosed herein in Example II, differential display and DNA microarray analyses were used to identify more than 80 differentially expressed nucleic acid molecules (SEQ ID NOS: 1 to 80). Using differential display, 17 transcripts were increased while 46 transcripts were decreased in *Appl^{d}* flies relative to *Appl⁺* flies. In particular, transcripts 27.1 (SEQ ID NO: 20); 27.1a (SEQ ID NO: 21); 27.4 (SEQ ID NO: 23); 27.5 (SEQ ID NO: 25); 27.5b (SEQ ID NO: 26); 30.3 (SEQ ID NO: 36); 30.3b (SEQ ID NO: 37); 30.9 (SEQ ID NO: 42); 30.9b (SEQ ID NO: 43); 48.1 (SEQ ID NO: 51); 48.1b (SEQ ID NO: 52); 48.2 (SEQ ID NO: 53); 48.3 (SEQ ID NO: 54); 49.3 (SEQ ID NO: 55); 58.1 (SEQ ID NO: 56); 58.4 (SEQ ID NO: 60); and 58.4a (SEQ ID NO: 61) were increased in *Appl^{d}* flies relative to *Appl⁺* flies. Furthermore, transcripts Al (SEQ ID NO: 1); 22.1 (SEQ ID NO: 2); 22.2 (SEQ ID NO: 3); 23.1 (SEQ ID NO: 4); 23.1b (SEQ ID NO: 5); 23.4 (SEQ ID NO: 6); 23.5 (SEQ ID NO: 7); 23.6 (SEQ ID NO: 8); 23.7 (SEQ ID NO: 9); 23.7b (SEQ ID NO: 10); 24.1 (SEQ ID NO: 11); 24.3 (SEQ ID NO: 12); 24.3a (SEQ ID NO: 13); 24.4 (SEQ ID NO: 14); 24.5 (SEQ ID NO: 15); 25.1 (SEQ ID NO: 16); 25.1b (SEQ ID NO: 17); 26.1 (SEQ ID NO: 18); 26.3 (SEQ ID NO: 19); 27.2 (SEQ ID NO: 22); 27.4b (SEQ ID NO: 24); 27.5c (SEQ ID NO: 27); 27.15 (SEQ ID NO: 28); 27.18 ' (SEQ ID NO: 29); 28.2 (SEQ ID NO: 30); 28.3 (SEQ ID NO: 31); 29.3 (SEQ ID NO: 32); 29.3b (SEQ ID NO: 33); 29.4 (SEQ ID NO: 34); 30.1 (SEQ ID NO: 35); 30.4 (SEQ ID NO: 38); 30.4b (SEQ ID NO: 39); 30.7 (SEQ ID NO: 40); 30.7b (SEQ ID NO: 41); 30.12 (SEQ ID NO: 44); 30.12b (SEQ ID NO: 45); 34.2 (SEQ ID NO: 46); 35.1 (SEQ ID NO: 47); 35.2 (SEQ ID NO: 48); 37.9 (SEQ ID NO: 49); 45.1 (SEQ ID NO: 50); 58.2 (SEQ ID NO: 57); 58.2b (SEQ ID NO: 58); 58.3 (SEQ ID NO: 59); and 60.2 (SEQ ID NO: 62) were decreased in *Appl^{d}* flies as compared to *Appl⁺* flies.

Differential expression also was observed for 45 mRNAs using a DNA microarray made from 400 randomly chosen ESTs from the Berkeley *Drosophila* Genome Project UniGene Library. Of these differentially expressed mRNAs, 24 had increased expression and 21 had decreased expression in *Appl^{d}* relative to *Appl⁺.* Among these mRNAs, GH03592 (SEQ ID NO: 64); GH03824 (SEQ ID NO: 65); GH01554 (SEQ ID NO: 66); GH01770 (SEQ ID NO: 67); GH01730 (SEQ ID NO: 68); GH01988 (SEQ ID NO: 69); GH01718 (SEQ ID NO: 70); GH01072 (SEQ ID NO: 71); GH03622 (SEQ ID NO: 72); GH01420 (SEQ ID NO: 73); GH05210 (SEQ ID NO: 74); GH01717 (SEQ ID NO: 75); and GH01942 (SEQ ID NO: 76) exhibited increased expression in *Appl^{d}* relative to *Appl⁺*. In contrast, GH04745 (SEQ ID NO: 77); GH04984 (SEQ ID NO: 78); GH04859 (SEQ ID NO: 79); and GH03649 (SEQ ID NO: 80) had decreased expression in *Appl*^{d} relative to *Appl*⁺*.*

In addition, several differentially expressed mRNAs were identified as known genes: *kismet (kis),* a gene encoding a chromatin factor that interacts with *Notch* (Daubresse et al., Development 126:1175-1187 (1999)), and mitochondrial processing peptidase beta-subunit/vesicle trafficking protein SEC22B (Paces et al., Proc. Natl. Acad. Sci. USA 90:5355-5358 (1993); Mao et al.., Proc. Natl. Acad. Sci. USA 95:8175-8180 (1998)) were increased in *Appl^{d}* relative to *Appl⁺. Frequenin,* encoding a calcium-sensitive-guanyl-cyclase-activator (Pongs et al., Neuron 11:15-28 (1993)); *myosin-IB (Myo61F,* Morgan et al., J. Mol. Biol. 239:347-356 (1994)); *leonardo* (*leo*), encoding a 14-3-3ζ protein (Skoulakis and Davis, Neuron 17:931-944 (1996)); and fly homologs of the mammalian phosphatidic acid phosphatase 2a2 (Leung et al., DNA Cell Biol. 17:377-385 (1998)) exhibited decreased expression in *Appl^{d}* relative to *Appl⁺.*

As further disclosed herein, several genes with an altered expression level in *Appl^{d}* flies were analyzed phenotypically for an interaction with the *Appl* network. After crossing the fly mutants with *Appl^{d}* flies, the various progeny classes were scored for viability as described above. Two temperature-sensitive alleles of the dynamin-encoding *shibire* locus were tested with *Appl^{d},* and both showed reductions in viability that were temperature-sensitive (see Table 3). α*-adaptin* (Dornan et al., Mol. Biol. Cell 8:1391-1403 (1997)) and the fly's δ*-adaptin* homolog, *garnet* (Ooi et al., EMBO J. 16:4508-4518 (1997)) also were tested for an effect on viability when combined with *Appl^{d}.* These mutants affect genes whose products are involved in the same endocytic processes as dynamin. As shown in Table 3, the α*-adaptin⁰⁶⁶⁹⁴* mutation increased viability in combination with *Appl^{d},* whereas the *garnet* (*g³*) mutation in δ-adaptin decreased viability, indicating that these genes may belong to a local network. Table 3 also shows that the *cnc⁰⁹³²¹* mutation in the locus of *cap-n-collar (cnc),* a gene encoding a transcription factor with bZIP homology, affects viability in flies carrying no functional copy of *Appl.*

**Table 3**

| **Viability Tests of *Appl^{d}* with Other Loci** | | | | | | |
|---|---|---|---|---|---|---|
| Male | x Female | | N | % viability¹ female progeny (1 dose *Appl⁺*) | N | % viability² male progeny (0 dose of *Appl⁺*) |
| *w* | *w* | | 10 | 92.16 | | |
| | *Appl^{d}*/*FM7* | | 2 | | | |
| *shi^{ts1}* | w | 20° | 12 | 93.85 | | |
| | *Appl^{d}*/*FM7* | C | 6 | | | |
| *shi^{ts1}* | *w* | 27° | 19 | 70.69* | | |
| | *Appl^{d}*/*FM7* | C | 8 | | | |
| *shi^{ts139}* | w | 20° | 18 | 88.41 | | |
| | *Appl^{d}*/*FM7* | C | 7 | | | |
| *shi^{ts139}* | *w* | | 52 | 52.94* | | |
| | *Appl^{d}*/*FM7* | | | | | |
| *sca¹* | *w* | | 75 | 97.37 | | |
| | *Appl^{d}*/*FM7* | | | | | |
| *na* | *w* | | 87 | 112.20 | | |
| | *Appl^{d}*/*FM7* | | | | | |
| *har38* | *w* | | 23 | 134.65* | | |
| | *Appl^{d}*/*FM7* | | 7 | | | |
| *g³ bw* | *w* | | 22 | 69.17** | | |
| | *Appl^{d}*/*FM7* | | 5 | | | |
| *eag¹* | *w* | | 91 | 93.62 | | |
| | *Appl^{d}*/*FM7* | | | | | |
| *rut²⁰⁵⁰* | *w* | | 14 | 107.04 | | |
| | *Appl^{d}*/*FM7* | | 7 | | | |
| *amn ^{28A}* | *w* | | 87 | 97.73 | | |
| | *Appl^{d}*/*FM7* | | | | | |
| *hsCreb17 -2* | *w* | 27° C | 87 | 112.20 | 72 | 414.29** |
| | *Appl^{d}*/*FM7* | | | | | |
| *hsCrebC2 8* | *w* | 27° C | 83 | 102.44 | 55 | 57.14* |
| | *Appl^{d}*/*FM7* | | | | | |
| *cnc⁰⁹³²¹*/ | *w* | | 10 | 94.33 | 67 | 48.89** |
| *CyO* | *Appl^{d}*/*FM7* | | 3 | | | |
| *Su (H) I* | *w* | | 10 | 62.90* | | |
| | *Appl^{d}*/*FM7* | | 1 | | | |
| *D1⁷*/*TM3* | *w* | | 85 | 296.36** | | |
| | *Appl^{d}*/*FM7* | | | | | |
| α*-adapti* | *w* | | 92 | 268.00** | | |
| *n*/ *CyO* | *Appl^{d}*/*FM7* | | | | | |
| *w Appl^{d}* | *stnA¹⁵lFM* | | 14 | 93.33 | | |
| | 7 | | 5 | | | |
| *w Appl^{d}* | *wN²⁶⁴⁻³⁹*/*FM* | | 51 | 43.77* | | |
| | 7 | | 9 | | | |
| s*h*i^{ts139} | w*N*²⁶⁴⁻³⁹/*FM* 7 | 18° C | 68 | 44.68** | | |
| *w Appl^{d}* | *bib¹*/*CyO* | | 14 | 65.11* | | |
| | | | 2 | | | |
| *w Appl^{d}* | *mam⁸*/*CyO* | | 36 | 381.82** | | |
| *w Appl^{d}* | *Dhod⁸*/*TM3* | | 42 | 90.91 | | |
| *w Appl^{d}* | *Psn^{B3}*/*TM3* | | 76 | 111.11 | | |
| *w App^{d}* | *CrebA*⁰³⁵⁷⁶ / *TM3* | | 90 | 177.788* | | |
| *hsCreb17 -2* | *α-adapti n* / *CyO* | | 57 | 185.00* | | |

| | | | | | | |
|---|---|---|---|---|---|---|
| ¹ for crosses 1-21, % viability = [(# mutant/w *Appl^{d})*/*(#FM7*/*w Appl^{d})*]x100 for crosses 22-26, % viability = [(# *w Appl^{d}*/*+;* mutant/+)/(#w *Appl^{d}*/+; *Balancer*/+)]x 100 ² % viability = [(# *w Appl^{d}*/*Y; mutant*/+)/(*#w Appl^{d}*/*Y; Balancer*/+)]x 100 * indicates significant departure (P<0.05) from 100% by χ*²* test ** indicates significant departure (P<0.01) from 100% by χ*²* test | | | | | | |

All crosses carried out at 25°C unless otherwise indicated

**Table 4**

| **Gene Expression Differences** | |
|---|---|
| **mRNA** | |
| **Decreased expression in *Appl^{d}*** | **Increased expression in *Appl^{d}*** |
| *cap-n-collar (cnc)* | *kismet (kis)* |
| *tat-binding protein-1 (Tbp-1)* | *Frequenin (Frq)* |
| *shibire (shi)* | *ribosomal protein L9 (RpL9)* |
| *14-3-3ζ (leo)* | *pheromone-binding protein-related protein 2 (Pbprp2)* |
| *dihydroorotate dehydrogenase (Dhod)* | *exocyst protein 84^{r}* |
| *myosin -IB (Myo6lF)* | *mitochondrial processing peptidase-β*/*vesicle trafficking protein SEC22B^{h}* |
| *mitochondrial aldehyde dehydrogenase^{m}* | |

| **Protein** | |
|---|---|
| **Decreased expression in *Appl^{d}*** | **Increased expression in *Appl^{d}*** |
| *numb* | *adenyl cyclase (Ac39E)* |
| *hunchback (hb)* | |
| α*-actinin (Actn)* | |
| *actin57B (Act57B)* | |
| *mutant nucleotide excision repair protein (mus201)* | |

| | |
|---|---|
| *^{r}rat homolog* *^{m}mouse homolog* *^{h}human homolog* | |

**Table 5**

| **Identified Genes Differing between *Appl^{d}* and Appl⁺** | | |
|---|---|---|
| **Gene** | **Fly** | **Human** |
| *Appl* | J04516 | Y00264 |
| *Notch* | M16150/M11664 | M73980 |
| *Delta* | X06289 | X80903 (mouse) |
| *big brain* | X53275 | U36308 |
| *mastermind* | X54251 | ------- |
| *Suppressor of Hairless* | M94383 | L07872 |
| *CrebA* | M87038 | AF009368 |
| *CrebB (inhibitor)* | S79274 | NM_001881 |
| *CrebB (activator)* | S79274 | NM_004379 |
| *cap-n-collar* | M37495 | X77366 |
| *kismet* | AF113847 | ------- |
| *tat-binding protein-1 (Tbp-1)* | AF134402 | M34079 |
| *Frequenin* | L08064 | S47565 |
| *shibire* | X59435 | NM_004408 |
| α*-adaptin* | Y13092 | AF049527 |
| δ*-adaptin* | AF002164 | AF002163 |
| *ribosomal protein L9* | X94613 | NM_000661 |
| *14-3-3ζ* | Y12573 | NM_003406 |
| *pheromone-binding protein-related protein-2* | U05981 | ------- |
| *dihydroorotate dehydrogenase* | L00964 | M94065 |
| *exocyst protein 84* | ------- | AF032669 |
| *myosin-IB* | U07596 | U57053 |
| *mitochondrial processing peptidase-β* | ------- | NM_004279 |
| *vesicle trafficking protein (SEC22B)* | ------- | NM_004892 |
| *phosphatidic acid phosphatase 2a2* | ------- | AF014403 |
| *mitochondrial aldehyde dehydrogenase* | ------- | NM_000690 |
| *numb* | M27815 | NM_003744 |
| *adenyl cyclase* | AF005629 | U65474 |
| *hunchback* | U17742 | ------- |
| *α-actinin* | X51753 | D89980 |
| *actin57B* | K00673 | X04098 |
| *mutant nucleotide excision repair protein (mus201)*/*human xeroderma pigmentosum VII* | AF162795 | X69978 |

The term "isolated," as used herein in reference to a nucleic acid molecule, nucleotide sequence or protein means a nucleic acid molecule, nucleotide sequence or protein that is in a form relatively free from contaminating lipids, unrelated nucleic acids, unrelated proteins and other cellular material normally associated with a nucleic acid molecule or protein in a cell.

As used herein, the term "nucleic acid molecule" means a deoxyribonucleic acid (DNA) or ribonucleic acid (RNA) molecule that can optionally include one or more non-native nucleotides, having, for example, one or more modifications to the base, sugar, or phosphate portion, or can include a modified phosphodiester linkage. The term nucleic acid molecule includes both single-stranded and double-stranded nucleic acid molecules, which can represent the sense strand, anti-sense strand, or both, and includes linear, circular and branched conformations. Exemplary nucleic acid molecules include genomic DNA, cDNA, mRNA and oligonucleotides, corresponding to either the coding or non-coding portion of the molecule. A nucleic acid molecule can additionally contain, if desired, a detectable moiety such as a radiolabel, fluorochrome, ferromagnetic substance, luminescent tag or a detectable agent such as biotin.

As used herein, the term "nucleotide sequence" means a single-stranded nucleic acid sequence that can range in size from about 10 contiguous nucleotides to the full-length of a nucleic acid molecule. A nucleotide sequence, which can be useful, for example, as a primer for PCR amplification, can have a sequence of at least, for example, 10, 11, 12, 13, 14, 15, 16, 17, 18, 19, 20, 25, 30, 35, 50, 100, 200, or more nucleotides.

The term "differentially expressed," as used herein, means the increased or decreased expression of a nucleic acid molecule or protein in a first genetic background as compared to a second genetic background. In general, the term differentially expressed is used herein to refer to increased or decreased expression of a nucleic acid molecule or protein in a genetic background in which the level or activity of an Alzheimer's disease gene product is abnormal compared to a genetic background of wild type gene product activity, for example *Appl^{d}* versus *Appl⁺.*

The term "substantially the sequence," as used herein in reference to a differentially expressed nucleic acid sequence is intended to mean one of the sequences shown as SEQ ID NOS:1 to 80, or a similar, non-identical sequence that is considered by those skilled in the art to be a functionally equivalent sequence. For example, a nucleic acid sequence that has one or more nucleotide additions, deletions or substitutions with respect to the indicated D. *melanogaster* nucleic acid sequence. so long as the nucleic acid molecule retains its ability to selectively hybridize with the D. *melanogaster* nucleic acid sequence. A nucleic acid molecule having substantially the sequence of one of the indicated differentially expressed transcripts can be, for example, an isotype variant or species homolog, such as a vertebrate or invertebrate homolog, including a mammalian homolog such as murine, primate or human homolog.

The disclosure further describes an isolated protein which is differentially expressed in *Appl^{d}* versus *Appl⁺ D. melanogaster* and has a specific molecular weight and isoelectric point, or a homolog thereof. Thus, the disclosure describes, for example, an isolated A1.2 protein which is differentially expressed in *Appl^{d}* versus *Appl⁺ D*. *melanogaster* and has an approximate molecular weight of about 40 kDa and an approximate isoelectric point of 9.5, or a homolog thereof. The disclosure similarly describes the proteins A1.3 to A1.29 and the proteins W1.2 to W1.25, which have the approximate molecular weights and isoelectric points shown in Table 6. Molecular weights were determined by 2D gel electrophoresis (see Example IIC).

2D gel electrophoresis followed by in-gel trypsinization and MALDI-TOF analysis of the resulting tryptic fragments, was used to identify 54 protein differences, of which 29 represented increased protein levels and 25 represented decreased protein levels in *Appl^{d}* relative to *Appl⁺.*

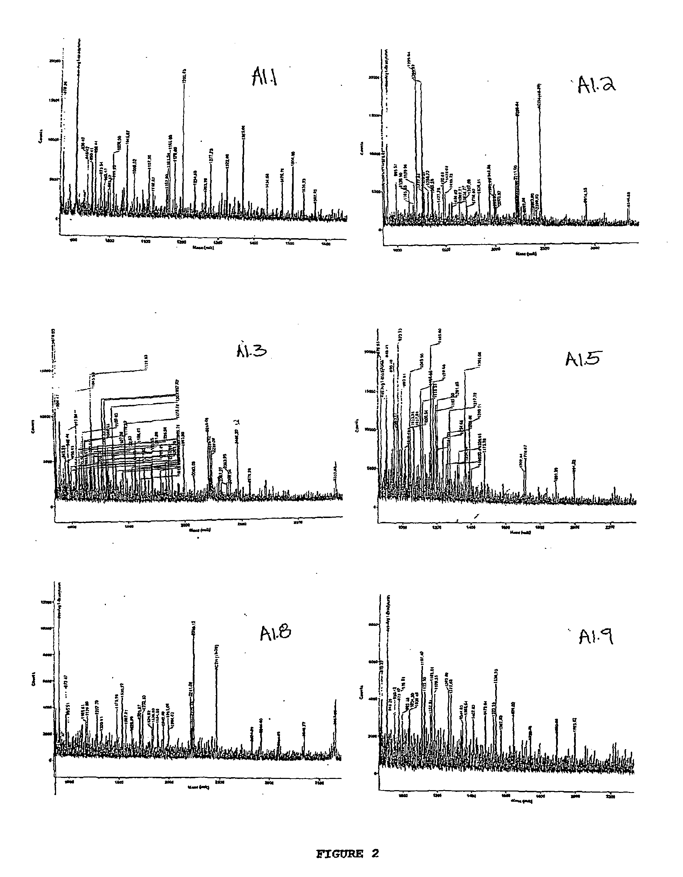

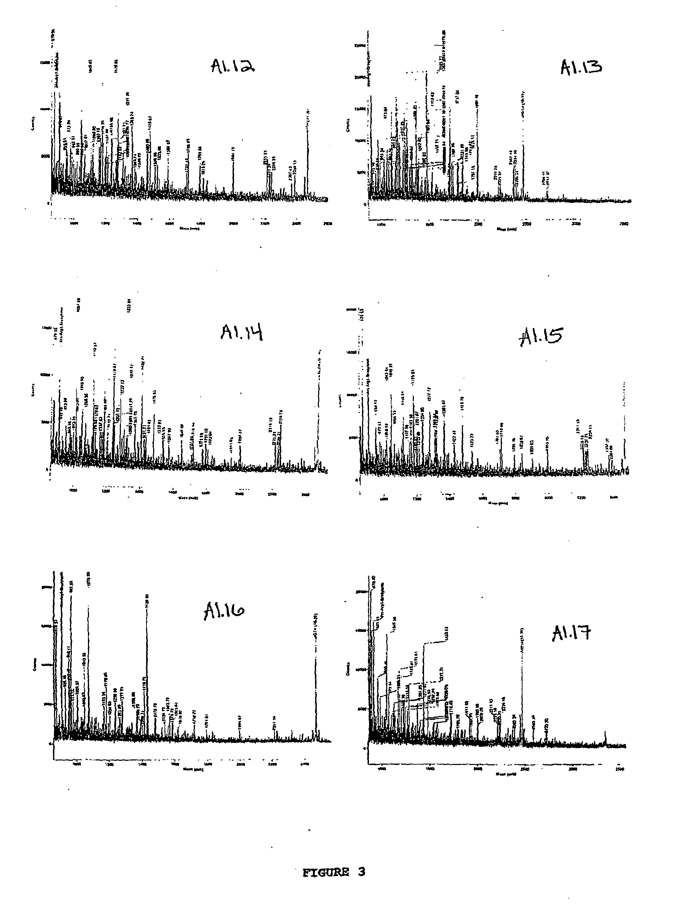

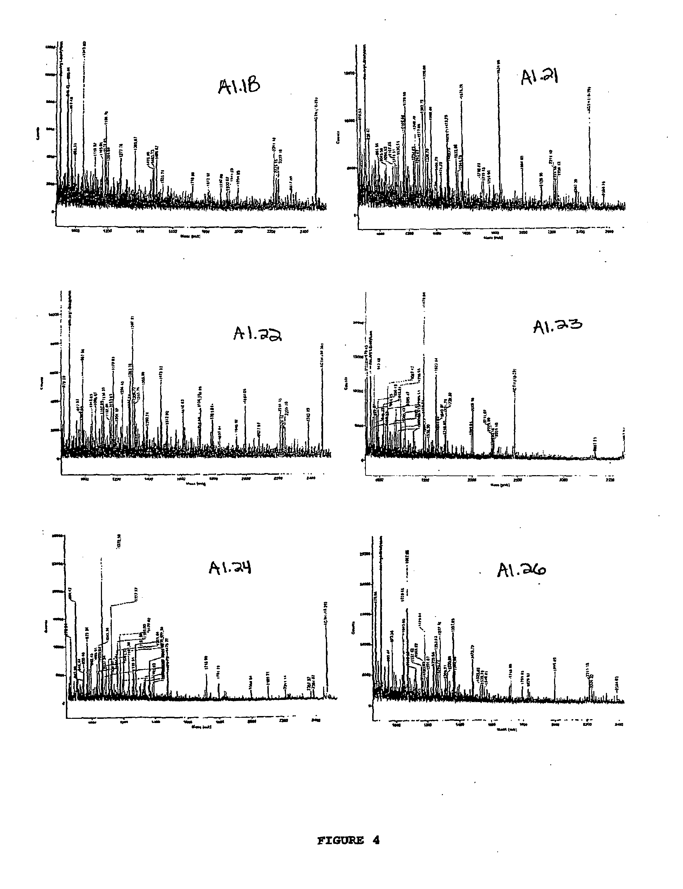

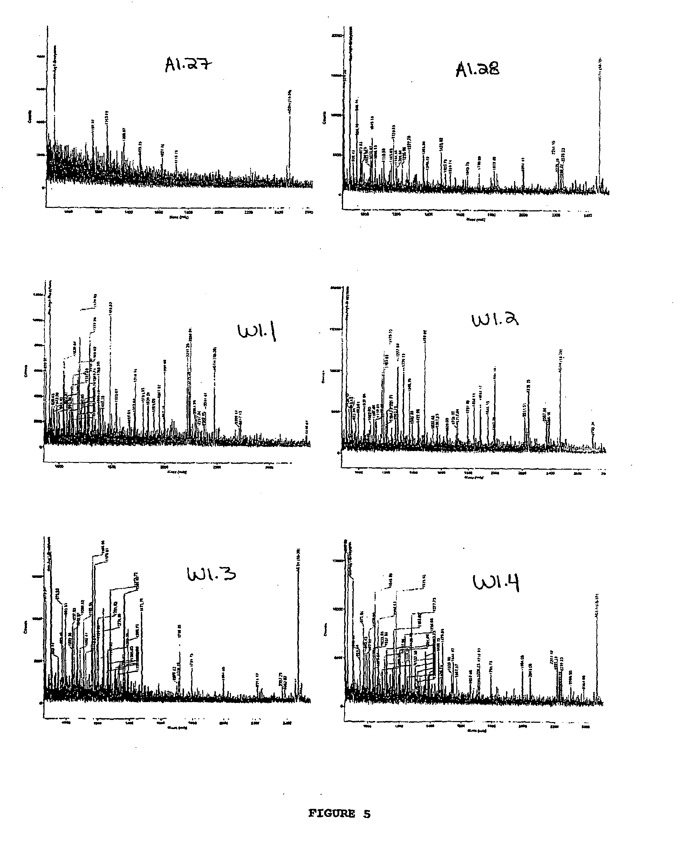

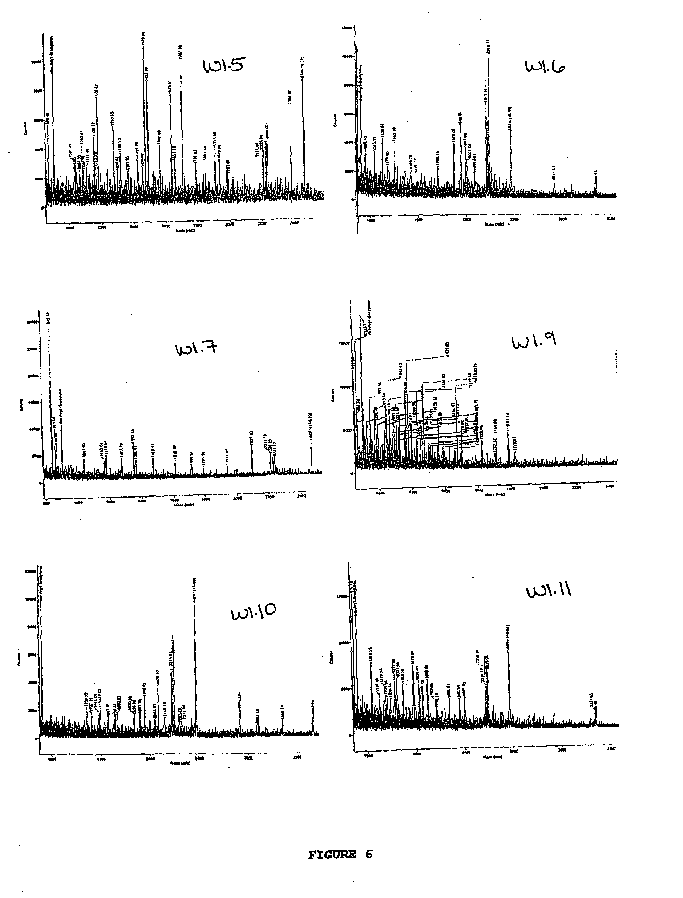

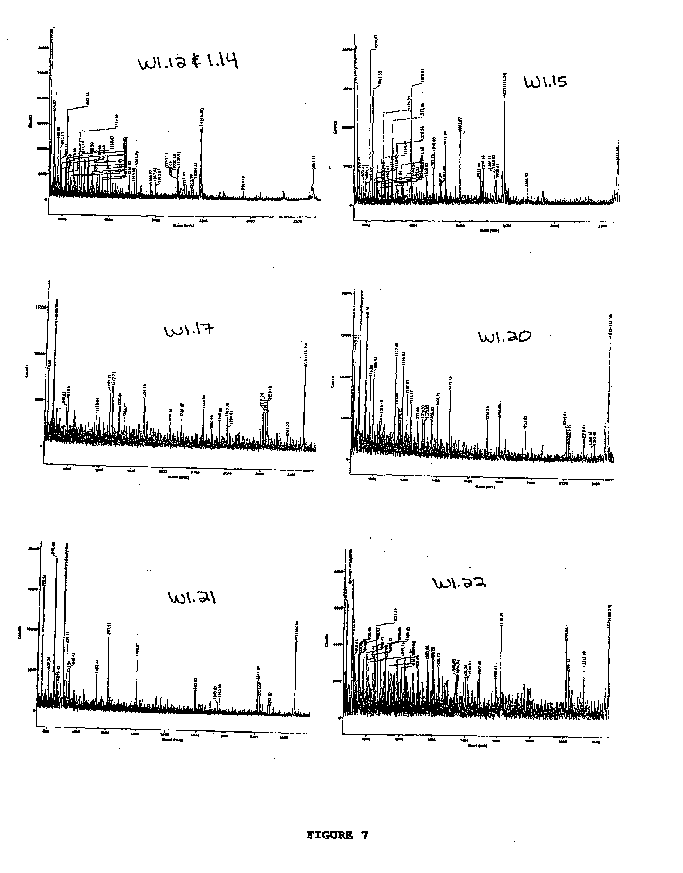

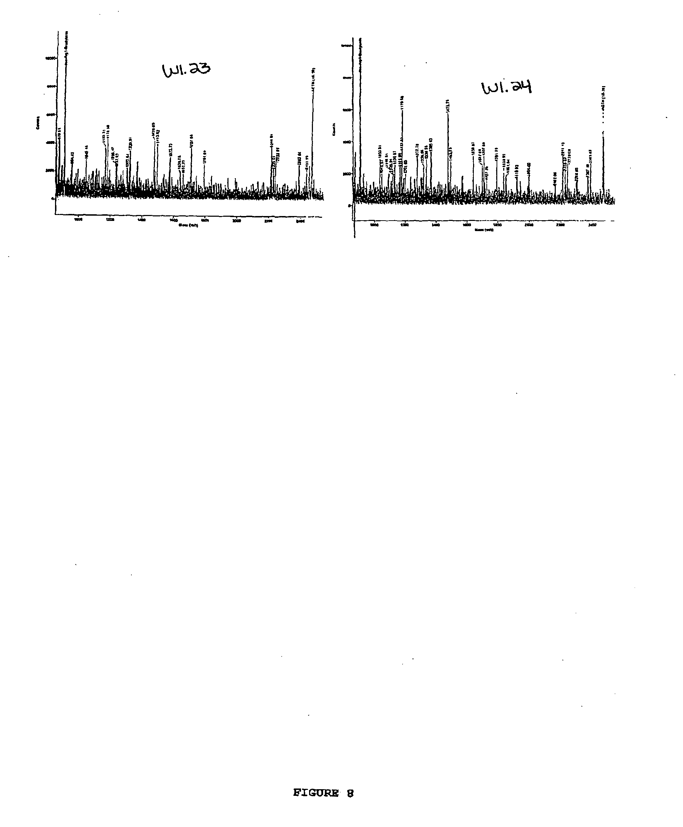

Proteins A1.2, A1.3, A1.4, A1.5, A1.6, A1.7, A1.8, A1.9, A1.10, A1.11, A1.12, A1.13, A1.14, A1.15, A1.16, A1.17, A1.18, A1.19, A1.20, A1.21, A1.22, A1.23, A1.24, A1.25, A1.26, A1.27, A1.28 and A1.29 are proteins with increased expression in *w Appl*^{d} as compared *to w Appl⁺.* W1.2, W1.4, W1.6, W1.7, W1.8, W1.9, W1.10, W1.12, W1.13, W1.14, W1.15, W1.16, W1.18, W1.19, W1.20, W1.22, W1.23, W1.24, and W1.25 are proteins with decreased expression *in w Appl^{d}* as compared *to w Appl⁺.* In addition to their differntial expression in *Appl^{d}* versus *Appl⁺,* these proteins are characterized by the molecular weights and isoelectric points shown in Table 6. Furthermore, these proteins are characterized by mass spectrophotometric analysis of in-gel tryptic digests, which are shown in Figures 2 to 8.

As shown in Table 4, five proteins with decreased expression in *Appl^{d}* flies were identified as follows: *numb,* a protein required for asymmetric cell divisions in neural development (Uemura et al., Cell 58:349-360 (1989)); *hunchback,* a transcription factor required for segmentation and for neural development (Tautz et al., Nature 327:383-389 (1987)); *mus201,an* excision repair protein (Houle and Friedberg, Gene 234:353-360 (1999)); and the cytoskeletal proteins *α-actinin* (Fyrberg et al., J. Cell Biol. 110:1999-2011 (1990)) and *actin57B* (Fyrberg et al., Cell 24:107-116 (1981)). One protein was identified with increased expression in *Appl^{d}* flies relative to *Appl⁺* flies: an isoform of adenyl cyclase, *Ac39E* (Genbank AF005629), which is distinct from the one encoded by the *rutabaga* locus (see Table 4). Table 5 summarizes the genes identified as having differential mRNA or protein expression in *Appl^{d}* as compared to Appl⁺.

**Table 6**

| **Molecular Weights and Isoelectric Points to Accompany Mass Spectra of Proteins Differing on w *Appl^{d}* (A1) vs. *w Appl⁺* (W1) 2D gels** | | | | | |
|---|---|---|---|---|---|
| | **MW** (kD) | **pI** | | **MW** (kD) | **pI** |
| A1.2 | 32.1 | 9.5 | W1.2 | 19.1 | 7.7 |
| A1.3 | 47.9 | 9.5 | W1.4 | 15.7 | 9.0 |
| A1.4 | 10.9 | 8.4 | W1.6 | 27.4 | 7.4 |
| A1.5 | 13.9 | 5.8 | W1.7 | 27.4 | 8.0 |
| A1.6 | 21.6 | 6.6 | W1.8 | 33.4 | 6.7 |
| A1.7 | 42.5 | 6.7 | W1.9 | 51.9 | 6.8 |
| A1.8 | 47.9 | 6.7 | W1.10 | 60.9 | 6.8 |
| A1.9 | 51.9 | 6.7 | W1.12 | 23.3 | 6.8 |
| A1.10 | 80.6 | 6.8 | W1.13 | 22.4 | 6.9 |
| A1.11 | 28.5 | 6.2 | W1.14 | 22.4 | 7.0 |
| A1.12 | 32.1 | 6.3 | W1.15 | 9.3 | 6.8 |
| A1.13 | 30.9 | 6.7 | W1.16 | 11.4 | 6.8 |
| A1.14 | 37.7 | 6.8 | W1.18 | 12.3 | 6.4 |
| A1.15 | 33.4 | 6.8 | W1.19 | 9.7 | 6.4 |
| A1.16 | 68.7 | 5.8 | W1.20 | 42.5 | 4.4 |
| A1.17 | 66.0 | 6.4 | W1.22 | 56.2 | 4.5 |
| A1.18 | 80.6 | 6.7 | W1.23 | 26.3 | 4.4 |
| A1.19 | 98.4 | 6.2 | W1.24 | 24.3 | 4.5 |
| A1.20 | 110.9 | 5.9 | W1.25 | 22.4 | 5.6 |
| A1.21 | 44.2 | 5.7 | | | |
| A1.22 | 29.7 | 5.7 | | | |
| A1.23 | 23.3 | 5.6 | | | |
| A1.24 | 12.3 | 5.6 | | | |
| A1.26 | 10.5 | 5.6 | | | |
| A1.27 | 11.8 | 5.6 | | | |
| A1.28 | 18.4 | 5.5 | | | |
| A1.29 | 19.1 | 5.6 | | | |

Also disclosed are homologs of the differentially expressed *D.* melanogaster proteins having the characteristic molecular weights and isoelectric points shown in Table 6. Such homologs include vertebrate and invertebrate homologs, including mammalian homologs such as murine, primate and human homologs and generally share conserved sequence and function with the homologous *D. melanogaster* protein. A human or other homolog can share, for example, at least about 25%, 30%, 40%, 50%, 60%, 75%, 90% or 95% amino acid identity with the indicated *D. melanogaster* homolog. Such homologous proteins can be used, for example, to prepare antibodies.

### EXAMPLE I

### GENE-NETWORK MAPPING OF GENES ACTING IN THE SAME NETWORK AS THE DROSOPHILA AMYLOID PROTEIN PRECURSOR-LIKE GENE (Appl)

This example describes identification of genes acting within the same genetic network as *Appl,* the *Drosophila* homolog of human amyloid protein precursor (APP).

### A. Interaction of Appl with X chromosome deficiencies

Male flies bearing a chromosome that lacks the *Appl* gene (*w Appl^{d};* Kalpana White, Brandeis University, Waltham, MS), which is not required for viability or for most other functions in *Drosophila* (Luo et al., *supra,* 1992), were crossed with a series of FM7 virgin females bearing individual deficiencies of the X chromosome, "*Df(1)s*"*.* This set of deficiencies altogether covers roughly 70% of the X chromosome, nearly 15% of the entire genome and thus serves as a representative sample of the genome. In each case, the number and genotype of female adults emerging from each cross were scored, and the viability of the test genotype relative to sibling controls calculated (see Table 1). In 34 combinations of X chromosomal deficiencies with the *Appl^{d}* chromosome (*Appl⁻*/*Df(1)*)*,* most had approximately normal viability, five had severely reduced viability (<35%), seven had moderately reduced viability, and three had increased viability relative to the control (Table 1).

Viability was determined as follows. Flies were cultured on brewer's yeast, dark corn syrup and agar food (modified from Bennett and van Dyke, Dros. Inform. Serv. 46:160 (1971)) at 25°C, 50-60% relative humidity and in 12hr:12hr light:dark cycles. Viability was scored by counting adult flies in the first or second day after emergence. Unless otherwise indicated, all fly stocks were obtained from the Bloomington *Drosophila* Stock Center.

### B. Interaction of Appl^{d} with network candidate genes

Four chromosome segments from the initial *Appl^{d}*/*Df(1)* screen described above were analyzed further.

As shown in Table 1, the deletion of segment 3C2; 3E4, (*Df(1)N8*)*,* when combined with *Appl^{d},* resulted in progeny with 32% viability relative to the controls. This segment contains the *Notch* locus (Artavanis-Tsakonas et al., Science 284:770-776 (1999)), a human homolog of which has been implicated in a form of hereditary degenerative dementia (Joutel et al., *supra,* 1996). *Notch* is also known to interact genetically with *Presenilin,* the fly homolog of human Presenilin (Struhl and Greenwald, Nature 398:522-525 (1999); Ye et al., Nature 398:525-529 (1999)). A null allele of *Notch, N²⁶⁴⁻³⁹,* was tested in combination with *Appl^{d}* and found to result in a viability reduction similar to that of the deficiency *Df (1) N8* (see Table 3).

Several mutants known to interact with *Notch* also were tested for their effects in the viability assay. Neither *scabrous* (*sca*), which encodes a protein with a fibrinogen-like beta and gamma chain C-terminal domain (Brand and Campos-Ortega, Roux Arch. Dev. Biol. 198:275-285 (1990)), nor *Presenilin (Psn^{B3})* mutations affected viability. *Suppressor of Hairless (Su(H)1),* which encodes a DNA-binding protein (Fortini and Artavanis-Tsakonas, Cell 79:273-282 (1994)), *and big brain (bib),* which encodes a channel-like transmembrane protein (Rao et al., Nature 345:163-167 (1990)), each gave moderate reductions in viability with *Appl^{d}.* Two other genes gave significant increases in relative viability when combined with *Appl^{d}: mastermind (mam),* a nuclear protein, (Smoller et al., Genes Dev. 4:1688-1700 (1990)) and *Delta* (*D1*)*,* a transmembrane protein that is a ligand of Notch (Vaessin et al., EMBO J. 6:3431-3440 (1987)). *Notch* and *Delta* gave reciprocal effects with *Appl^{d}*; these genes also are reciprocally regulated on cells as part of their normal signaling function (Heitzler and Simpson, *supra,* 1991).

The deletion of segment 17A1;18A2 *(Df(1)N19)* in combination with *Appl^{d}* resulted in 62.8% viability relative to sibling controls (Table 1). The 17A1;18A2 segment contains the *dCrebB* locus, a transcription factor implicated in neuronal plasticity and long-term memory formation (Dubnau and Tully, *supra,* 1998). The *Appl^{d}* chromosome was tested in combination with transgenic strains of *Drosophila* expressing either an activator form of *dCrebB (C28)* or an inhibitor form (17-2). As summarized in Table 3, the activator form produced a decrease in viability, whereas the inhibitor form produced an increase in viability relative to controls. These interactions were not apparent in flies carrying one dose of *Appl⁺* (heterozygous females) but were evident in hemizygous males, which lack a functional copy of *Appl⁺.* The alterations in the viability seen with the Creb variants paralleled the reciprocity of cellular function for the two forms of the transcription factor. A mutation of the other *dCreb* locus on the third chromosome *(CrebA ⁰³⁵⁷⁶;* Andrew et al., Development 124:181-193 (1997)), also was tested for the ability to interact with *Appl^{d}.* Increased viability was observed in flies carrying one dose of *Appl⁺* (heterozygous females) and one dose of the hypomorphic *CrebA⁰³⁵⁷⁶* mutation, as also observed for the *dCrebB* inhibitor.

The deletion of segment 12D2;13A5 *(Df(1)RK2)* in combination with *Appl^{d}* resulted in 122% viability relative to controls (Table 1). Of the several mutations from this region that are available, *rutabaga* (rut), encoding an adenyl cyclase involved in neuronal plasticity and learning (Livingstone et al., Cell 37:205-215 (1984)) and *eag,* encoding a potassium channel subunit (Warmke et al., Science 252:1560-1562 (1991)), gave no significant effects when combined with *Appl^{d}.* However, the *halothane-resistant* mutant (*har38,* Krishnan and Nash, Proc. Natl. Acad. Sci. USA 87:8632-8636 (1990)) resulted in increased viability in combination with *Appl^{d},* consistent with the deficiency phenotype. *har38* is allelic to *narrow abdomen* (*na*, Krishnan and Nash, *supra,* 1990), a morphological mutation, which showed no viability effect in combination with *Appl^{d}.* The allele-specific interaction indicates that *har38* can interact directly with *Appl* in *Drosophila.* Given that halothane-sensitivity in flies is virtually identical to that of humans (Krishnan and Nash, *supra,* 1990), these results also indicate that the human homolog of *har38* can interact with APP.

The deletion of a fourth segment, 18E-20 (*Df(1)HF396*), resulted in 11.9% viability relative to controls when combined with *Appl^{d}* (Table 1). Subdivision of the region into smaller segments showed no comparable reduction for any segments from 19A2 to 20EF. A mutant implicated in neuronal plasticity, *amnesiac (amn^{28A}),* was tested for interactions with *Appl^{d}.* This mutant, which is located at and encodes a PACAP-like neuropeptide required for the formation of medium-term memory, resulted in normal viability in combination with Appl^{d}, as did the *stoned* (*stnA¹⁵*) mutation at 20A4, which affects synaptic physiology and plasticity.

### EXAMPLE II

### ANALYSIS OF DIFFERENCES IN GENE EXPRESSION IN Appl^{d} VERSUS WILD TYPE FLIES

This example demonstrates the use of differential display, DNA microarray analysis and 2D gel electrophoresis to identify genes involved in an Alzheimer's disease gene network.

### A. Differential display analysis

Differential display analysis of RNA isolated from adult heads of *w Appl^{d}* vs. wild type (*w*) siblings was used to identify genes with increased or decreased expression in *Appl^{d}*. Briefly, 20 fly heads were homogenized in TRIzol (Life Technologies, Inc., Frederick, MD) and extracted according to the manufacturer's instructions. Differential display of mRNA was performed essentially as described in Cirelli and Tononi, Mol. Brain Res. 56:293-305 (1998). About 38 transcripts were identified with expression levels altered in *Appl^{d}*flies. Of these, 16 had increased expression, and 22 had decreased expression in *Appl^{d}* flies relative to *Appl⁺* flies.

Sequence analysis using the BLAST program at either the Berkeley Drosophila Genome Project <http://www.fruitfly.org/blast/> or NCBI <http://www.ncbi.nlm.nih.gov/BLAST/> revealed that about 15% of the transcripts represented sequences currently available in *Drosophila* databases (Table 4). In particular, the following genes were identified with altered RNA expression levels in *Appl^{d}* flies. *Shibire* (*shi*), which encodes dynamin (van der Bliek and Meyerowitz, Nature 351:411-414 (1991)); *cap-n-collar* (cnc), which encodes a bZIP-like transcription factor (Mohler et al., Mech. Dev. 34:3-9 (1991)), *Pbprp-2,* which encodes a pheromone-binding-protein-related-protein (Pikielny et al., Neuron 12:35-49 (1994)); *RpL9,* encoding ribosomal protein L9 (Schmidt et al., Mol. Gen. Genet. 251:381-387 (1996)); *Dhod* (Jones et al., Mol. Gen. Genet. 219:397-403 (1989)), 18S ribosomal RNA; *Tat-binding protein-1 (Tbp-1,* FlyBase FBgn0026321) of the proteasome; and a homolog of rat *exo84* of the exocyst secretion complex (Kee et al., Proc. Natl. Acad. Sci. USA 94:14438-14443 (1997)).

### B. DNA microarray analysis

mRNA levels also were compared using a DNA microarray made from 400 randomly chosen ESTs from the Berkeley *Drosophila* Genome Project UniGene Library (http://www.fruitfly.org/EST/). Briefly, a glass slide DNA microarray was prepared as described in White et al., Science 286:2179-2184 (1999), from the UniGene Library (plates #54, 55, 56 and 57; Research Genetics, Huntsville, AL). PolyA+ RNA was prepared from groups of 100 whole flies using MicroPoly(A)Pure (Ambion, Austin, TX) according to the manufacturer's instructions, and subsequently labeled and hybridized to the arrays as described in White et al., *supra,* 1999. ESTs showing >2-fold expression difference were analyzed as described above.

Differential expression was observed for 45 mRNAs (24 increased and 21 decreased in *Appl^{d}* relative to *Appl⁺).* Of the 45 mRNAs, six were identified as known genes as follows: *kismet* (*kis*)*,* a gene encoding a chromatin factor that interacts with Notch (Daubresse et al., supra, 1999); Frequenin, encoding a calcium-sensitive-guanyl-cyclase-activator (Pongs et al., *supra,* 1993); *leonardo* (*leo*), encoding a 14-3-3z protein (Skoulakis and Davis, *supra,* 1996); *myosin-IB (Myo61F,* Morgan et al., *supra,* 1994); fly homologs of the mammalian phosphatidic acid phosphatase 2a2 (Leung et al., *supra,* 1998) and mitochondrial processing peptidase beta-subunit/vesicle trafficking protein SEC22B (Paces et al., *supra,* 1993; Mao et al., *supra,* 1998).

### C. 2D gel electrophoretic analysis of differentially expresses proteins

2D gel electrophoresis/mass spectrophotometry analysis was performed on adult head extracts from *w Appl^{d}* vs. w siblings (Gygi et al., Mol. Cell.Biol. 19:1720-1730 (1999)). Briefly, 35 fly heads were homogenized using the extraction protocol described in Unlu et al., Electrophoresis 18:2071-2077 (1997), and 2D gel electrophoresis was performed as follows. For the first dimension, samples were diluted up to 125µl with a rehydration solution consisting of 8 M urea, 2 M thiourea, 2% CHAPS, 0.5% 3-10L IPG Buffer, and a trace of bromophenol blue. The samples were allowed to sit in the rehydration solution for 30 minutes before being applied to 3-10L Immobiline DryStrips. After rehydrating the strips for 12 hours at 20°C, they were electrophoresed on the Pharmacia IPGPhor in steps of 250Vh, 500Vh and 8000Vh. For the second dimension, the strips were equilibrated for 15 minutes in 5 mls of an equilibration solution consisting of 50 mM Tris-Cl pH 8.8, 6 M urea, 30% glycerol, 2% SDS, and 50 mgs of dithiothreitol. The gels were then overlayed on 4-20% pre-cast Biorad Mini-gels and electrophoresed for 33 minutes at 200V using standard SDS running buffer. Gels were silver stained using the Pharmacia Silver Staining Kit (Amersham-Pharmacia Biotech, Inc., Piscataway, NJ). Molecular weights were approximated to +/- 25% of the actual molecular weight using the following molecular weight standards 200kD, 135kD, 81kD, 41.9kD, 31.4kD, 18kD, and 6.9kD.

Spots were matched between gels using the PDQuest program (BioRad, Hercules, CA), and those showing the strongest differences were then excised with a scalpel and subjected to in-gel trypsin digestion, extraction and purification in preparation for MALDI-TOF analysis (Helmann et al, Anal. Biochem. 224:451-455, (1995)), which was performed at the Scripps Research Institute Mass Spec Facility. The resulting spectra were analyzed by matching peptide patterns to those in the database (http://prowl.rockefeller.edu/cai-bin/ProFound).

Using the above procedure, fifty-four protein differences were analyzed, of which 29 represented increased protein levels and 25 represented decreased protein levels in *Appl^{d}* relative to *Appl⁺.* Table 6 shows the molecular weights and isoelectric points of proteins differing in expression between *Appl^{d}* and *Appl⁺*.

Of the fifty-four protein differences analyzed, five proteins were identified as gene products of the following loci: *numb,* a protein required for asymmetric cell divisions in neural development (Uemura et al., Cell 58:349-360 (1989)); *hunchback,* a transcription factor required for segmentation and for neural development (Tautz et al., *supra,* 1987); *mus201,* an excision repair protein (Houle and Friedberg, *supra,* 1999); and the cytoskeletal proteins α-actinin (Fyrberg et al., *supra,* 1990) and *actin57B* (Fyrberg et al., *supra,* 1981). As shown in Table 4, one protein was elevated in *Appl^{d}* flies relative to *Appl⁺* flies: an isoform of adenyl cyclase, *Ac39E* (Genbank AF005629), which is distinct from the one encoded by the rutabaga locus. Table 5 summarizes the genes identified as differing in expression between *Appl^{d}* and *Appl⁺.*

### EXAMPLE III

### GENETIC AND BEHAVIORAL ANALYSIS OF LOCI WITH DIFFERING EXPRESSION IN Appl^{d} FLIES

This example describes the genetic analysis of loci that exhibit altered expression levels in *Appl^{d}.*

### A. Genetic analysis of genes identified by expression analysis

Genes with an altered expression level in *Appl^{d}* flies were analyzed phenotypically for an interaction with the *Appl* network. Available mutants for the genes identified by expression analysis were obtained and crossed with *Appl^{d}* flies. The various progeny classes were scored for viability as described above.

The dynamin-encoding *shibire* locus is on the X chromosome at salivary chromosome band 14A1. A chromosomal deficiency including 14A1, *Df(1)sd72b,* showed a moderate decrease in viability with *Appl^{d}* (see Table 1). Two temperature-sensitive alleles of *shibire* were tested with *Appl^{d},* and both showed reductions in viability that were temperature-sensitive (see Table 3).

Two other mutants in genes whose products are involved in the same endocytic processes as dynamin were tested for interactions with *Appl^{d}: α-adaptin* (Dornan et al., *supra,* 1997) and the fly's δ-*adaptin* homolog, *garnet* (Ooi et al., *supra,* (1997). The α-*adaptin⁰⁶⁶⁹⁴* mutation increased viability in combination with *Appl^{d}* whereas the *garnet (g³)* mutation in δ-adaptin decreased viability (Table 3), indicating that these genes may belong to a local network. Although no fly mutant exists for *exo84*, a protein component of the exocyst complex involved in vesicle fusion and secretion in yeast and mammals (Kee et al., *supra,* 1997), this gene, which is differentially increased in *Appl^{d}*, also can be involved in the *Appl* network, given the phenotypic involvement of dynamin, α-*adaptin* and δ-*adaptin*. The *cnc⁰⁹³²¹* mutation in the locus of *cap-n-collar (cnc),* a gene encoding a transcription factor with bZIP homology, affects viability in flies carrying no functional copy of *Appl* (see Table 3).

### B. Analysis of genetic interactions between genes affecting Appl.

*Notch* and *shibire*, which share a common interaction with *Appl^{d},* were analyzed further. When mutations in *Notch (N²⁶⁴⁻³⁹)* and *shibire (shi^{st139})* were combined, the result was a major reduction in viability. Of those flies that did survive to adulthood, emergence was significantly delayed and the notched wing phenotype resulting from the combination of *N²⁶⁴⁻³⁹* and *shi^{st139}* was much more severe than for *N²⁶⁴⁻³⁹* alone.

Possible lateral interactions were further analyzed between α-*adaptin* and the *dCreb* variants. Crosses between α-adaptin and the *dCreb* variants were performed, and an effect on viability of the *hsCreb17-2* inhibitor was observed. The effect showed the same polarity, an increase in viability, as had been seen previously in its interaction with *Appl^{d}* (Table 3).

### C. Behavioral tests of Appl^{d} Interactions

*Appl^{d}* flies have a characteristic behavioral phenotype: a defect in fast phototaxis (Luo et al., *supra,* 1992). *Appl^{d}* flies are non-phototactic, and the phenotype is fully recessive. Thus, *Appl^{d}*/*+* flies are phenotypically normal (see Table 7). Fast phototaxis was assayed as described in Benzer, Proc. Natl. Acad. Sci. USA 58:1112-1119 (1967), on flies aged 3-5 days. Flies were analyzed to determine whether the normal phototaxis of flies with one dose of *Appl⁺* could be made abnormal in combination with the loss of one dose from an interacting loci. Conversely, flies were analyzed to determine whether the defective phototaxis in flies with no functional *Appl⁺* could be ameliorated by loss of one dose from one of these loci. Of the various loci shown to affect viability (Table 3), two showed significant phototaxis interactions with *Appl^{d}*/*+* flies: *Notch* and *Delta.* Three showed significant phototaxis interactions with *Appl^{d}* flies: *α-adaptin, dCrebB* and *dCrebA*.

These results utilize a second phenotypic assay to independently confirm the involvement of several loci indicated to be part of an Alzheimer's disease network based on their effects on viability when combined with *Appl^{d}.*

**Table 7**

| **Phototaxis Tests of Appl with Other Loci** | | | |
|---|---|---|---|
| Females | N | Phototaxis Score | S.E.M. |
| *w Appl^{d}*/*w Appl^{d}* | 2 | 1.95 | 0.46 |
| *w Appl^{d}*/*+* | 2 | 5.16* | 0.16 |
| *w Appl^{d}*/*+; D1⁷*/*+* | 2 | 2.26 | 1.07 |
| *Dl*⁷/+ | 2 | 4.32* | 0.48 |
| *w Appl^{d}*/*w N²⁶⁴⁻³⁹* | 5 | 2.53 | 0.29 |
| w/w *N*²⁶⁴⁻³⁹ | 3 | 4.20* | 0.32 |

| Males | | | |
|---|---|---|---|
| *w Appl^{d}*/*Y* | 2 | 2.07 | 0.14 |
| *w Appl^{d}*/*Y;* α*-adaptin⁰⁶⁶⁹⁴*/*+* | 8 | 3.78* | 0.57 |
| *w Appl^{d}*/*Y; hs-Creb 17-2*/*+* | 10 | 4.96* | 0.18 |
| *w Appl^{d}*/*Y; CrebA⁰⁸⁵⁷⁶*/*+* | 2 | 4.81* | 0.40 |

| | | | |
|---|---|---|---|
| Phototaxis tested as described in Benzer, *supra,* 1967, with 20-40 files per assay. *indicates significant difference (P<0.05) from control *(w Appl^{d}*/*+* for Females, *w Appl^{d}*/*Y* for Males) by Dunnett's test. | | | |

## Claims

1. A method of mapping a network of functional gene interactions relating to Alzheimer's disease, comprising the steps of:
(a) performing matings between (1) a first parent strain of *Drosophila melanogaster* carrying a mutation in the *amyloid precursor-protein-like* (*Appl)* gene and (2) a series of parent strains each containing one of a series of genetic variations, wherein the series of genetic variations comprises at least twenty genetic variations,
to produce a series of test progeny, each of said test progeny carrying a mutation in said Appl gene and one of said series of genetic variations; and
(b) screening said series of test progeny for an altered phenotype relative to at least one sibling control, thereby localizing a gene that is a member of an Alzheimer's disease genetic network to one of said series of genetic variations.

2. The method of claim 1, further comprising identifying said gene that is a member of an Alzheimer's disease genetic network.

3. The method of claim 1, further comprising iteratively repeating steps (a) and (b), thereby identifying a network of functional gene interactions relating to Alzheimer's disease.

4. The method of claim 1, wherein the series of genetic variations comprises at least one hundred individual genetic variations.

5. The method of claim 1, wherein the series of genetic variations comprises a genetic variation that maps to the X-chromosome.

6. The method of claim 1, wherein each of the genetic variations in the series maps to the X-chromosome.

7. The method of claim 1, wherein the series of genetic variations comprises a genetic variation that maps to an autosome.

8. The method of claim 1, wherein each of the genetic variations in the series maps to an autosome.

9. The method of claim 1, wherein each of the series of test progeny is doubly heterozygous for the mutation in the Appl gene and one of the series of genetic variations.

10. The method of claim 1, wherein at least one parental strain comprises a balancer chromosome.

11. The method of claim 1, wherein the mutation in the Appl gene is selected from the group consisting of an amorph, hypomorph, antimorph, hypermorph and neomorph.

12. The method of claim 1, wherein the mutation in the Appl gene is a deficiency.

13. The method of claim 1, wherein the phenotype is selected from the group consisting of viability, morphology and behavior.

## Patentansprüche

1. Ein Verfahren zum Kartieren eines Netzwerkes von funktionellen Genwechselwirkungen, welche sich auf Morbus Alzheimer beziehen, Schritte umfassend, bei denen man:
(a) Kreuzungen durchführt zwischen (1) einem ersten Elternstamm von *Drosophila melanogaster,* welcher eine Mutation in dem *amyloid precursor-Protein-artig (amyloid precursor protein-like, Appl)* Gen trägt, und (2) einer Folge von Elternstämmen, von denen jeder eine Folge von genetischen Variationen enthält, wobei die Folge von genetischen Variationen mindestens 20 genetische Variationen umfasst,
um eine Folge von Testnachfahren zu produzieren, wobei jeder dieser Testnachfahren eine Mutation in dem Appl Gen und eine aus der Folge von genetischen Variationen trägt; und
(b) die Folge von Testnachfahren auf einen in Bezug auf mindestens ein Kontroll-Geschwister veränderten Phänotyp screent,
wodurch ein Gen auf eine aus der Folge von genetischen Variationen lokalisiert wird, welches ein Mitglied eines genetischen Netzwerkes bei Morbus Alzheimer ist.

2. Das Verfahren nach Anspruch 1, ferner umfassend, dass man das Gen identifiziert, welches ein Mitglied eines genetischen Netzwerks bei Morbus Alzheimer ist.

3. Das Verfahren nach Anspruch 1, ferner umfassend, dass man Schritte (a) und (b) iterativ wiederholt und **dadurch** ein Netzwerk von funktionellen Genwechselwirkungen in Bezug auf Morbus Alzheimer identifiziert.

4. Das Verfahren nach Anspruch 1, **dadurch gekennzeichnet, dass** die Folge von genetischen Variationen mindestens einhundert individuelle genetische Variationen umfasst.

5. Das Verfahren nach Anspruch 1, **dadurch gekennzeichnet, dass** die Folge von genetischen Variationen eine genetische Variation umfasst, die auf dem X-Chromosom kartiert.

6. Das Verfahren nach Anspruch 1, **dadurch gekennzeichnet, dass** jede der genetischen Variationen der Folge auf dem X-Chromosom kartiert.

7. Das Verfahren nach Anspruch 1, **dadurch gekennzeichnet, dass** die Folge von genetischen Variationen eine genetische Variation umfasst, die auf einem Autosom kartiert.

8. Das Verfahren nach Anspruch 1, **dadurch gekennzeichnet, dass** jede der genetischen Variationen der Folge auf einem Autosom kartiert.

9. Das Verfahren nach Anspruch 1, **dadurch gekennzeichnet, dass** jeder aus der Serie der Testnachfahren doppelt heterozygot in Bezug auf die Mutation in dem Appl Gen und eine aus der Folge von genetischen Variationen ist.

10. Das Verfahren nach Anspruch 1, **dadurch gekennzeichnet, dass** mindestens ein Elternstamm ein Balancer Chromosom umfasst.

11. Das Verfahren nach Anspruch 1, **dadurch gekennzeichnet, dass** die Mutation in dem Appl Gen ausgewählt ist aus der Gruppe bestehend aus einem Amorph, Hypomorph, Antimorph, Hypermorph und Neomorph.

12. Das Verfahren nach Anspruch 1, **dadurch gekennzeichnet, dass** die Mutation in dem Appl Gen eine Defizienz ist.

13. Das Verfahren nach Anspruch 1, **dadurch gekennzeichnet, dass** der Phenotyp ausgewählt ist aus der Gruppe bestehend aus Lebensfähigkeit, Morphologie und Verhalten.

## Revendications

1. Procédé permettant de cartographier un réseau d'interactions de gènes fonctionnels en rapport avec la maladie d'Alzheimer, lequel procédé comporte les étapes suivantes :
a) effectuer des accouplements entre, primo, une première souche parente de *Drosophila mélanogaster* dont le gène Appl *(amyloid precursor-protein-like)* porte une mutation et, secundo, une série de souches parentes dont chacune porte un élément d'un jeu de variations génétiques, lequel jeu de variations génétiques comprend au moins vingt variations génétiques, de manière à obtenir une série de descendances de test dont chacune est porteuse d'une mutation au niveau dudit gène Appl ainsi que d'un élément dudit jeu de variations génétiques ;
b) et passer au crible ladite série de descendances de test pour y rechercher un phénotype altéré par rapport à au moins une fratrie témoin, ce qui permet de localiser un gène membre d'un réseau de gènes en rapport avec la maladie d'Alzheimer au niveau d'un élément dudit jeu de variations génétiques.

2. Procédé conforme à la revendication 1, qui comporte en outre l'identification dudit gène membre d'un réseau de gènes en rapport avec la maladie d'Alzheimer.

3. Procédé conforme à la revendication 1, qui comporte en outre la réitération des étapes (a) et (b), ce qui permet d'identifier un réseau d'interactions de gènes fonctionnels en rapport avec la maladie d'Alzheimer.

4. Procédé conforme à la revendication 1, dans lequel le jeu de variations génétiques comprend au moins cent variations génétiques individuelles.

5. Procédé conforme à la revendication 1, dans lequel le jeu de variations génétiques comprend une variation génétique située sur le chromosome X.

6. Procédé conforme à la revendication 1, dans lequel chacune des variations génétiques du jeu est située sur le chromosome X.

7. Procédé conforme à la revendication 1, dans lequel le jeu de variations génétiques comprend une variation génétique située sur un autosome.

8. Procédé conforme à la revendication 1, dans lequel chacune des variations génétiques du jeu est située sur un autosome.

9. Procédé conforme à la revendication 1, dans lequel chacune des descendances de test de la série est doublement hétérozygote, pour la mutation située dans le gène Appl et pour l'une des variations génétiques du jeu.

10. Procédé conforme à la revendication 1, dans lequel au moins une souche parente comprend un chromosome balanceur.

11. Procédé conforme à la revendication 1, dans lequel la mutation située sur le gène Appl est choisie parmi les mutations amorphes, hypomorphes, antimorphes, hypermorphes et néomorphes.

12. Procédé conforme à la revendication 1, dans lequel la mutation située sur le gène Appl est une déficience.

13. Procédé conforme à la revendication 1, dans lequel le phénotype est choisi parmi les caractéristiques de viabilité, de morphologie ou de comportement.
